# EUROPEAN PATENT APPLICATION

(11) **EP 3 406 606 A1**
(43) Date of publication of application: **28.11.2018**
(21) Application number: 17741091.7
(22) Date of filing: 20.01.2017
(51) Int. Cl.: C07D 401/14, A61K 31/4709, A61P 11/00, A61P 11/06

(54) **NITROGENOUS HETEROCYCLIC AMIDE DERIVATIVE, PREPARATION METHOD THEREOF, AND PHARMACEUTICAL APPLICATION**

(30) Priority: 22.01.2016 CN 201610046089
(71) Applicant: Sichuan Haisco Pharmaceutical Co., Ltd., Sichuan Province 611130 (CN)
(72) Inventor: WEI, Yonggang, Chengdu City Sichuan 611130 (CN); QIU, Guanpeng, Chengdu City Sichuan 611130 (CN); LEI, Bolin, Chengdu City Sichuan 611130 (CN); WANG, Song, Chengdu City Sichuan 611130 (CN)
(74) Representative: Schmidt, Steffen J.
(86) International application number: PCT/CN2017/071821
(87) International publication number: WO 2017/125060

(57) **Abstract**

Provided are a compound represented by formula (I) or a stereoisomer, hydrate, metabolite, solvate, pharmaceutically acceptable salt, eutectic mixture, or prodrug thereof, and a preparation method thereof, and an application for preparing a pharmaceutical product for treating a disease related to obstructed airways, wherein each substituent in the compound represented by formula (I) is as described in the specification.

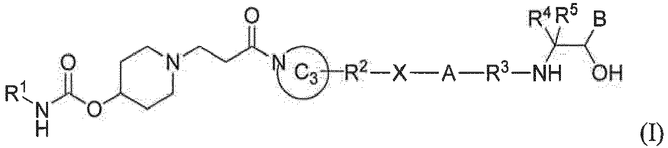

## Description

### TECHNICAL FIELD

Embodiments of the present invention relate to an azacyclic amide derivative, a method for the preparation thereof, and medical use thereof, and in particular relate to a new azacyclic amide derivative having dual activity of a muscarinic receptor antagonist and a β₂-adrenergic receptor agonist, or a stereoisomer, a hydrate, a solvate, a metabolite, a pharmaceutically acceptable salt, a cocrystal or a prodrug thereof, a pharmaceutical composition containing the same, and medical use thereof.

### BACKGROUND ART

Bronchodilators have been playing an important role in treatment of respiratory diseases such as the chronic obstructive pulmonary disease (COPD) and asthma. Bronchodilators widely used in clinical scenarios include muscarinic receptor antagonists and β₂-adrenergic agonists. Muscarinic receptor antagonists exert a bronchial dilating function by lowering the vagal cholinergic level in airway smooth muscle. Currently used inhalational muscarinic receptor antagonists include ipratropium bromide, oxitropium bromide, glycopyrronium bromide, tiotropium bromide, aclidinium bromide, and umeclidinium bromide. β₂-adrenergic agonists lead to bronchial dilatation by stimulating adrenergic receptors in airway smooth muscle, and reverse the effect of bronchial-constricting agents on various media such as acetylcholine. Currently used β₂-adrenergic agonists include salbutamol, salmeterol, arformoterol, formoterol, vilanterol and indacaterol. These drugs not only improve the function of lung, but also improve the quality of life of patients and arrest deterioration of the diseases.

Extensive clinical studies have shown that the combinational use of a muscarinic receptor antagonist and a β₂-adrenergic agonist is more effective than use of either of these therapeutic agents alone. Currently, muscarinic receptor antagonists and β₂-adrenergic agonists are clinically prepared into a combination formulation for treatment of asthma and moderate-to-severe COPDs. Such combination formulations mainly include Anoro Ellipta (umeclidinium bromide / vilanterol), Ultibro Breezhaler (glycopyrronium bromide / indacaterol), ipratropium bromide / salbutamol, and the like. Although the combination formulations have a better therapeutic effect than either individual drug contained therein, their preparation has strict requirements.

Therefore, it is desirable to develop a medicament having a dual effect of both a muscarinic receptor antagonist and a β₂-adrenergic agonist, which has the pharmacological advantages of both ingredients and also has a single molecular pharmacokinetics. These compounds are administered in the form of a single therapeutic agent, and can produce a bronchodilatory effect by two different and possibly synergistic modes of action. In addition, compounds having the dual effect of both a muscarinic receptor antagonist and a β₂-adrenergic agonist (MABA) can also be combined with corticosteroid (ICS) anti-inflammatory agents, to provide, as two therapeutic agents (MABA/ICS), a triplet therapeutic effect (Expert Opin. Investig. Drugs (2014) 23(4):453-456).

Therefore, it is necessary to develop a novel medicament having the dual activity of both a muscarinic receptor antagonist and a β₂-adrenergic agonist, to provide a single therapeutic agent or combination formulation that is more efficacious, thereby providing patients with more options of clinical medication.

### SUMMARY OF INVENTION

Embodiments of the present invention provide a compound represented by general formula (I), or a stereoisomer, a hydrate, a metabolite, a solvate, a pharmaceutically acceptable salt, a cocrystal, or a prodrug thereof, 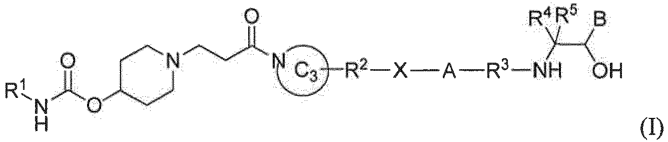 wherein
R¹ is selected from 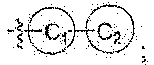 rings C₁ and C₂ are each independently selected from a C₆₋₁₀carbocycle or a 5- to 10-membered heterocycle, wherein the carbocycle or heterocycle is optionally further substituted with 0, 1, 2, 3, 4 or 5 substituents selected from the group consisting of F, Cl, Br, I, CF₃, NH₂, OH, carboxyl, cyano, a C₁₋₄alkyl, a C₁₋₄alkoxy, a C₁₋₄alkylthio, -NHC₁₋₄alkyl, -N(C₁₋₄alkyl)₂, -S(=O)-C₁₋₄alkyl, -S(=O)₂-C₁₋₄alkyl, or -C(=O)O-C₁₋₄alkyl, and wherein the heterocycle contains 1, 2 or 3 heteroatoms selected from N, O or S;
ring C₃ is selected from a 4- to 7-membered azacycle, wherein the azacycle is optionally further substituted with 0, 1, 2, 3, or 4 substituents selected from the group consisting of F, Cl, Br, I, OH, cyano, CF₃, a C₁₋₄alky1, a C₁₋₄alkoxy, and wherein the azacycle contains 1, 2 or 3 heteroatoms selected from N, O or S;
R² is selected from a direct bond or a C₁₋₄alkylene, wherein the alkylene is optionally further substituted with 0, 1, 2, 3, 4 or 5 substituents selected from the group consisting of F, Cl, Br, I, OH, cyano, a C₁₋₄alkyl, and a C₁₋₄alkoxy;
X is selected from a direct bond, -O-, -C(=O)O-, -OC(=O)-, -S-, -S(=O)-, -S(=O)₂-, -C(=O)NR^{x}-, -NR^{x}C(=O)-, -OC(=O)NR^{x}-, -NR^{x}C(=O)O-, -NR^{x}C(=O)NR^{x}-, -NR^{x}S(=O)₂-, -S(=O)₂NR^{x}-, -NR^{x}S(=O)₂NR^{x}-, or -NR^{x}-;
R^{x}s are each independently selected from H or a C₁₋₄alkyl;
A is selected from a direct bond, a C₆₋₁₀carbocycle or a 5- to 10-membered heterocycle, wherein the carbocycle or heterocycle is optionally further substituted with 0, 1, 2, 3, 4 or 5 R^{A}s, and wherein the heterocycle contains 1, 2, 3 or 4 heteroatoms selected from N, O or S;
R^{A} is selected from F, Cl, Br, I, OH, NH₂, carboxyl, cyano, nitro, (=O), a C₁₋₄alkyl, a C₂₋₄ alkenyl, a C₂₋₄ alkynyl, a C₁₋₄alkoxy, a -OC₃₋₆cycloalkyl, a C₁₋₄alkylthio, -S(=O)-C₁₋₄alkyl, -S(=O)₂-C₁₋₄alkyl, -C(=O)-C₁₋₄alkyl, -C(=O)O-C₁₋₄alkyl, -OC(=O)-C₁₋₄alkyl, a 5- or 6-membered heteroaryl, or -C(=O)NH₂, wherein the alkyl, alkoxy, cycloalkyl, alkenyl, alkynyl, heteroaryl, NH₂ and -C(=O)NH₂ are optionally further substituted with 0, 1, 2, 3, or 4 substituents selected from the group consisting of F, Cl, Br, I, CF₃, a C₁₋₄alkyl, a C₁₋₄alkoxy, or -C(=O)-C₁₋₄alkyl;
R³ is selected from a C₁₋₆ alkylene, wherein the alkylene is optionally further substituted with 0, 1, 2, 3, 4 or 5 substituents selected from R^{3a};
R^{3a} is selected from F, Cl, Br, I, cyano, OH, a C₁₋₄alkyl, a C₁₋₄alkoxy, phenyl, or phenyl-C₁₋₄alkylene;
alternatively, two R^{3a}s may form a 3- to 6-membered carbocycle together with the atoms to which they are attached, wherein the carbocycle is optionally further substituted with 0, 1, 2, 3, 4 or 5 substituents selected from the group consisting of F, Cl, Br, I, cyano, OH, a C₁₋₄alkyl, or a C₁₋₄alkoxy;
R⁴ and R⁵ are each independently selected from H or a C₁₋₄alkyl; and 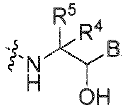 represents a β-adrenergic receptor binding group.

A preferred embodiment of the present invention is a compound represented by general formula (I), or a stereoisomer, a hydrate, a metabolite, a solvate, a pharmaceutically acceptable salt a cocrystal, or a prodrug thereof, wherein: 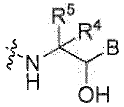 represents a β-adrenergic receptor binding group;
B is preferably 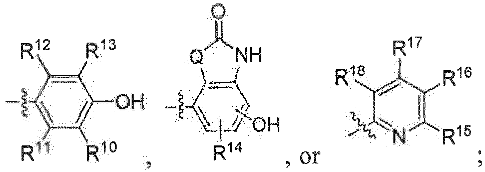 wherein R¹⁰, R¹¹, R¹², R¹³, R¹⁴, R¹⁵, R¹⁶, R¹⁷ or R¹⁸ is each independently selected from H, F, Cl, Br, I, CF₃, OH, -CH₂OH, cyano, carboxy, a C₁₋₄alkyl, a C₁₋₄alkoxy, a -C(=O)C₁₋₄alkyl, a -C(=O)OC₁₋₄alkyl, -NHC(=O)H, -NHS(=O)₂-C₁₋₄alkyl, -NHS(=O)₂-NH₂ or -NHS(=O)₂-NHC₁₋₄alkyl; and Q is selected from -CR^{q1}=CR^{q2}-, -CR^{q1}R^{q2}CR^{q3}R^{q4}-, -O-, -S-, -OCR^{q1}R^{q2}-, -CR^{q1}R^{q2}O-, -SCR^{q1}R^{q2}-, or -CR^{q1}R^{q2}S-, wherein R^{q1}, R^{q2}, R^{q3} or R^{q4} is each independently selected from H, F, Cl, Br, I or a C₁₋₄alkyl;
B is more preferably 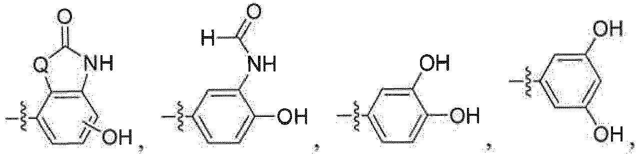 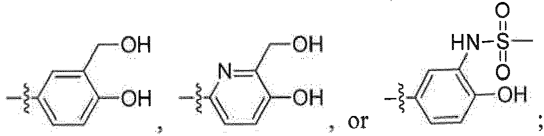 Q is selected from -CH=CH-, -CH₂CH₂-, -O-, -S-, -CH₂O-, -OCH₂-, -C(CH₃)₂O- or -OC(CH₃)₂-;
B is even more preferably 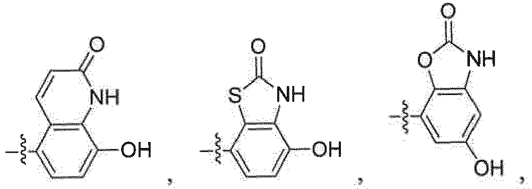 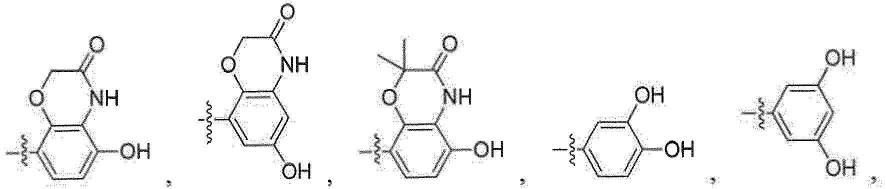 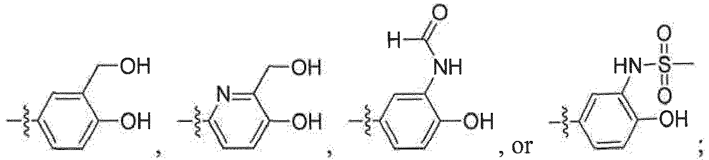
R¹ is selected from 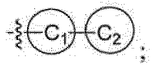 rings C₁ and C₂ are each independently selected from a C₆₋₁₀carbocycle or a 5- to 10-membered heterocycle, wherein the carbocycle or heterocycle is optionally further substituted with 0, 1, 2, 3, 4 or 5 substituents selected from the group consisting of F, Cl, Br, I, CF₃, NH₂, OH, carboxyl, cyano, a C₁₋₄alkyl, a C₁₋₄alkoxy, a C₁₋₄alkylthio, -NHC₁₋₄alkyl, -N(C₁₋₄alkyl)₂, -S(=O)-C₁₋₄alkyl, -S(=O)₂-C₁₋₄alkyl, or -C(=O)O-C₁₋₄alkyl, and wherein the heterocycle contains 1, 2 or 3 heteroatoms selected from N, O or S;
ring C₃ is selected from a 4- to 7-membered azacycle, wherein the azacycle is optionally further substituted with 0, 1, 2, 3, or 4 substituents selected from the group consisting of F, Cl, Br, I, OH, cyano, CF₃, a C₁₋₄alkyl, a C₁₋₄alkoxy, and wherein the azacycle contains 1, 2 or 3 heteroatoms selected from N, O or S;
R² is selected from a direct bond or a C₁₋₄alkylene, wherein the alkylene is optionally further substituted with 0, 1, 2, 3, 4 or 5 substituents selected from the group consisting of F, Cl, Br, I, OH, cyano, a C₁₋₄alkyl, and a C₁₋₄alkoxy;
X is selected from a direct bond, -O-, -C(=O)O-, -OC(=O)-, -S-, -S(=O)-, -S(=O)₂-, -C(=O)NR^{x}-, -NR^{x}C(=O)-, -OC(=O)NR^{x}-, -NR^{x}C(=O)O-, -NR^{x}C(=O)NR^{x}-, -NR^{x}S(=O)₂-, -S(=O)₂NR^{x}-, -NR^{x}S(=O)₂NR^{x}-, or -NR^{x}-;
R^{x}s are each independently selected from H or a C₁₋₄alkyl;
A is selected from a direct bond, a C₆₋₁₀carbocycle or a 5- to 10-membered heterocycle, wherein the carbocycle or heterocycle is optionally further substituted with 0, 1, 2, 3, 4 or 5 R^{A}s, and wherein the heterocycle contains 1, 2, 3 or 4 heteroatoms selected from N, O or S;
R^{A} is selected from F, Cl, Br, I, OH, NH₂, carboxyl, cyano, nitro, (=O), a C₁₋₄alkyl, a C₂₋₄ alkenyl, a C₂₋₄ alkynyl, a C₁₋₄alkoxy, a -OC₃₋₆cycloalkyl, a C₁₋₄alkylthio, -S(=O)-C₁₋₄alkyl, -S(=O)₂-C₁₋₄alkyl, -C(=O)-C₁₋₄alkyl, -C(=O)O-C₁₋₄alkyl, -OC(=O)-C₁₋₄alkyl, a 5- or 6-membered heteroaryl, or -C(=O)NH₂, wherein the alkyl, alkoxy, cycloalkyl, alkenyl, alkynyl, heteroaryl, NH₂ and -C(=O)NH₂ are optionally further substituted with 0, 1, 2, 3, or 4 substituents selected from the group consisting
of F, Cl, Br, I, CF₃, a C₁₋₄alkyl, a C₁₋₄alkoxy, or -C(=O)-C₁₋₄alkyl;
R³ is selected from a C₁₋₆ alkylene, wherein the alkylene is optionally further substituted with 0, 1, 2, 3, 4 or 5 substituents selected from R^{3a};
R^{3a} is selected from F, Cl, Br, I, cyano, OH, a C₁₋₄alkyl, a C₁₋₄alkoxy, phenyl, or phenyl-C₁₋₄alkylene;
alternatively, two R^{3a}s may form a 3- to 6-membered carbocycle together with the atoms to which they are attached, wherein the carbocycle is optionally further substituted with 0, 1, 2, 3, 4 or 5 substituents selected from the group consisting of F, Cl, Br, I, cyano, OH, a C₁₋₄alkyl, or a C₁₋₄alkoxy;
R⁴ and R⁵ are each independently selected from H or a C₁₋₄alkyl;

A preferred embodiment of the present invention is a compound represented by general formula (I), or a stereoisomer, a hydrate, a metabolite, a solvate, a pharmaceutically acceptable salt, a cocrystal, or a prodrug thereof, wherein:
R¹ is selected from 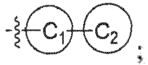
rings C₁ and C₂ are each independently selected from a C₆₋₁₀carbocycle or a 5- to 10-membered heterocycle, preferably a benzene ring, thiophene ring, thiazole ring, isothiazole ring, furan ring, oxazole ring, isoxazole ring, pyrrole ring, imidazole ring, pyridine ring, benzothiophene ring, benzothiazole ring, benzofuran ring or benzoxazole ring, wherein the carbocycle, heterocycle, benzene ring, thiophene ring, thiazole ring, isothiazole ring, furan ring, oxazole ring, isoxazole ring, pyrrole ring, imidazole ring, pyridine ring, benzothiophene ring, benzothiazole ring, benzofuran ring or benzoxazole ring is optionally further substituted with 0, 1, 2, 3, 4 or 5 substituents selected from the group consisting of F, Cl, Br, I, CF₃, NH₂, OH, cyano, a C₁₋₄alkyl, a C₁₋₄alkoxy, a C₁₋₄alkylthio, -NHC₁₋₄alkyl, or N(C₁₋₄alkyl)₂, and wherein the heterocycle contains 1, 2 or 3 heteroatoms selected from N, O or S;
ring C₃ is selected from a 4- to 7-membered azacycle, wherein the azacycle is optionally further substituted with 0, 1, 2, 3, or 4 substituents selected from the group consisting of F, Cl, Br, I, OH, cyano, CF₃, a C₁₋₄alkyl, a C₁₋₄alkoxy, and wherein the azacycle contains 1, 2 or 3 heteroatoms selected from N, O or S;
R² is selected from a direct bond or a C₁₋₄alkylene, preferably a direct bond, methylene, ethylene, propylene or butylene, wherein the alkylene, methylene, ethylene, propylene or butylene is optionally further substituted with 0, 1, 2, 3, 4 or 5 substituents selected from the group consisting of F, Cl, Br, I, OH, cyano, a C₁₋₄alkyl, and a C₁₋₄alkoxy;
X is selected from a direct bond, -O-, -C(=O)O-, -OC(=O)-, -S-, -S(=O)-, -S(=O)₂-, -C(=O)NR^{x}-, -NR^{x}C(=O)-, -OC(=O)NR^{x}-, -NR^{x}C(=O)O-, -NR^{x}C(=O)NR^{x}-, -NR^{x}S(=O)₂-, -S(=O)₂NR^{x}-, -NR^{x}S(=O)₂NR^{x}-, or -NR^{x}-; preferably a direct bond, -O-, -C(=O)NR^{x}-, -NR^{x}C(=O)-, -OC(=O)NR^{x}-, or -NR^{x}C(=O)O-;
R^{x}s are each independently selected from H or a C₁₋₄alkyl; preferably H, methyl, ethyl or propyl;
A is selected from a direct bond, a C₆₋₁₀carbocycle or a 5- to 10-membered heterocycle, preferably phenylene, or pyridylene, wherein the carbocycle, heterocycle, phenylene or pyridylene is optionally further substituted with 0, 1, 2, 3, 4 or 5 R^{A}s, and wherein the heterocycle contains 1, 2, 3 or 4 heteroatoms selected from N, O or S;
R^{A} is selected from F, Cl, Br, I, OH, NH₂, carboxyl, cyano, (=O), a C₁₋₄alkyl, a C₂₋₄ alkynyl, a C₁₋₄alkoxy, a -OC₃₋₆cycloalkyl, or a C₁₋₄alkylthio, preferably F, Cl, Br, cyano, methyl, ethyl, propyl, isopropyl, CHF₂, CF₃, methoxy, ethoxy, -OCHF₂, -OCF₃, ethynyl, or propynyl, wherein the alkyl, alkynyl, alkoxy, cycloalkyl, and NH₂ are optionally further substituted with 0, 1, 2, 3, or 4 substituents selected from the group consisting of F, Cl, Br, I, CF₃, a C₁₋₄alkyl, a C₁₋₄alkoxy, or -C(=O)-C₁₋₄alkyl;
R³ is selected from a C₁₋₆ alkylene, preferably methylene, ethylene, propylene, or butylene, wherein the alkylene, methylene, ethylene, propylene, or butylene is optionally further substituted with 0, 1, 2, 3, 4 or 5 substituents selected from R^{3a};
R^{3a} is selected from F, Cl, Br, I, cyano, OH, a C₁₋₄alkyl, a C₁₋₄alkoxy, phenyl, or phenyl-C₁₋₄alkylene;
alternatively, two R^{3a}s may form a 3- to 6-membered carbocycle together with the atoms to which they are attached, wherein the carbocycle is optionally further substituted with 0, 1, 2, 3, 4 or 5 substituents selected from the group consisting of F, Cl, Br, I, cyano, OH, a C₁₋₄alkyl, or a C₁₋₄alkoxy;
R⁴ and R⁵ are each independently selected from H or a C₁₋₄alkyl; preferably H, methyl, or ethyl;
B is selected from 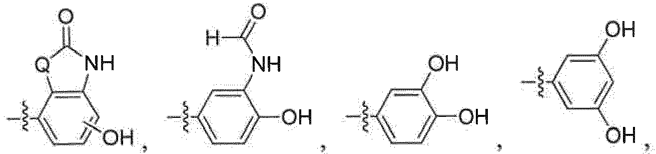 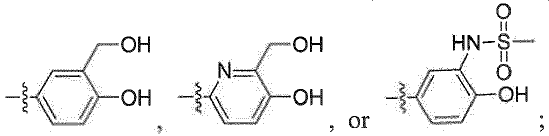 Q is selected from -CH=CH-, -CH₂CH₂-, -O-, -S-, -CH₂O-, -OCH₂-, -C(CH₃)₂O- or -OC(CH₃)₂-;
B is preferably

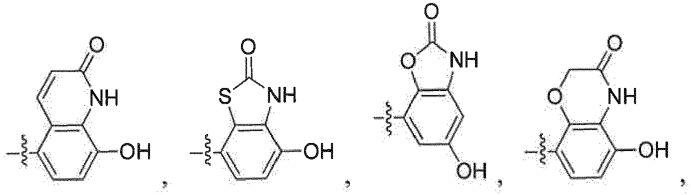

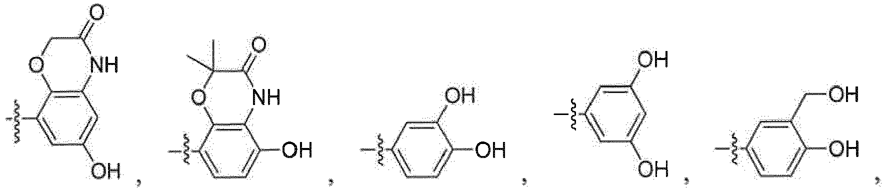

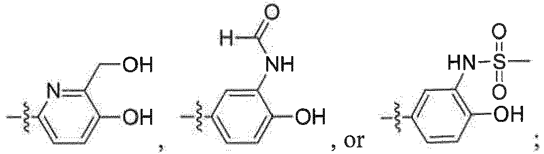

A preferred embodiment of the present invention is a compound represented by general formula (I), or a stereoisomer, a hydrate, a metabolite, a solvate, a pharmaceutically acceptable salt, a cocrystal, or a prodrug thereof, wherein:
R¹ is selected from 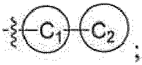 rings C₁ and C₂ are each independently selected from a benzene ring, thiophene ring, thiazole ring, isothiazole ring, furan ring, oxazole ring, isoxazole ring, pyrrole ring, imidazole ring, pyridine ring, benzothiophene ring, benzothiazole ring, benzofuran ring or benzoxazole ring, wherein the benzene ring, thiophene ring, thiazole ring, isothiazole ring, furan ring, oxazole ring, isoxazole ring, pyrrole ring, imidazole ring, pyridine ring, benzothiophene ring, benzothiazole ring, benzofuran ring or benzoxazole ring is optionally further substituted with 0, 1, 2, 3, 4 or 5 substituents selected from the group consisting of F, Cl, Br, I, CF₃, NH₂, OH, cyano, a C₁₋₄alkyl, a C₁₋₄alkoxy, a C₁₋₄alkylthio, -NHC₁₋₄alkyl, or -N(C₁₋₄alkyl)₂;
ring C₃ is selected from a 4- to 7-membered azacycle, wherein the azacycle is optionally further substituted with 0, 1, 2, 3, or 4 substituents selected from the group consisting of F, Cl, Br, I, CF₃, methyl or ethyl, and wherein the azacycle contains 1, 2 or 3 heteroatoms selected from N, O or S;
R² is selected from a direct bond, methylene, ethylene or propylene, wherein the methylene, ethylene or propylene is optionally further substituted with 0, 1, 2, 3, 4 or 5 substituents selected from the group consisting of F, Cl, Br, I, cyano, OH, methyl, ethyl, methoxy, or ethoxy;
X is selected from a direct bond, -O-, -C(=O)O-, -OC(=O)-, -S-, -S(=O)-, -S(=O)₂-, -C(=O)NR^{x}-, -NR^{x}C(=O)-, -OC(=O)NR^{x}-, -NR^{x}C(=O)O-, -NR^{x}C(=O)NR^{x}-, -NR^{x}S(=O)₂-, -S(=O)₂NR^{x}-, -NR^{x}S(=O)₂NR^{x}-, or -NR^{x}-; preferably a direct bond, -O-, -C(=O)NR^{x}-, -NR^{x}C(=O)-, -OC(=O)NR^{x}-, or -NR^{x}C(=O)O-;
R^{x}s are each independently selected from H, methyl, ethyl or propyl;
A is selected from a direct bond, phenylene, or pyridylene, wherein the phenylene or pyridylene is optionally further substituted with 0, 1, 2, 3, or 4 substituents selected from the group consisting of F, Cl, Br, cyano, methyl, ethyl, propyl, isopropyl, CHF₂, CF₃, methoxy, ethoxy, -OCHF₂, -OCF₃, ethynyl, or propynyl;
R³ is selected from methylene, ethylene, propylene, butylene, pentylene, or 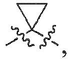 wherein the methylene, ethylene, propylene, butylene, pentylene, or 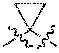 is optionally further substituted with 0, 1, 2, 3, 4 or 5 substituents selected from the group consisting of F, Cl, Br, I, cyano, OH, methyl, ethyl, methoxy or ethoxy;
R⁴ and R⁵ are each independently selected from H, methyl or ethyl;
B is selected from 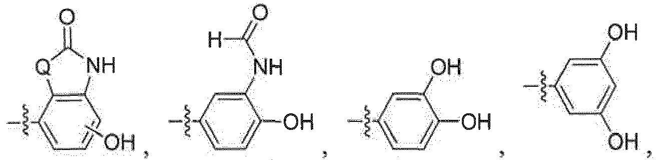 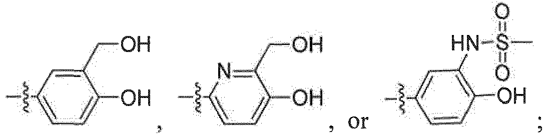 Q is selected from -CH=CH-, -CH₂CH₂-, -O-, -S-, -CH₂O-, -OCH₂-, -C(CH₃)₂O- or -OC(CH₃)₂-;
B is preferably

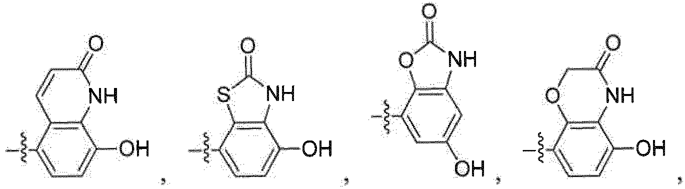

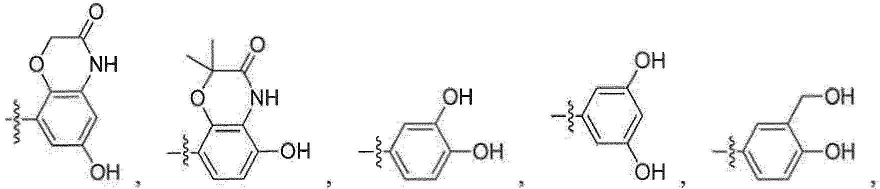

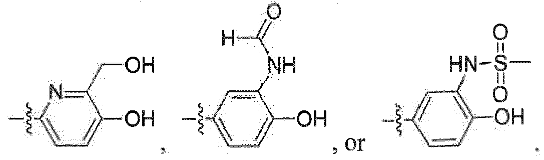

A preferred embodiment of the present invention is a compound represented by general formula (I), or a stereoisomer, a hydrate, a metabolite, a solvate, a pharmaceutically acceptable salt, a cocrystal, or a prodrug thereof, wherein:
R¹ is selected from 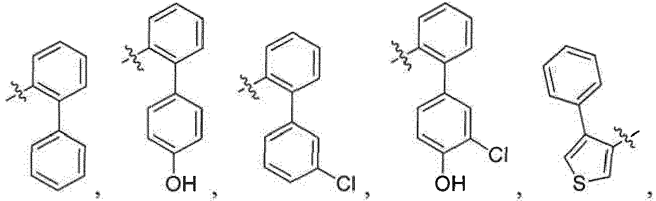 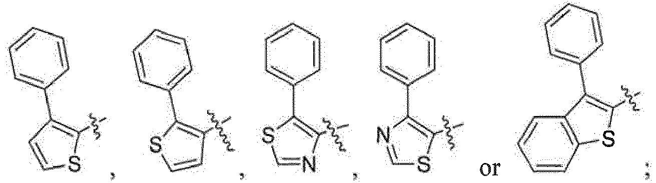
R² is selected from a direct bond, methylene, ethylene or propylene;
X is selected from a direct bond, -O-, -C(=O)NR^{x}-, -NR^{x}C(=O)-, -OC(=O)NR^{x}-, or -NR^{x}C(=O)O-;
R^{x} is selected from H, methyl, ethyl or propyl;
A is selected from a direct bond, phenylene, or pyridylene, wherein the phenylene or pyridylene is optionally further substituted with 0, 1, 2, 3, or 4 substituents selected from the group consisting of F, Cl, Br, CHF₂, CF₃, cyano, methyl, ethyl, methoxy, ethoxy, -OCHF₂, -OCF₃, ethynyl, or propynyl;
R³ is selected from methylene, ethylene, propylene, -CH₂CH(CH₃)-, -CH(CH₃)CH₂-, -CH₂C(CH₃)₂-, -C(CH₃)₂CH₂-, butylene, -CH(CH₃)CH₂CH₂-, -CH₂CH(CH₃)CH₂-, -CH₂CH(CH₃)CH₂-, 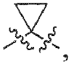 or pentylene;
R⁴ and R⁵ are each independently selected from H, methyl or ethyl;
B is selected from 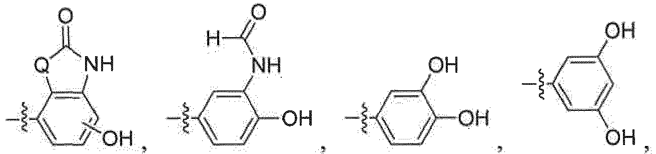 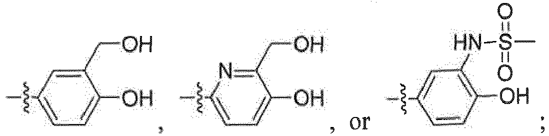 Q is selected from -CH=CH-, -CH₂CH₂-, -O-, -S-, -CH₂O-, -OCH₂-, -C(CH₃)₂O- or -OC(CH₃)₂-;
B is preferably

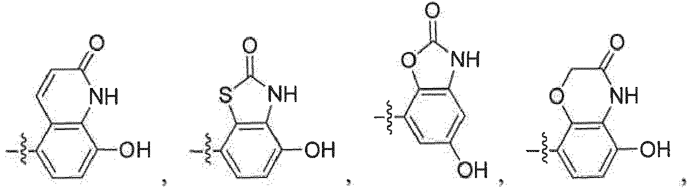

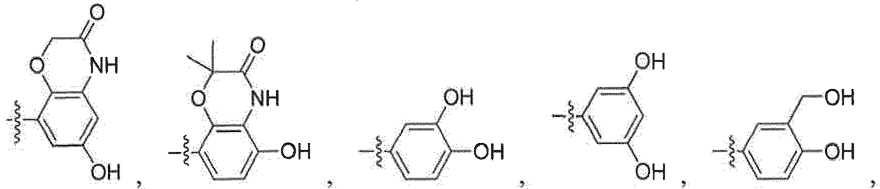

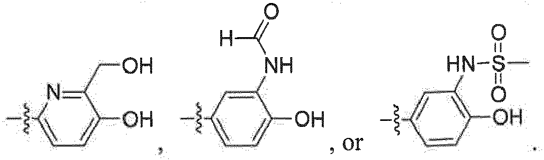

Embodiments of the present invention also provide a compound represented by general formula (II), or a stereoisomer, a hydrate, a metabolite, a solvate, a pharmaceutically acceptable salt, a cocrystal, or a prodrug thereof, wherein: 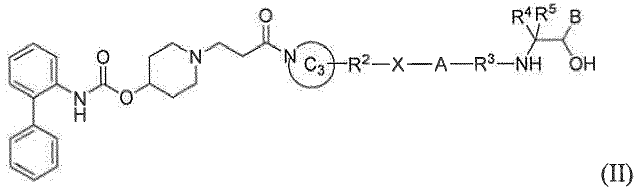
ring C₃ is selected from 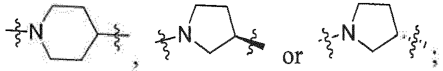
R² is selected from a direct bond, methylene, ethylene or propylene;
X is selected from a direct bond, -O-, -C(=O)NR^{x}-, -NR^{x}C(=O)-, -OC(=O)NR^{x}-, or -NR^{x}C(=O)O-;
R^{x} is selected from H, methyl, ethyl or propyl;
A is selected from a direct bond or phenylene, wherein the phenylene is optionally further substituted with 0 to 4 substituents selected from the group consisting of F, Cl, Br, CHF₂, CF₃, cyano, methyl, ethyl, methoxy, ethoxy, -OCHF₂, -OCF₃, ethynyl, or propynyl;
R³ is selected from methylene, ethylene, propylene, -CH₂CH(CH₃)-, -CH(CH₃)CH₂-, -CH₂C(CH₃)₂-, -C(CH₃)₂CH₂-, butylene, -CH(CH₃)CH₂CH₂-, -CH₂CH(CH₃)CH₂-, -CH₂CH(CH₃)CH₂-, 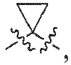 or pentylene;
R⁴ and R⁵ are each independently selected from H, methyl or ethyl;
B is selected from 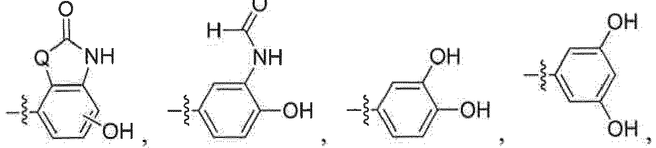 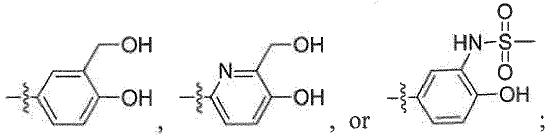 Q is selected from -CH=CH-, -CH₂CH₂-, -O-, -S-, -CH₂O-, -OCH₂-, -C(CH₃)₂O- or -OC(CH₃)₂-;
B is preferably

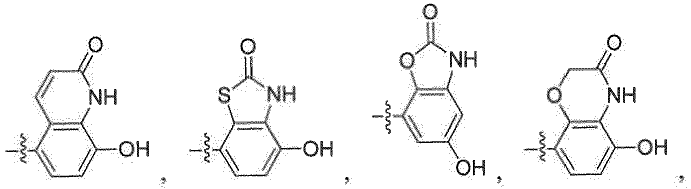

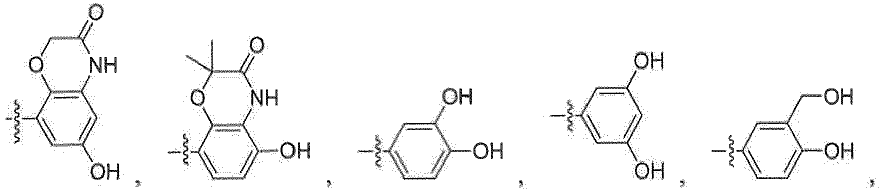

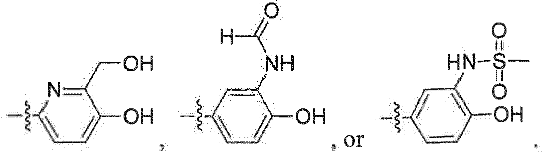

In a preferred embodiment of the present invention, the compound according to the present invention is selected from, but not limited to:

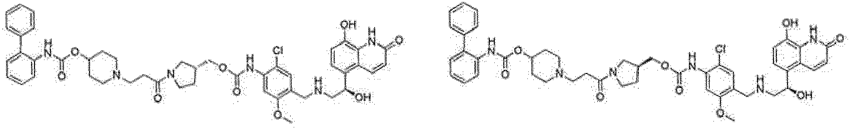

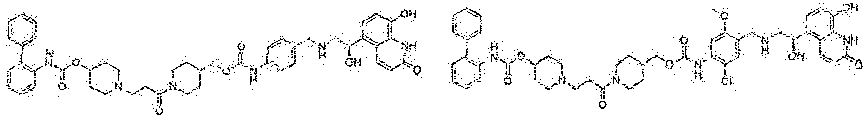

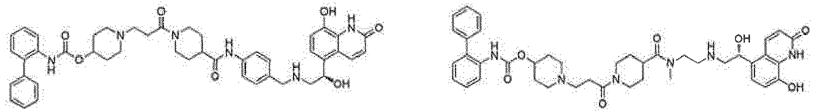

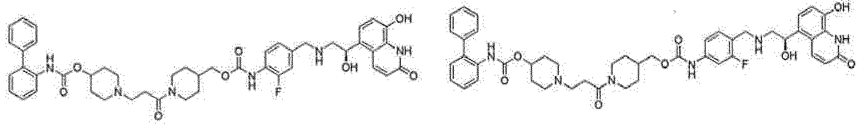

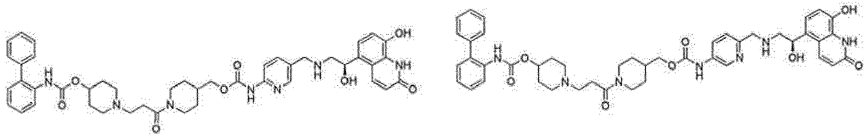

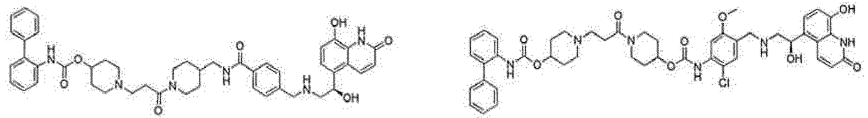

An embodiment of the present invention provides a pharmaceutical composition comprising: i) a therapeutically effective amount of any compound of general formula (I) or (II), or a stereoisomer, a hydrate, a metabolite, a solvate, a pharmaceutically acceptable salt, a cocrystal, or a prodrug thereof; and ii) a pharmaceutically acceptable carrier, diluent, adjuvant, medium, or excipient. The composition may further comprise one or more secondary therapeutic agents, which are preferably selected from one or more of PDE4 inhibitors, muscarinic receptor antagonists, corticosteroids and β-adrenergic receptor agonists.

An embodiment of the present invention provides use of a compound of general formula (I) or (II), or a stereoisomer, a hydrate, a metabolite, a solvate, a pharmaceutically acceptable salt, a cocrystal, or a prodrug thereof, or the pharmaceutical composition defined above, in the manufacture of a medicament for treatment of an airway obstructive disease, preferably asthma, chronic obstructive pulmonary disease (COPD) or bronchitis.

An embodiment of the present invention provides a method for treating an airway obstructive disease, comprising administering a compound of general formula (I) or (II), or a stereoisomer, a hydrate, a metabolite, a solvate, a pharmaceutically acceptable salt, a cocrystal, or a prodrug thereof, or the pharmaceutical composition described above.

An embodiment of the present invention provides a method for treating asthma, COPD or bronchitis, comprising administering a compound of general formula (I) or (II), or a stereoisomer, a hydrate, a metabolite, a solvate, a pharmaceutically acceptable salt, a cocrystal, or a prodrug thereof, or the pharmaceutical composition described above.

An embodiment of the present invention provides an intermediate for preparing a compound of general formula (I) or a stereoisomer thereof, said intermediate being selected from compounds of general formula (I-M1) or (I-M2) or a stereoisomer thereof: 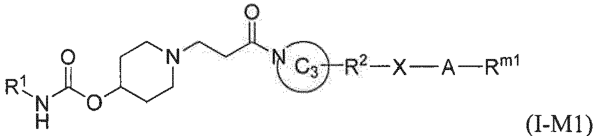 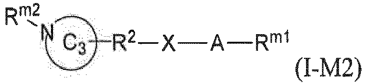 wherein R^{m1} is selected from -COOH, a -COOC₁₋₄alkl, cyano, -CHO, 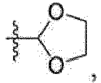 C(OC₁₋₄alkyl)₂, or 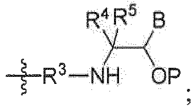 R^{m2} is selected from H or a protective group for amino, wherein the protective group for amino is preferably t-butoxycarbonyl or benzyl;
P is a protective group for hydroxyl, preferably benzyl or t-butyldimethylsilyl;
R¹, R², R³, R⁴, R⁵, A, B, ring C₃ and X are as defined for the compound of general formula (I).

A preferred embodiment of the present invention provides an intermediate for preparing a compound of general formula (I) or a stereoisomer thereof, said intermediate being selected from the following compounds:

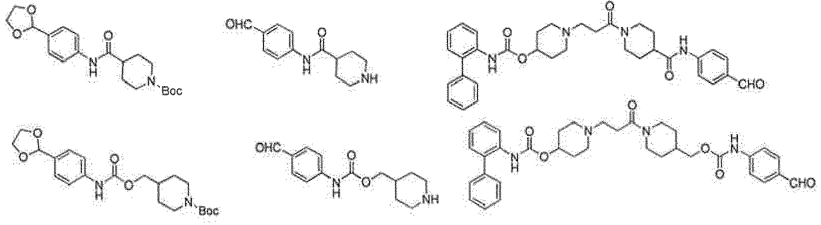

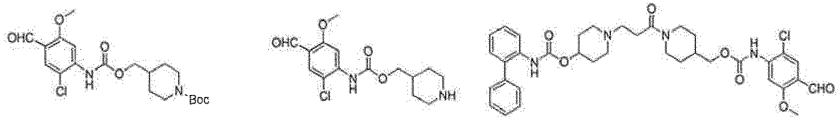

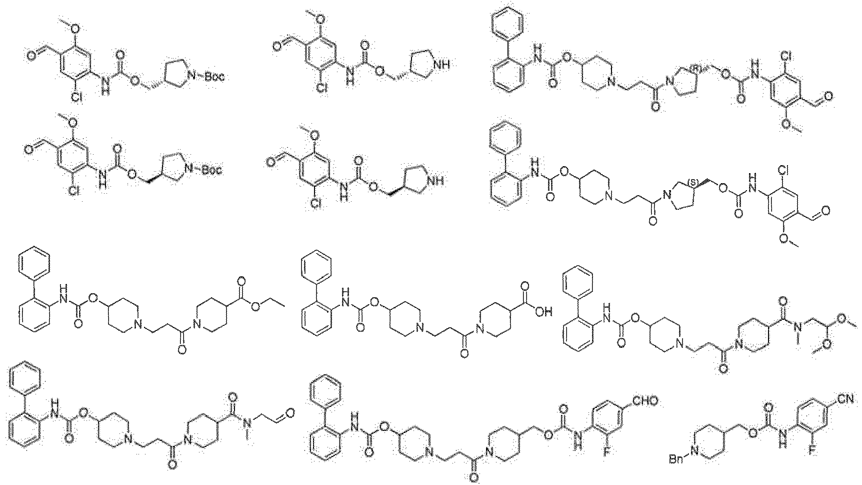

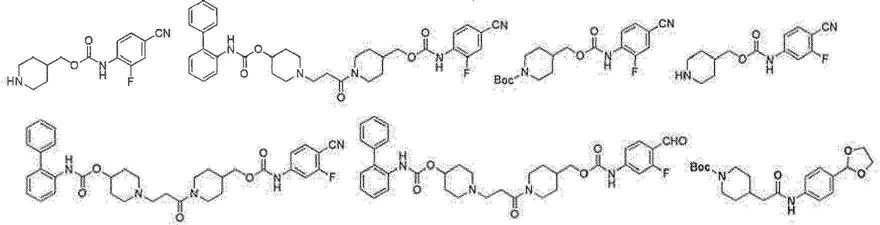

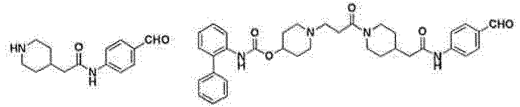

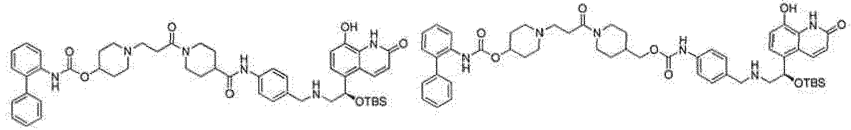

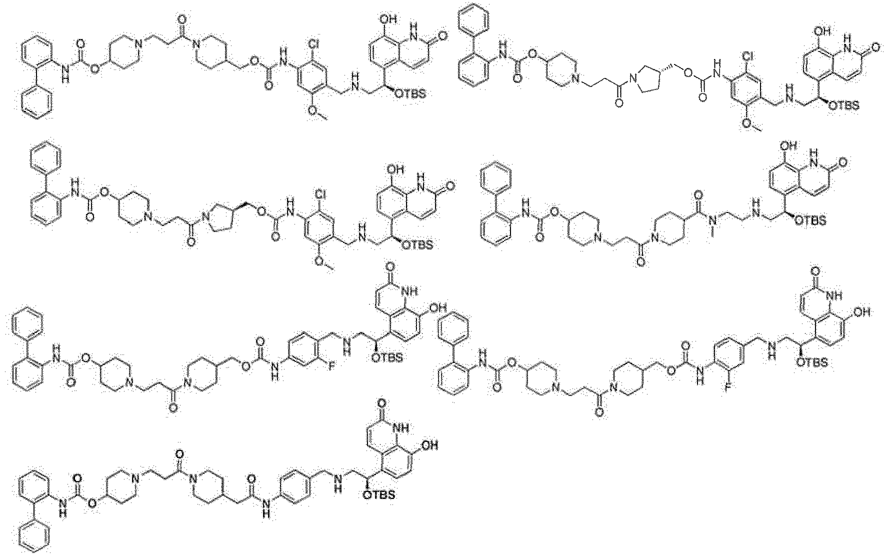

Unless otherwise indicated, the terms used throughout the specification and claims have the following meanings.

All of the carbon, hydrogen, oxygen, sulfur, nitrogen or halogen involved in the groups and compounds according to the present invention include their isotopes. All of the carbon, hydrogen, oxygen, sulfur, nitrogen or halogen involved in the groups and compounds according to the present invention are optionally further replaced by one or more of their corresponding isotopes, wherein the carbon isotopes include ¹²C, ¹³C and ¹⁴C, the hydrogen isotopes include protium (H), deuterium (D, also known as heavy hydrogen) and tritium (T, also known as superheavy hydrogen), the oxygen isotopes include ¹⁶O, ¹⁷O and ¹⁸O, the sulfur isotopes include ³²S, ³³S, ³⁴S and ³⁶S, the nitrogen isotopes include ¹⁴N and ¹⁵N, the fluorine isotopes include ¹⁹F, the chlorine isotopes include ³⁵Cl and ³⁷Cl, and the bromine isotopes include ⁷⁹Br and ⁸¹Br.

"Alkyl" means a linear or branched saturated monovalent hydrocarbon group, having in the main chain 1 to 10 carbon atoms, preferably 1 to 8 carbon atoms, more preferably 1 to 6 carbon atoms, even more preferably 1 to 4 carbon atoms, and most preferably 1 to 2 carbon atoms. Examples of alkyl include, but are not limited to, methyl, ethyl, n-propyl, isopropyl, n-butyl, isobutyl, sec-butyl, t-butyl, n-pentyl, 2-pentyl, 3-pentyl, 2-methyl-2-butyl, 3-methyl-2-butyl, n-hexyl, n-heptyl, n-octyl, n-nonyl, n-decyl, and the like. The alkyl may be optionally further substituted with 0, 1, 2, 3, 4 or 5 substituents selected from F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}, wherein R¹⁹ and R^{19a} are each independently selected from H, hydroxyl, amino, carboxy, C₁₋₈ alkyl, C₁₋₈ alkoxy, C₂₋₈ alkenyl, C₂₋₈ alkynyl, 3- to 10-membered carbocycle, 4- to 10-membered heterocyclyl, 3- to 10-membered carbocyclyloxy, or 4- to 10-membered heterocyclyloxy, k is selected from 0, 1, 2, 3, 4 or 5, and j is selected from 0, 1 or 2. Alkyl, k, j, R¹⁸ and R^{18a} used throughout the specification are defined as above.

"Alkylene" refers to a linear and branched saturated divalent hydrocarbon group, including -(CH₂)ᵥ- (v is an integer from 1 to 10). Examples of alkylene include, but are not limited to, methylene, ethylene, propylene, butylene and the like. The alkylene may be optionally further substituted with 0, 1, 2, 3, 4 or 5 substituents selected from F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₐ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. When the number of substituents in the alkylene is 2 or more, the substituents may fuse together to form a cyclic structure. Alkylene used throughout the specification is defined as above.

"Alkoxy" means a monovalent group -O-alkyl, wherein the alkyl is as defined above. Examples of alkoxy include, but are not limited to, methoxy, ethoxy, 1-propoxy, 2-propoxy, 1-butoxy, 2-methyl-1-propoxy, 2-butoxy, 2-methyl-2-propoxy, 1-pentyloxy, 2-pentyloxy, 3-pentyloxy, 2-methyl-2-butoxy, 3-methyl-2-butoxy, 3-methyl-1-butoxy, 2-methyl-1-butoxy, and the like.

"Alkenyl" refers to a linear or branched unsaturated monovalent hydrocarbon group having at least one, generally one, two or three carbon-carbon double bonds, and having 2 to 10 carbon atoms, more preferably 2 to 6 carbon atoms, and even more preferably 2 to 4 carbon atoms in the main chain. Examples of alkenyl include, but are not limited to, vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadienyl, 1,3-pentadienyl, 1,4-pentadienyl, 1,4-hexadienyl, and the like. The alkenyl may be optionally further substituted with 0, 1, 2, 3, 4 or 5 substituents selected from F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Alkenyl used throughout the specification is defined as above.

"Alkenylene" is a divalent group derived from alkenyl, wherein the alkenyl is defined as above.

"Alkynyl" refers to a linear or branched unsaturated monovalent hydrocarbon group having at least one, generally one, two or three carbon-carbon triple bonds, and having 2 to 10 carbon atoms, more preferably 2 to 6 carbon atoms, and even more preferably 2 to 4 carbon atoms in the main chain. Examples of alkynyl include, but are not limited to, ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, and the like. The alkynyl may be optionally further substituted with 0, 1, 2, 3, 4 or 5 substituents selected from F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Alkynyl used throughout the specification is defined as above.

"Alkynylene" is a divalent group derived from alkynyl, wherein the alkynyl is defined as above.

"Cycloalkyl" refers to a saturated monovalent cyclic hydrocarbon group which generally has 3 to 10 carbon atoms. Non-limiting examples thereof include cyclopropyl, cyclobutyl, cyclopentyl, cyclohexyl, cycloheptyl, and the like. The cycloalkyl may be optionally further substituted with 0, 1, 2, 3, 4 or 5 substituents selected from F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Cycloalkyl used throughout the specification is defined as above.

"Cycloalkylene" is a divalent group derived from cycloalkyl, wherein the cycloalkyl is defined as above.

"Aryl" refers to a monovalent aromatic hydrocarbonyl having a monocyclic or fused ring, and generally having 6 to 10 carbon atoms. Non-limiting examples thereof include phenyl, naphtha-1-yl or naphtha-2-yl. The aryl may be optionally further substituted with 0, 1, 2, 3, 4 or 5 substituents selected from F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, ⁻(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Aryl used throughout the specification is defined as above.

"Arylene" is a divalent aryl, wherein the aryl is defined as above.

"Heteroaryl" refers to a monovalent aromatic group having a monocyclic ring or two rings fused together, containing at least one heteroatom selected from N, O or S in the backbone of the ring, and is generally 5- to 8-membered. Non-limiting examples thereof include pyrrolyl, imidazolyl, thiazolyl, thienyl, furanyl, pyrazolyl, isoxazolyl, oxazolyl, pyridyl or pyrazinyl. The heteroaryl may be optionally further substituted with 0, 1, 2, 3, 4 or 5 substituents selected from F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Heteroaryl used throughout the specification is defined as above.

"Heteroarylene" is a divalent heteroaryl, wherein the heteroaryl is defined as above.

"Carbocyclyl" or "carbocycle" refers to a saturated or unsaturated aromatic or non-aromatic cyclic group which may be a 3- to 10-membered monocyclic ring, a 4-to 12-membered bicyclic ring, or a 10- to 15-membered tricyclic ring system, and may be attached with a bridged or spiro ring. Non-limiting examples include cyclopropyl, cyclobutyl, cyclopentyl, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, cyclohexadienyl, cycloheptyl, cyclooctyl, cyclononyl, cyclodecyl, cycloundecyl, cyclododecyl, phenyl, naphthyl, 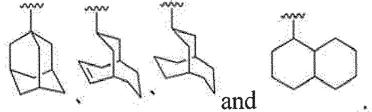 The carbocyclyl may be optionally further substituted with 0, 1, 2, 3, 4 or 5 substituents selected from F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈alkenyl, C₂₋₈alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Carbocyclyl used throughout the specification is defined as above.

"Heterocyclyl" or "heterocycle" refers to a saturated or unsaturated, aromatic or non-aromatic ring having 1 to 4 heteroatoms selected from N, O or S, and the aromatic or non-aromatic ring may be a 3- to 10-membered monocyclic ring, a 4- to 12-membered bicyclic ring, or a 10- to 15-membered tricyclic ring system. A 3- to 8-membered heterocycle is preferred. The optional N and S substituted in the ring of a heterocyclyl may be oxidized to various oxidative states. A heterocyclyl may be attached to a heteroatom or a carbon atom, and may be attached with a bridged or spiro ring. Non-limiting examples include epoxyethyl, epoxypropyl, aziridinyl, oxetanyl, azetidinyl, thietanyl, 1,3-dioxolanyl, 1,4-dioxolanyl, 1,3-dioxetanyl, azepanyl, oxepanyl, thiepanyl, oxazepinyl, diazepinyl, thiazepinyl, pyridyl, piperidinyl, homopiperidyl, furyl, thienyl, pyranyl, N-alkylpyrrolyl, pyrimidinyl, pyrazinyl, pyridazinyl, piperazinyl, homopiperazinyl, imidazolyl, piperidinyl, morpholinyl, thiomorpholinyl, thioxanyl, 1,3-dithiyl, dihydrofuryl, dihydropyranyl, dithiolanyl, tetrahydrofuranyl, tetrahydrothienyl, tetrahydropyranyl, tetrahydrothiopyranyl, tetrahydropyrrolyl, tetrahydroimidazolyl, tetrahydrothiazolyl, tetrahydropyranyl, benzimidazolyl, benzopyridyl, pyrrolopyridyl, benzodihydrofuranyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3H-indolylquinolizinyl, N-pyridylurea, 1,1-dioxothiomorpholinyl, azabicyclo[3.2.1]octanyl, azabicyclo[5.2.0]nonanyl, oxatricyclo[5.3.1.1]dodecanyl, azadamantyl, and oxaspiro[3.3]heptyl. The heterocyclyl may be optionally further substituted with 0, 1, 2, 3, 4 or 5 substituents selected from F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈alkenyl, C₂₋₈alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Heterocyclyl used throughout the specification is defined as above.

A "β-adrenergic receptor binding group" refers to groups capable of binding a β-adrenergic receptor, see for example the review "β-adrenergic receptors" in Comprehensive Medicinal Chemistry, 1990, B.E.Main, p187 (Pergamon Press), and also see WO/2005092841, US/20050215542, WO/2005070872, WO/2006023460, WO/2006051373, WO/2006087315, and WO/2006032627. Non-limiting examples include 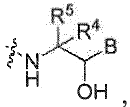 wherein R⁴ and R⁵ are each independently selected from H or a C₁₋₄alkyl, and B is selected from 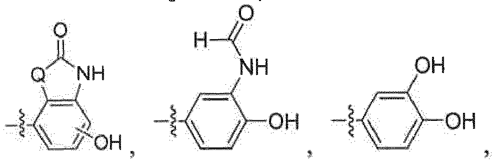 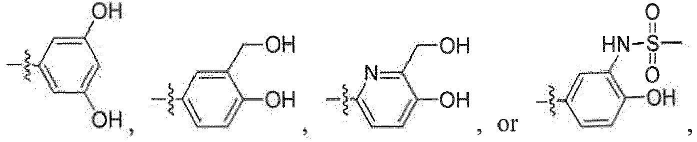 with Q selected from -CH=CH-, -CH₂CH₂-, -O-, -S-, -CH₂O-, -OCH₂-, -C(CH₃)₂O-, or -OC(CH₃)₂-.

A "protective group for amino" refers to a group for protecting amino, which is suitable for protecting an amino group from undergoing a chemical reaction but is easily removed after the desired chemical reaction is completed at other parts of the molecule. It includes, but is not limited to, benzyl, p-methoxybenzyl, trityl, t-butoxycarbonyl, benzyloxycarbonyl, 9-fluorenylmethoxycarbonyl, 2,2,2-trichloroethoxycarbonyl, ethoxycarbonyl, benzyloxycarbonyl, trifluoroacetyl, acetyl or benzoyl.

A "protective group for hydroxyl" refers to a group for protecting hydroxyl, which is suitable for protecting a hydroxyl group from undergoing a chemical reaction but is easily removed after the desired chemical reaction is completed at other parts of the molecule. It includes, but is not limited to, benzyl, trimethylsilyl, t-butyldimethylsilyl, t-butyldiphenylsilyl, tri(isopropyl)silyl, tri(t-butyl)silyl, methyl, t-butyl, allyl, triphenylmethyl, methoxymethyl, ethoxymethyl, methoxyethoxymethyl, benzyloxymethyl, monochloroacetyl, dichloroacetyl, trichloroacetyl, benzoyl or t-butylacyl, wherein the benzyl, benzyloxymethyl and benzoyl are optionally substituted with 0 to 5 substituents selected from the group consisting of a C₁₋₄alkyl, a C₁₋₄alkoxy, F, Cl, Br or I.

"Optional" or "optionally" means that the event or scenario described by them may, but does not have to, happen, and encompasses both cases where the event or scenario happens and does not happen. For example, "an alkyl optionally substituted with F" means that the alkyl may, but does not have to, be substituted with F, encompassing both the case where the alkyl is substituted with F and the case where the alkyl is not substituted with F.

A "pharmaceutical composition" refers to a mixture of one or more of the compounds according to the present disclosure or pharmaceutically/physiologically acceptable salts thereof with additional components, wherein the additional components include pharmaceutically/physiologically acceptable carriers or excipients.

"Carrier" means a vehicle or diluent that does not cause significant stimulation to an organism and does not eliminate the biological activity and characteristics of a given compound.

"Excipient" means an inert substance added into a pharmaceutical composition to further facilitate administration of a compound. Examples thereof include, but are not limited to, calcium carbonate, calcium phosphate, various sugars, different types of starch, cellulose derivatives (including microcrystalline cellulose), gelatin, vegetable oils, polyethylene glycols, diluent, a granulating agent, lubricant, binder, disintegrant, and so on.

A "prodrug" means a compound that can be converted under physiological conditions or under the action of solvent into the biologically active compound of the present invention. A prodrug of the present invention is prepared by modification of a functional group of the compound of the present invention. Such a modification can be removed *in vivo* or by conventional operations, so as to produce the parent compound.

A "stereoisomer" refers to an isomer due to a different spatial arrangement of atoms in a molecule, including cis-trans isomers, enantiomers, and conformational isomers.

An "effective amount" means an amount that causes a physiological or medical response in a tissue, system or subject and is a desirable amount, including the amount of a compound that is, after administered to a subject to be treated, sufficient to prevent occurrence of one or more symptoms of the disease or disorder to be treated or to reduce the symptom(s) to a certain degree.

A "solvate" refers to the compound of the present invention or a salt thereof that further contains a stoichiometric or non-stoichiometric amount of solvent bound via a non-covalent intermolecular force. When the solvent is water, the solvate is hydrate.

"IC₅₀" means half maximal inhibitory concentration, the concentration that achieves half of the maximum inhibitory effect.

### DETAILED DESCRIPTION OF INVENTION

The technical solutions of the present invention are described in detail hereinafter in connection with the figures and Examples, but the scope of protection of the present invention is not limited thereto.

The structures of compounds were determined by nuclear magnetic resonance (NMR) and/or mass spectroscopy (MS). NMR shifts (δ) are presented in 10⁻⁶ ppm. NMR measurements were performed with a Bruker ADVANCE III 400 NMR device and a Brucker ADVANCE 300 NMR device, wherein the measurement solvents were hexadeuterodimethyl sulfoxide (DMSO-*d*₆), deuterochloroform (CDCl₃), and deuteromethanol (CD₃OD), and the internal reference was tetramethylsilane (TMS).

MS measurements were performed with Agilent 6120B (ESI) and Agilent 6120B (APCI).

HPLC measurements were performed with Agilent 1260DAD High-pressure Liquid Chromatograph (Zorba x SB-C18 100 x 4.6 mm).

Thin-layer chromatography silica gel plate: HSGF254 silica gel plate (Huanghai, Yantai) or GF254 silica gel plate (Qingdao). The specification of the silica gel plate used for thin-layer chromatography (TLC) was 0.15 mm to 0.20 mm, and that for product isolation and purification by TLC was 0.4 mm to 0.5 mm.

The chromatography column generally used the silica gel (Huanghai, Yantai) of 200 to 300 mesh as a carrier.

Known starting materials in connection with the present invention can be synthesized following or using methods known in the art, or can be purchased from companies such as Titansci, Energy Chemical, Demochem (Shanghai), Kelong Chemical (Chengdu), Accela ChemBio, and J&K Scientific.

A N₂ atmosphere means that the reaction vessel is connected to a N₂ balloon of about 1 L in volume.

A H₂ atmosphere means that the reaction vessel is connected to a H₂ balloon of about 1 L in volume.

Hydrogenation reactions generally involve a vacuuming and H₂-charging operation repeated 3 times.

In the Examples, unless particularly specified, reactions were carried out under a N₂ atmosphere.

In the Examples, unless particularly specified, solutions refer to aqueous solutions.

In the Examples, unless particularly specified, reaction temperatures are room temperature, and the most suitable room temperature as a reaction temperature is 20°C to 30°C.

M refers to mole/litre.
TBS means t-butyldimethylsilyl.
Boc means t-butoxycarbonyl.
Bn means benzyl.
TFA means trifluoroacetic acid.
HATU: 2-(7-azabenzotriazolyl)-N,N,N',N'-tetramethyluronium hexafluorophosphate (CAS: 148893-10-1)

### Intermediate 1:

### [1-[3-[4-[(4-formylphenyl)carbamoyl]-1-piperidyl]-3-oxo-propyl]-4-piperidyl] N-(2-phenylphenyl)carbamate (Intermediate 1)

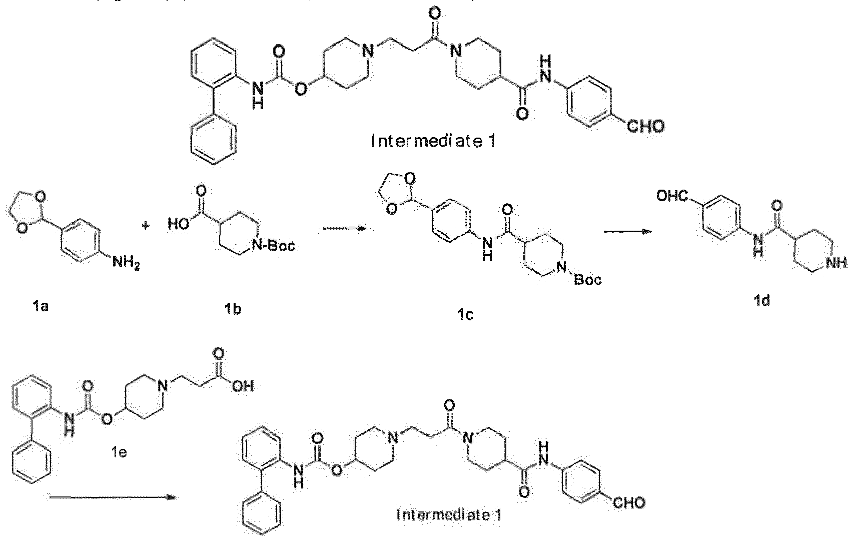

### Step 1: tert-butyl 4-[[4-(1,3-dioxolan-2-yl)phenyl]carbamoyl]piperidine-1-carboxylate (1c)

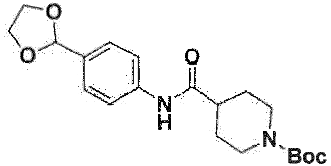

1-t-butoxycarbonyl-piperidyl-4-carboxylic acid (1b) (3.0 g, 13 mmol) was dissolved in dichloromethane (100 mL), to which 4-(1,3-dioxolan-2-yl)aniline (1a) (2.2 g, 13 mmol), HATU (5.5 g, 14 mmol) and N,N-diisopropylethylamine (8.5 g, 65 mmol) were sequentially added, followed by a reaction at room temperature for 3 hours. The reaction solution was extracted and partitioned by addition of methylene chloride (50 ml) and water (50 ml). The aqueous phase was extracted with methylene chloride (20 mL×1). The organic phases were combined. The organic phase was dried over anhydrous sodium sulfate, and concentrated. The residue was purified by silica gel column chromatography (ethyl acetate:petroleum ether (v/v) = 1:9 to 1:0) to give the yellow oily tert-butyl 4-[[4-(1,3-dioxolan-2-yl)phenyl]carbamoyl]piperidine-1-carboxylate (1c) (5.0 g, yield: 100%).
LCMS m/z =399.3 [M+23].

### Step 2: N-(4-formylphenyl)piperidine-4-carboxamide (1d)

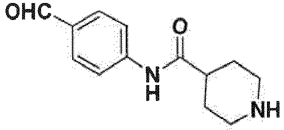

Tert-butyl 4-[[4-(1,3-dioxolan-2-yl)phenyl]carbamoyl]piperidine-1-carboxylate (1c) (5.0 g, 13 mmol) was dissolved in methylene chloride (15 mL), to which trifluoroacetic acid (7.6 g, 66 mmol) was added, followed by a reaction at room temperature for 2 hours. The reaction solution was adjusted to pH 8∼9 with aqueous ammonia, and extracted and partitioned by addition of methylene chloride (20 ml) and water (20 ml). The aqueous phase was extracted with methylene chloride (50 mL×6). The organic phases were combined. The organic phase was dried over anhydrous sodium sulfate, filtered, and the filtrate was concentrated under reduced pressure, to obtain N-(4-formylphenyl)piperidine-4-carboxamide (1d) as a yellow solid (0.550 g, yield 18%).
LCMS m/z =233.3[M+1].

### Step 3: [1-[3-[4-[(4-formylphenyl)carbamoyl]-1-piperidyl]-3-oxo-propyl]-4-piperidyl] N-(2-phenylphenyl)carbamate (Intermediate 1)

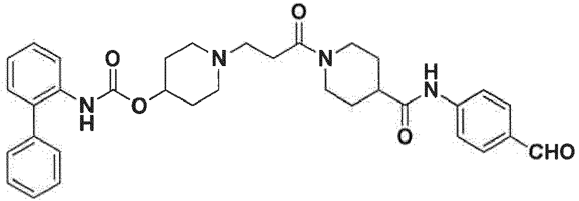

N-(4-formylphenyl)piperidine-4-carboxamide (1d) (0.441 g, 1.90 mmol) was dissolved in methylene chloride (15 ml), to which 3-[4-[(2-phenylphenyl)carbamoyloxy]-1-piperidyl]propanoic acid (1e) (0.700 g, 1.90 mmol), HATU (1.08 g, 2.85 mmol) and N,N-diisopropylethylamine (1.96 g, 15.2 mmol) were sequentially added, followed by a reaction at room temperature for 3 hours. The reaction solution was extracted and partitioned by addition of methylene chloride (50 ml) and water (50 ml). The aqueous phase was extracted with methylene chloride (20 mL×1). The organic phases were combined. The organic phase was dried over anhydrous sodium sulfate, filtered, and the filtrate was concentrated under reduced pressure, The residue was purified by silica gel column chromatography (ethyl acetate:petroleum ether (v/v) = 1:1 to 1:0, methanol:methylene chloride (v/v)=3:97 to 1:19) to give [1-[3-[4-[(4-formylphenyl)carbamoyl]-1-piperidyl]-3-oxo-propyl]-4-piperidyl] N-(2-phenylphenyl)carbamate **(Intermediate 1)** as a yellow solid (0.470 g, yield 42.4%).
1H NMR (400 MHz, CDCl3) δ 9.90 (s, 1H), 8.27 (s, 1H), 8.06 (s, 1H), 7.96 - 7.68 (m, 4H), 7.49 (t, 2H), 7.45 - 7.39 (m, 1H), 7.39 - 7.32 (m, 3H), 7.25-7.19 (m, 1H), 7.19-7.10 (m, 1H), 6.64 (d, 1H), 4.86 (s, 1H), 4.60 (d, 1H), 3.93 (d, 1H), 3.17-3.06 (m, 2H), 3.01-2.91 (m, 3H), 2.86 - 2.67 (m, 5H), 2.66 - 2.48 (m, 2H), 2.26-2.08 (m, 2H), 2.00-1.85 (m, 5H).
LCMS m/z =583.3[M+1].

### Intermediate 2:

### [1-[3-[4-[(2-phenylphenyl)carbamoyloxy]-1-piperidyl]propanoyl]-4-piperidyl]methyl N-(4-formylphenyl)carbamate (Intermediate 2)

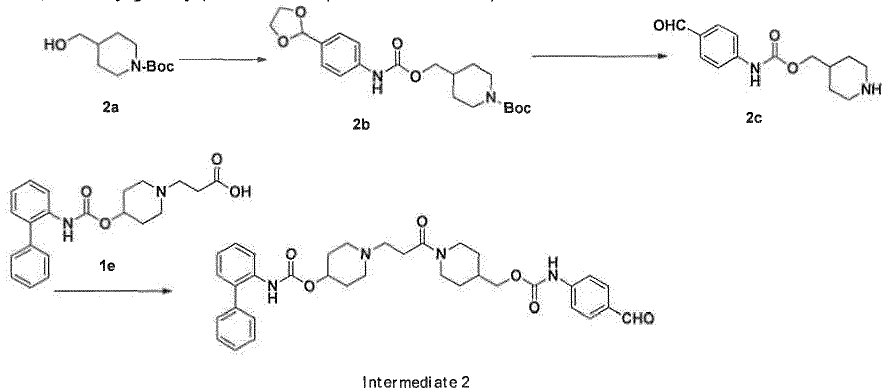

### Step 1: tert-butyl 4-[[4-(1,3-dioxolan-2-yl)phenyl]carbamoyloxymethyl]piperidine-1-carboxylate (2b)

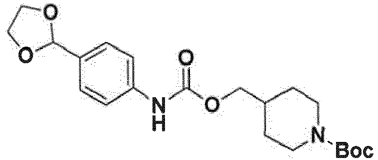

Tert-butyl 4-(hydroxymethyl)piperidine-1-carboxylate (2a) (1.0 g, 4.64 mmol) was dissolved in methylene chloride (20 ml), to which N,N-diisopropylethylamine (1.8 g, 13.9 mmol) was added, and then a solution of triphosgene (0.689 g, 2.32 mmol) in methylene chloride (10 ml) was added dropwise at 0°C, and the temperature was gradually elevated to room temperature to allow a reaction to proceed for 1 hour, to obtain Reaction solution 1. 4-(1,3-dioxolan-2-yl)aniline (1a) (0.767 g, 4.64 mmol) was dissolved in tetrahydrofuran (20 ml), to which N,N-diisopropylethylamine (1.8 g, 13.9 mmol) was added, then Reaction solution 1 was added dropwise at 0°C, and the temperature was gradually elevated to room temperature to allow a reaction to proceed for 1 hour. The reaction solution was concentrated, and extracted and partitioned by addition of methylene chloride (30 mL) and water (30 ml). The organic phase was dried over anhydrous sodium sulfate, filtered, and the filtrate was concentrated under reduced pressure. The residue was purified by silica gel column chromatography (ethyl acetate:petroleum ether (v/v) = 1:9 to 3:7) to give tert-butyl 4-[[4-(1,3-dioxolan-2-yl)phenyl]carbamoyloxymethyl]piperidine-1-carboxylate (2b) as a white solid (0.850 g, yield: 45%).
LCMS m/z =429.3 [M+23].

### Step 2: 4-piperidylmethyl N-(4-formylphenyl)carbamate (2c)

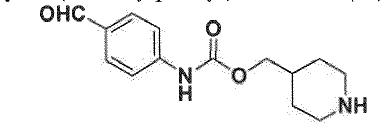

Tert-butyl 4-[[4-(1,3-dioxolan-2-yl)phenyl]carbamoyloxymethyl]piperidine-1-carboxylate (2b) (0.850 g, 2.09 mmol) was dissolved in methylene chloride (15 ml), to which trifluoroacetic acid (1.19 g, 10.5 mmol) was added, followed by a reaction at room temperature for 2 hours. The reaction solution was adjusted to pH 8 ∼9 with aqueous ammonia, and extracted and partitioned by addition of methylene chloride (20 ml) and water (20 ml). The aqueous phase was extracted with methylene chloride (20 mL×1). The organic phases were combined. The organic phase was dried over anhydrous sodium sulfate, filtered, and the filtrate was concentrated under reduced pressure, to obtain 4-piperidylmethyl N-(4-formylphenyl)carbamate (2c) as a white solid (0.490 g, yield 89.3%).
LCMS m/z =263.1[M+1].

### Step 3:

### [1-[3-[4-[(2-phenylphenyl)carbamoyloxy]-1-piperidyl]propanoyl]-4-piperidyl]methyl N-(4-formylphenyl)carbamate (Intermediate 2)

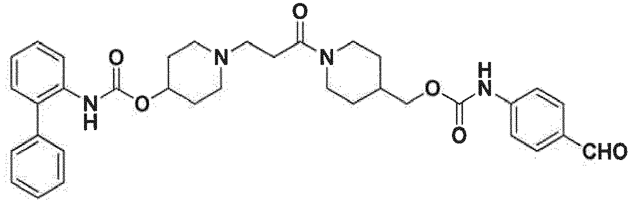

4-piperidylmethyl N-(4-formylphenyl)carbamate (2c) (0.427 g, 1.63 mmol) was dissolved in methylene chloride (15 ml), to which 3-[4-[(2-phenylphenyl)carbamoyloxy]-1-piperidyl]propanoic acid (0.600 g, 1.63 mmol), HATU (0.929 g, 2.44 mmol) and 2-isopropylethylamine (1.68 g, 13.0 mmol) were sequentially added, followed by a reaction at room temperature for 3 hours. The reaction solution was extracted and partitioned by addition of methylene chloride (50 ml) and water (50 ml). The aqueous phase was extracted with methylene chloride (20 mL×1). The organic phases were combined. The organic phase was dried over anhydrous sodium sulfate, and concentrated. The residue was purified by silica gel column chromatography (ethyl acetate:petroleum ether (v/v) = 1:1 to 1:0, methanol:methylene chloride (v/v)=3:97 to 1:19) to give [1-[3-[4-[(2-phenylphenyl)carbamoyloxy]-1-piperidyl]propanoyl]-4-piperidyl]methyl N-(4-formylphenyl)carbamate (Intermediate 2) as a white solid (0.740 g, yield 74.2%).
1H NMR (400 MHz, CDCl3) δ 9.88 (s, 1H), 7.96 (d, 1H), 7.85 - 7.76 (m, 2H), 7.68 - 7.53 (m, 3H), 7.52 - 7.43 (m, 2H), 7.43 - 7.31 (m, 4H), 7.23 (dd, 1H), 7.20-7.12 (m, 1H), 6.75 (s, 1H), 4.90 (s, 1H), 4.57 (d, 1H), 4.06 (d, 2H), 3.86 (d, 1H), 3.28 - 2.82 (m, 10H), 2.82 - 2.71 (m, 1H), 2.64-2.51 (m, 1H), 2.18-2.06 (m, 2H), 2.00 - 1.85 (m, 3H), 1.73 (t, 2H).
LCMS m/z =613.2[M+1],

### Intermediate 3:

### [1-[3-[4-[(2-phenylphenyl)carbamoyloxy]-1-piperidyl]propanoyl]-4-piperidyl]methyl N-(2-chloro-4-formyl-5-methoxy-phenyl)carbamate (Intermediate 3)

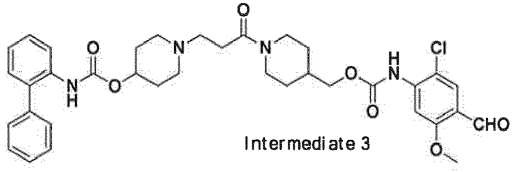

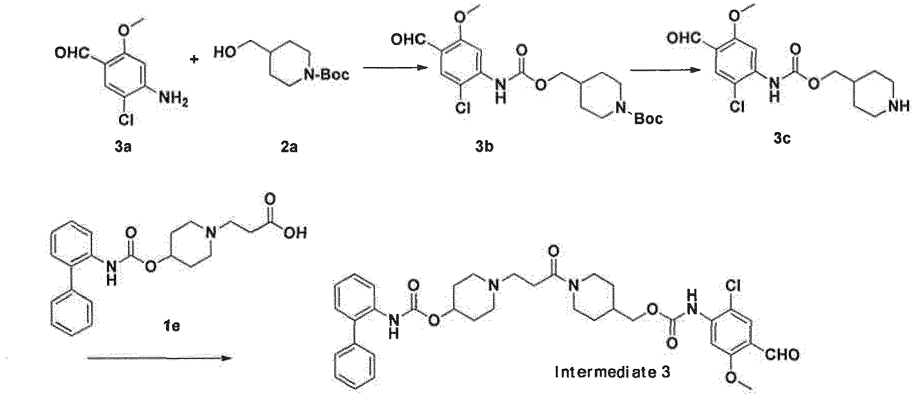

### Step 1: tert-butyl

### 4-[(2-chloro-4-formyl-5-methoxy-phenyl)carbamoyloxymethyl]piperidine-1-carboxyl ate (3b)

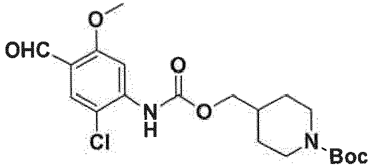

4-amino-5-chloro-2-methoxybenzaldehyde (3a) (6.9 g, 37.2 mmol) was dissolved in toluene (100 ml), to which triphosgene (5.51 g, 18.6 mmol) was added, followed by a reaction at 120°C for 2 hours, and the resultant was concentrated to obtain Reaction solution 1. Tert-butyl 4-(hydroxymethyl)piperidine-1-carboxylate (2a) (2.00 g, 9.29 mmol) was dissolved in tetrahydrofuran (50 ml), to which Reaction solution 1 and then triethylamine (3.76 g, 37.2 mmol) were added, followed by a reaction at 70°C for 2 hours. The reaction solution was concentrated, and the residue was purified by silica gel column chromatography (ethyl acetate:petroleum ether (v/v) = 1:9 to 3:7) to give yellow oily tert-butyl 4-[(2-chloro-4-formyl-5-methoxy-phenyl)carbamoyloxymethyl]piperidine-1-carboxyl ate (3b) (2.2 g, yield: 74%).
¹H NMR (400 MHz, CDCl₃) δ 10.29 (s, 1H), 8.05 (s, 1H), 7.83 (s, 1H), 7.41 (s, 1H), 4.16 (d, 2H), 4.09 (d, 2H), 3.95 (s, 3H), 2.73 (m, 2H), 1.96 - 1.83 (m, 1H), 1.75 (d, 2H), 1.46 (s, 9H), 1.32 - 1.18 (m, 2H).
LCMS m/z =449.3 [M+23].

### Step 2: 4-piperidylmethyl N-(2-chloro-4-formyl-5-methoxy-phenyl)carbamate (3c)

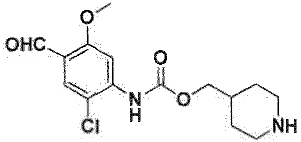

Tert-butyl 4-[(2-chloro-4-formyl-5-methoxy-phenyl)carbamoyloxymethyl]piperidine-1-carboxyl ate (3b) (2.2 g, 5.2 mmol) was dissolved in methylene chloride (15 ml), to which trifluoroacetic acid (2.9 g, 26 mmol) was added, followed by a reaction at room temperature for 2 hours. The reaction solution was adjusted to pH 8 ∼ 9 with aqueous ammonia, and extracted and partitioned by addition of methylene chloride (20 ml) and water (20 ml). The aqueous phase was extracted with methylene chloride (20 mL×1). The organic phases were combined. The organic phase was dried over anhydrous sodium sulfate, filtered, and the filtrate was concentrated under reduced pressure, to obtain 4-piperidylmethyl N-(2-chloro-4-formyl-5-methoxy-phenyl)carbamate (3c) as a yellow solid (1.6 g, yield 95%).
¹H NMR (400 MHz, CDCl₃) δ 10.29 (s, 1H), 8.05 (d, 1H), 7.82 (d, 1H), 7.42 (s, 1H), 4.09 (d, 2H), 3.95 (s, 3H), 3.20 (d, 2H), 2.69 (m, 2H), 1.95-1.83 (m, 1H), 1.80 (d, 2H), 1.42-1.30 (m, 2H).
LCMS m/z =327.2[M+1].

### Step 3:

### [1-[3-[4-[(2-phenylphenyl)carbamoyloxy]-1-piperidyl]propanoyl]-4-piperidyl]methyl N-(2-chloro-4-formyl-5-methoxy-phenyl)carbamate (Intermediate 3)

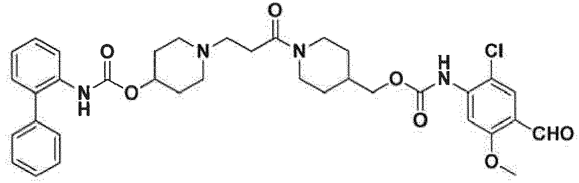

4-piperidylmethyl N-(2-chloro-4-formyl-5-methoxy-phenyl)carbamate (3c) (0.532 g, 1.63 mmol) was dissolved in methylene chloride (15 ml), to which 3-[4-[(3-phenylphenyl)carbamoyloxy]-1-piperidyl]propanoic acid (1e) (0.600 g, 1.63 mmol), HATU (0.929 g, 2.44 mmol) and 2-isopropylethylamine (1.68 g, 13.0 mmol) were sequentially added, followed by a reaction at room temperature for 3 hours. The reaction solution was extracted and partitioned by addition of methylene chloride (50 ml) and water (50 ml). The aqueous phase was extracted with methylene chloride (20 mL×1). The organic phases were combined. The organic phase was dried over anhydrous sodium sulfate, and concentrated. The residue was purified by silica gel column chromatography (ethyl acetate:petroleum ether (v/v) = 1:1 to 1:0, methanol:methylene chloride (v/v)=1:99 to 3:97) to give [1-[3-[4-[(2-phenylphenyl)carbamoyloxy]-1-piperidyl]propanoyl]-4-piperidyl]methyl N-(2-chloro-4-formyl-5-methoxy-phenyl)carbamate (Intermediate 3) as a yellow solid (1.0 g, yield 91%).
¹H NMR (400 MHz, CDCl₃) δ 10.29 (s, 1H), 8.04 (s, 2H), 7.82 (s, 1H), 7.52 - 7.44 (m, 2H), 7.45 - 7.38 (m, 2H), 7.38 - 7.32 (m, 3H), 7.22 (dd, 1H), 7.17-7.11 (m, 1H), 6.65 (s, 1H), 4.86 - 4.73 (m, 1H), 4.64 (d, 1H), 4.09 (d, 2H), 3.92 (d, 4H), 3.01 - 2.83 (m, 5H), 2.73 - 2.51 (m, 6H), 2.06-1.92 (m, 4H), 1.92-1.71 (m, 4H).
LCMS m/z =677.3[M+1].

### Example 1

### [1-[3-[4-[[4-[[[(2R)-2-hydroxy-2-(8-hydroxy-2-oxo-1H-quinolin-5-yl)ethyl]amino]me thyl]phenyl]carbamoyl]-1-piperidyl]-3-oxo-propyl]-4-piperidyl] N-(2-phenylphenyl)carbamate; ditrifluoroacetic acid (Compound 1)

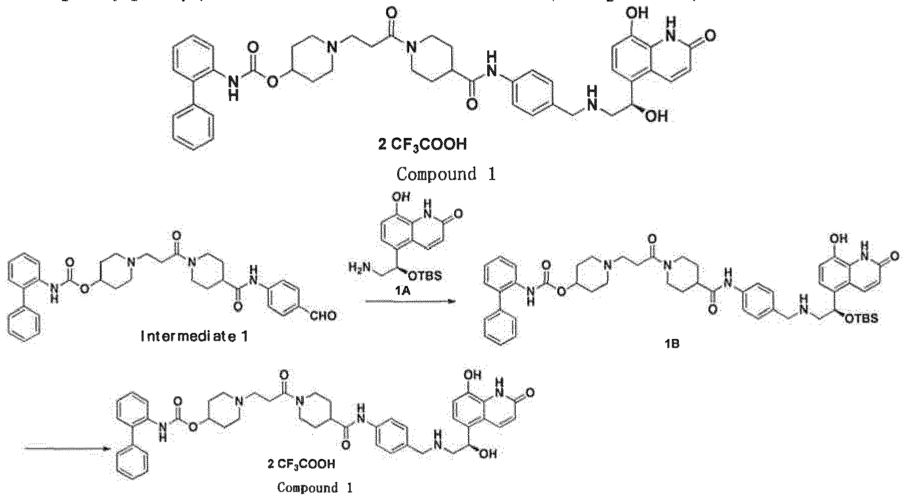

### Step 1:

### [1-[3-[4-[[4-[[[(2R)-2-[tert-butyl(dimethyl)silyl]oxy-2-(8-hydroxy-2-oxo-1H-quinolin -5-yl)ethyl]amino]methyl]phenyl]carbamoyl]-1-piperidyl]-3-oxo-propyl]-4-piperidyl] N-(2-phenylphenyl)carbamate (1B)

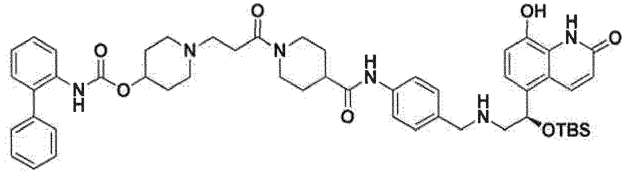

[1-[3-[4-[(4-formylphenyl)carbamoyl]-1-piperidyl]-3-oxo-propyl]-4-piperidyl] N-(2-phenylphenyl)carbamate **(Intermediate** 1) (0.470 g, 0.807 mmol) was dissolved in methanol (10 ml), to which 5-[(1R)-2-amino-1-[t-butyl(dimethyl)silyl]oxymethyl]-8-hydroxy-1H-quinolin-2-one (1A) (refer to WO2007102771A1 for its preparation) (0.270 g, 0.807 mmol) and anhydrous zinc chloride (0.440 g, 3.23 mmol) were added, followed by a reaction at 55°C for 1 h. Sodium cyanoborohydride (0.152 g, 2.42 mmol) was added, followed by a reaction at 55°C for 2 h. The reaction solution was extracted and partitioned by addition of methylene chloride (50 ml) and a saturated solution of sodium bicarbonate (20 ml). The aqueous phase was extracted with methylene chloride (30 mL×1). The organic phases were combined, dried over anhydrous sodium sulfate, filtered, and the filtrate was concentrated under reduced pressure, to obtain [1-[3-[4-[[4-[[[(2R)-2-[tert-butyl(dimethyl)silyl]oxy-2-(8-hydroxy-2-oxo-1H-qumolin -5-yl)ethyl]amino]methyl]phenyl]carbamoyl]-1-piperidyl]-3-oxo-propyl]-4-piperidyl] N-(2-phenylphenyl)carbamate (1B) as a yellow solid (0.80 g, yield 100%).
LCMS m/z =451.4[(M+2)/2].

### Step 2:

### [1-[3-[4-[[4-[[[(2R)-2-hydroxy-2-(8-hydroxy-2-oxo-1H-quinolin-5-yl)ethyl]amino]me thyl]phenyl]carbamoyl]-1-piperidyl]-3-oxo-propyl]-4-piperidyl] N-(2-phenylphenyl)carbamate; ditrifluoroacetic acid (Compound 1)

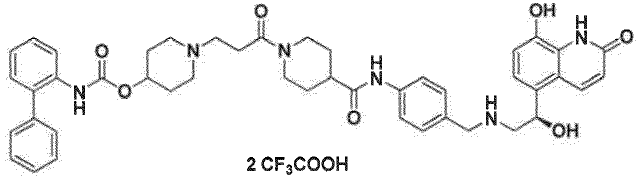

[1-[3-[4-[[4-[[[(2R)-2-[tert-butyl(dimethyl)silyl]oxy-2-(8-hydroxy-2-oxo-1H-quinolin -5-yl)ethyl]amino]methyl]phenyl]carbamoyl]-1-piperidyl]-3-oxo-propyl]-4-piperidyl] N-(2-phenylphenyl)carbamate (1B) (0.800 g, 0.888 mmol) was dissolved in tetrahydrofuran (5 ml), and triethylamine trihydrofluoride (1.43 g, 8.88 mmol) was added thereto, followed by a reaction at room temperature for 24 hours. The reaction solution was extracted and partitioned by addition of a 10% (v/v) methanol/methylene chloride solution (50 ml) and addition of a saturated solution of sodium bicarbonate to adjust the pH to about 8. The aqueous phase was extracted with a 10% (v/v) methanol/methylene chloride solution (20 mL×2). The organic phases were combined. The organic phases were washed with saturated brine (20 mL×1), dried over anhydrous sodium sulfate, filtered, and the filtrate was concentrated under reduced pressure. The residue was purified by preparative liquid column chromatography (liquid preparation condition: C18 reverse preparative column; Mobile phase: 0.05% TFA in deionized water (A) and acetonitrile (B); Isocratic elution with B:A=25% for 20 min) to obtain [1-[3-[4-[[4-[[[(2R)-2-hydroxy-2-(8-hydroxy-2-oxo-1H-quinolin-5-yl)ethyl]amino]me thyl]phenyl]carbamoyl]-1-piperidyl]-3-oxo-propyl]-4-piperidyl] N-(2-phenylphenyl)carbamate; ditrifluoroacetic acid **(Compound 1)** as a white solid (0.060 g, yield 6.7%).
¹H NMR (400 MHz, CD₃OD) δ 8.22 (d, 1H), 7.67 (d, 2H), 7.54 (s, 1H), 7.48-7.43 (m, 3H), 7.43 - 7.28 (m, 7H), 7.26 (d, 1H), 7.02 (d, 1H), 6.62 (d, 1H), 5.38 (t, 1H), 4.90 (s, 1H), 4.58 (d, 1H), 4.26 (s, 2H), 3.99 (d, 1H), 3.48 (s, 2H), 3.41 (s, 2H), 3.26 - 3.13 (m, 4H), 3.13 (s, 1H), 2.94 (s, 2H), 2.84-2.62 (m, 2H), 2.16 (s, 1H), 1.06-1.90 (m, 4H), 1.86 - 1.59 (m, 3H).
¹⁹F NMR (376 MHz, CD₃OD) δ -74.83.
LCMS m/z =394.3[(M+2)/2].

### Example 2

### [1-[3-[4-[(2-phenylphenyl)carbamoyloxy]-1-piperidyl]propanoyl]-4-piperidyl]methyl N-[4-[[[(2R)-2-hydroxy-2-(8-hydroxy-2-oxo-1H-quinolin-5-yl)ethyl]amino]methyl]p henyl]carbamate; ditrifluoroacetic acid (Compound 2)

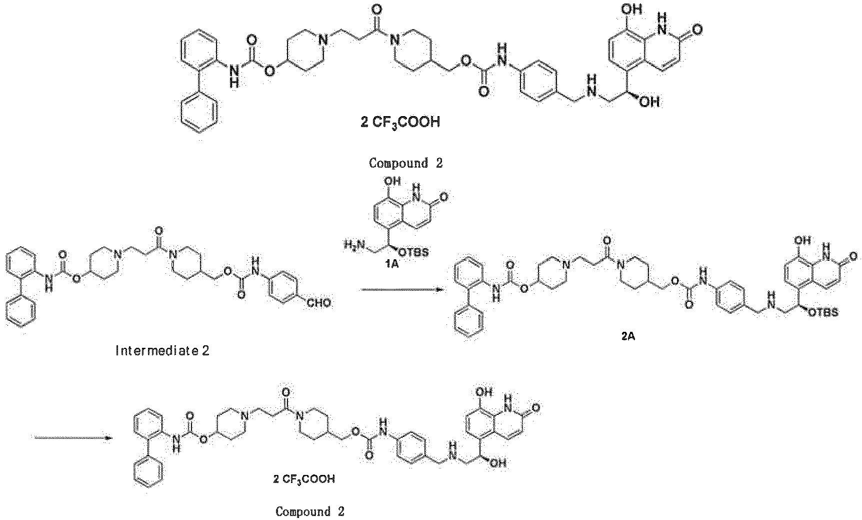

### Step 1:

### [1-[3-[4-[(2-phenylphenyl)carbamoyloxy]-1-piperidyl]propanoyl]-4-piperidyl]methyl N-[4-[[[(2R)-2-[tert-butyl(dimethyl)silyl]oxy-2-(8-hydroxy-2-oxo-1H-quinolin-5-yl)et hyl]amino]methyl]phenyl]carbamate (2A)

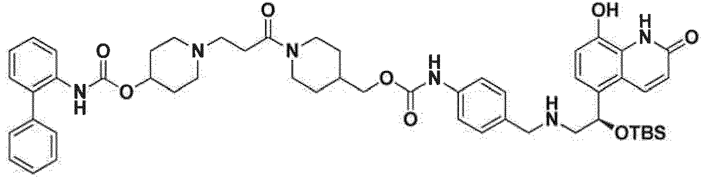

[1-[3-[4-[(2-phenylphenyl)carbamoyloxy]-1-piperidyl]propanoyl]-4-piperidyl]methyl N-(4-formylphenyl)carbamate (Intermediate 2) (0.400 g, 0.653 mmol) was dissolved in methanol (10 ml), to which 5-[(1R)-2-amino-1-[t-butyl(dimethyl)silyl]oxymethyl]-8-hydroxy-1H-quinolin-2-one (1A) (0.262 g, 0.783 mmol) and anhydrous zinc chloride (0.356 g, 2.61 mmol) were added, followed by a reaction at 55°C for 1 h. Sodium cyanoborohydride (0.123 g, 1.96 mmol) was added, followed by a reaction at 55°C for 2 h. The reaction solution was extracted and partitioned by addition of methylene chloride (50 ml) and a saturated solution of sodium bicarbonate (20 ml). The aqueous phase was extracted with methylene chloride (30 mL×1). The organic phases were combined, dried over anhydrous sodium sulfate, filtered, and the filtrate was concentrated under reduced pressure, to obtain [1-[3-[4-[(2-phenylphenyl)carbamoyloxy]-1-piperidyl]propanoyl]-4-piperidyl]methyl N-[4-[[[(2R)-2-[tert-butyl(dimethyl)silyl]oxy-2-(8-hydroxy-2-oxo-1H-quinolin-5-yl)et hyl]amino]methyl]phenyl]carbamate (2A) as a yellow solid (0.40 g, yield: 65.8%).
LCMS m/z =466.4[(M+2)/2].

### Step 2:

### [1-[3-[4-[(2-phenylphenyl)carbamoyloxy]-1-piperidyl]propanoyl]-4-piperidyl]methyl N-[4-[[[(2R)-2-hydroxy-2-(8-hydroxy-2-oxo-1H-quinolin-5-yl)ethyl]amino]methyl]p henyl]carbamate; ditrifluoroacetic acid (Compound 2)

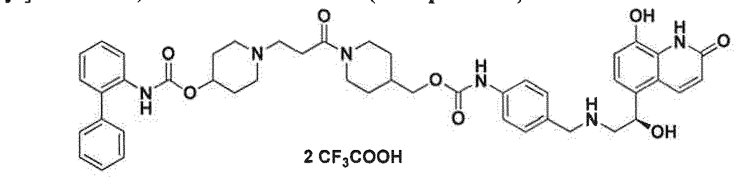

[1-[3-[4-[(2-phenylphenyl)carbamoyloxy]-1-piperidyl]propanoyl]-4-piperidyl]methyl N-[4-[[[(2R)-2-[tert-butyl(dimethyl)silyl]oxy-2-(8-hydroxy-2-oxo-1H-quinolin-5-yl)et hyl]amino]methyl]phenyl]carbamate (2A) (0.400 g, 0.430 mmol) was dissolved in tetrahydrofuran (5 ml), and triethylamine trihydrofluoride (0.692 g, 4.30 mmol) was added thereto, followed by a reaction at room temperature for 24 hours. The reaction solution was extracted and partitioned by addition of a 10% (v/v) methanol/methylene chloride solution (50 ml) and addition of a saturated solution of sodium bicarbonate to adjust the pH to about 8. The aqueous phase was extracted with a 10% (v/v) methanol/methylene chloride solution (20 mL×2). The organic phases were combined. The organic phases were washed with saturated brine (20 mL×1), dried over anhydrous sodium sulfate, filtered, and the filtrate was concentrated under reduced pressure. The residue was purified by preparative liquid column chromatography (liquid preparation condition: C18 reverse preparative column; Mobile phase: 0.05% TFA in deionized water (A) and acetonitrile (B); Isocratic elution with B:A=25% for 20 min) to obtain [1-[3-[4-[(2-phenylphenyl)carbamoyloxy]-1-piperidyl]propanoyl]-4-piperidyl]methyl N-[4-[[[(2R)-2-hydroxy-2-(8-hydroxy-2-oxo-1H-quinolin-5-yl)ethyl]amino]methyl]p henyl]carbamate; ditrifluoroacetic acid **(Compound 2)** as a white solid (0.050 g, yield 11%).
¹H NMR (400 MHz, CD₃OD) δ 8.20 (d, 1H), 7.54 (d, 3H), 7.47 - 7.22 (m, 11H), 7.02 (d, 1H), 6.63 (d, 1H), 5.37 (t, 1H), 4.90 (s, 1H), 4.56 (d, 1H), 4.24 (s, 2H), 4.05 (d,2H), 3.94 (d, 1H), 3.59 (s, 1H), 3.48 (d, 1H), 3.39 (s, 2H), 3.22 - 3.03 (m, 5H), 2.92 (s, 2H), 2.70 (t, 1H), 2.22-1.95 (m, 4H), 1.93-1.70 (m, 3H), 1.40 - 1.10 (m, 2H).
¹⁹F NMR (376 MHz, CD₃OD) δ -75.49.
LCMS m/z =817.4[M+1].

### Free base of Compound 2:

### [1-[3-[4-[(2-phenylphenyl)carbamoyloxy]-1-piperidyl]propanoyl]-4-piperidyl]methyl N-[4-[[[(2R)-2-hydroxy-2-(8-hydroxy-2-oxo-1H-quinolin-5-yl)ethyl]amino]methyl]p henyl]carbamate

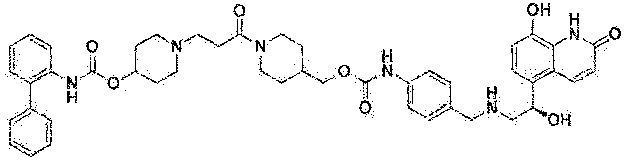

Compound 2 (0.040 g, 0.038 mmol) was dissolved in methylene chloride (10 ml), a saturated solution of sodium bicarbonate was added thereto, followed by mixing for about 15 min. The reaction solution was at a pH of 8 to 9 as measured, and then partitioned. The aqueous layer was extracted with methylene chloride. The organic layers were combined, dried over anhydrous sodium sulfate, filtered, and the filtrate was concentrated under reduced pressure, to obtain **the free base of Compound 2** as a light-yellow solid (0.025 g, yield 80.6%).
¹H NMR (400 MHz, CD₃OD) δ 8.27 (d, 1H), 7.59 (d, 1H), 7.48 - 7.36 (m, 8H), 7.34-7.21 (m, 4H), 7.22 (d, 1H), 7.00 (d, 1H), 6.63 (d, 1H), 5.24 (m, 1H), 4.65 (m, 1H), 4.58 (d, 1H), 4.07 (m, 3H), 3.84 (m, 2H), 3.17 (m,1H), 2.95-2.83 (m, 2H), 2.72-2.61 (m, 7H), 2.38 (t, 2H), 2.02 (m, 1H), 1.88 (m, 4H), 1.66 (m, 2H), 1.40-1.21 (m, 2H).
LCMS m/z =817.4[M+1].

### Example 3

### [1-[3-[4-[(2-phenylphenyl)carbamoyloxy]-1-piperidyl]propanoyl]-4-piperidyl]methyl N-[2-chloro-4-[[[(2R)-2-hydroxy-2-(8-hydroxy-2-oxo-1H-quinolin-5-yl)ethyl]amino] methyl]-5-methoxy-phenyl]carbamate; di(2,2,2-trifluoroacetic acid) (Compound 3)

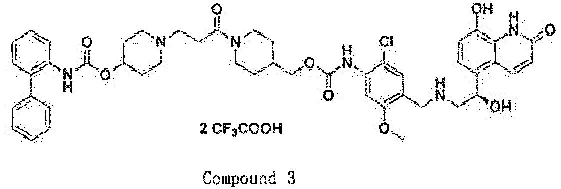

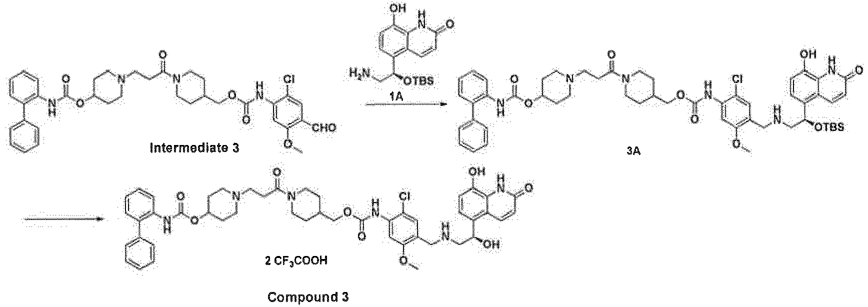

### Step 1:

### [1-[3-[4-[(2-phenylphenyl)carbamoyloxy]-1-piperidyl]propanoyl]-4-piperidyl]methyl N-[4-[[[(2R)-2-[tert-butyl(dimethyl)silyl]oxy-2-(8-hydroxy-2-oxo-1H-quinolin-5-yl)et hyl]amino]methl]-2-chloro-5-methox-henyl]carbamate (3A)

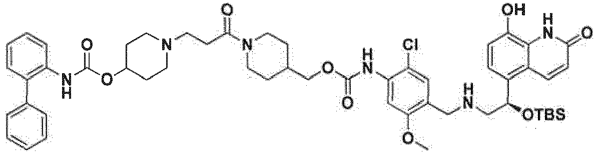

[1-[3-[4-[(2-phenylphenyl)carbamoyloxy]-1-piperidyl]propanoyl]-4-piperidyl]methyl N-(2-chloro-4-formyl-5-methoxy-phenyl)carbamate **(Intermediate 3)** (1.00 g, 1.48 mmol) was dissolved in methanol (10 ml), to which 5-[(1R)-2-amino-1-[t-butyl(dimethyl)silyl]oxymethyl]-8-hydroxy-1H-quinolin-2-one (1A) (0.494 g, 1.48 mmol) and anhydrous zinc chloride (0.805 g, 5.91 mmol) were added, followed by a reaction at 55°C for 1 h. Sodium cyanoborohydride (0.278 g, 4.43 mmol) was added, followed by a reaction at 55°C for 2 h. The reaction solution was extracted and partitioned by addition of methylene chloride (50 ml) and a saturated solution of sodium bicarbonate (20 ml). The aqueous phase was extracted with methylene chloride (30 mL×1). The organic phases were combined, dried over anhydrous sodium sulfate, filtered, and the filtrate was concentrated under reduced pressure, to obtain [1-[3-[4-[(2-phenylphenyl)carbamoyloxy]-1-piperidyl]propanoyl]-4-piperidyl]methyl N-[4-[[[(2R)-2-[tert-butyl(dimethyl)silyl]oxy-2-(8-hydroxy-2-oxo-1H-quinolin-5-yl)et hyl]amino]methyl]-2-chloro-5-methoxy-phenyl]carbamate (3A) as a yellow solid (0.80 g, yield: 54.4%).
LCMS m/z =498.4[(M+2)/2].

### Step 2:

### [1-[3-[4-[(2-phenylphenyl)carbamoyloxy]-1-piperidyl]propanoyl]-4-piperidyl]methyl N-[2-chloro-4-[[[(2R)-2-hydroxy-2-(8-hydroxy-2-oxo-1H-quinolin-5-yl)ethyl]amino] methyl]-5-methoxy-phenyl]carbamate; ditrifluoroacetic acid (Compound 3)

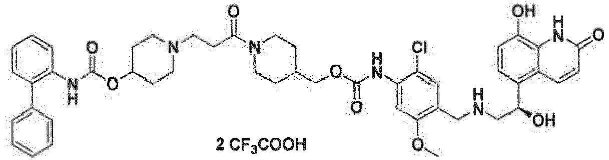

[1-[3-[4-[(2-phenylphenyl)carbamoyloxy]-1-piperidyl]propanoyl]-4-piperidyl]methyl N-[4-[[[(2R)-2-[tert-butyl(dimethyl)silyl]oxy-2-(8-hydroxy-2-oxo-1H-quinolin-5-yl)et hyl]amino]methyl]-2-chloro-5-methoxy-phenyl]carbamate (3A) (0.800 g, 0.803 mmol) was dissolved in tetrahydrofuran (5 ml), and triethylamine trihydrofluoride (1.3 g, 8.03 mmol) was added thereto, followed by a reaction at room temperature for 24 hours. The reaction solution was extracted and partitioned by addition of a 10% (v/v) methanol/methylene chloride solution (50 ml) and addition of a saturated solution of sodium bicarbonate to adjust the pH to about 8. The aqueous phase was extracted with a 10% (v/v) methanol/methylene chloride solution (20 mL×2). The organic phases were combined. The organic phases were washed with saturated brine (20 mL×1), dried over anhydrous sodium sulfate, filtered, and the filtrate was concentrated under reduced pressure. The residue was purified by preparative liquid column chromatography (liquid preparation condition: C18 reverse preparative column; Mobile phase: 0.05% TFA in deionized water (A) and acetonitrile (B); Isocratic elution with B:A=25% for 20 min) to obtain [1-[3-[4-[(2-phenylphenyl)carbamoyloxy]-1-piperidyl]propanoyl]-4-piperidyl]methyl N-[2-chloro-4-[[[(2R)-2-hydroxy-2-(8-hydroxy-2-oxo-1H-quinolin-5-yl)ethyl]amino] methyl]-5-methoxy-phenyl]carbamate; ditrifluoroacetic acid **(Compound 3)** as a white solid (0.450 g, yield 50.5%).
¹H NMR (400 MHz, CD₃OD) δ 8.20 (d, 1H), 7.76 (s, 1H), 7.54 (s, 1H), 7.48 - 7.23 (m, 10H), 7.03 (d, 1H), 6.64 (d, 1H), 5.40 (dd, 1H), 4.90 (s, 1H), 4.57 (d, 1H), 4.27 (s, 2H), 4.10 (d, 2H), 3.99 - 3.85 (m, 4H), 3.66 - 3.34 (m, 4H), 3.26 - 3.00 (m, 5H), 2.92 (s, 2H), 2.71 (t, 1H), 2.23 - 1.94 (m, 4H), 1.94-1.68 (m, 3H), 1.40-1.12 (m, 2H).
¹⁹F NMR (376 MHz, CD₃OD) δ -75.03.
LCMS m/z =441.3[(M+2)/2].

### Example 4

### [(3R)-1-[3-[4-[(2-phenylphenyl)carbamoyloxy]-1-piperidyl]propanoyl]pyrrolidin-3-yl ]methyl N-[2-chloro-4-[[[(2R)-2-hydroxy-2-(8-hydroxy-2-oxo-1H-quinolin-5-yl)ethyl]amino]methyl]-5-methoxy-phenyl]carbamate; ditrifluoroacetic acid

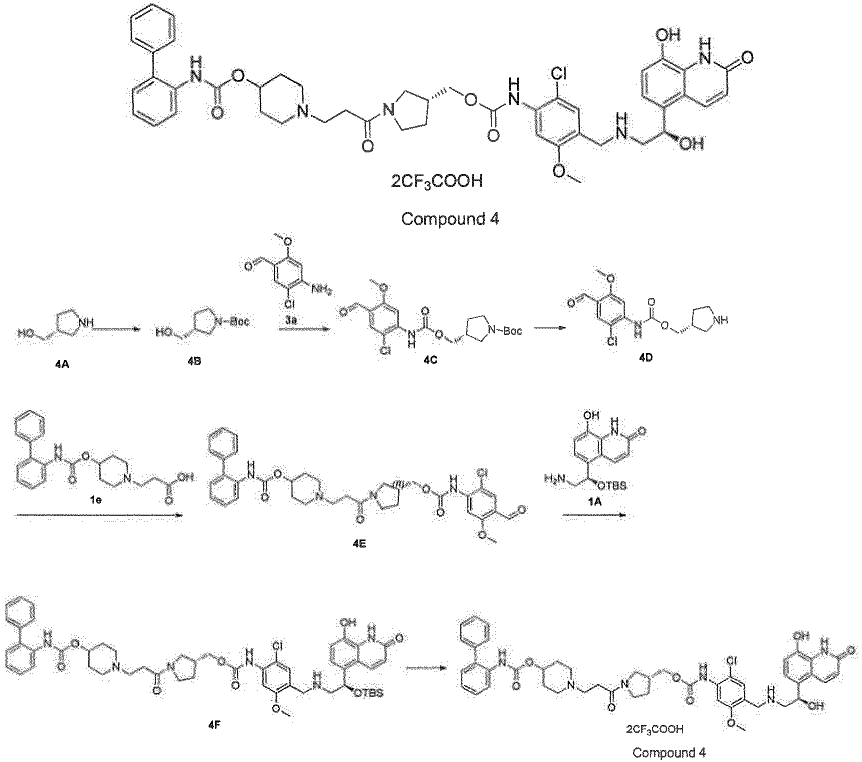

### Step 1: tert-butyl (3R)-3-(hydroxymethyl)pyrrolidine-1-carboxylate (4B)

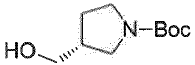

(R)-pyrrolidin-3-ylmethanol (4A) (2 g, 19.77 mmol) was dissolved in acetonitrile (10 ml), and di(tert-butyl) dicarbonate (4.75 g, 21.75 mmol) was added thereto, followed by a reaction at room temperature overnight. The reaction solution was directly concentrated under reduced pressure, to obtain tert-butyl (3R)-3-(hydroxymethyl)pyrrolidine-1-carboxylate (4B) as a colorless viscous liquid (3.97 g, yield 100.00%).
LCMS m/z =224.2[M+23].

### Step 2: tert-butyl (3R)-3-[(2-chloro-4-formyl-5-methoxy-phenyl)carbamoyloxymethyl] pyrrolidine-1-carboxylate (4C)

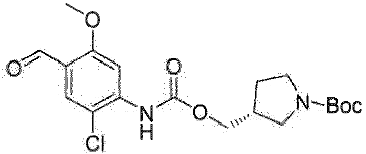

4-amino-5-chloro-2-methoxybenzaldehyde (3a) (462 mg, 2.49 mmol) was dissolved in toluene (15 ml), triethylamine (630 mg, 6.22 mmol) was added thereto, and then triphosgene (554 mg, 1.87 mmol) was added under N₂ protection, followed by a reaction at 120°C for 1 hour. After cooling to room temperature, Reaction Solution 1 was obtained. Tert-butyl (3R)-3-(hydroxymethyl)pyrrolidine-1-carboxylate (4B) (500 mg, 2.49 mmol) was dissolved in tetrahydrofuran (15 ml), to which triethylamine (630 mg, 6.22 mmol) and then Reaction Solution 1 were added, followed by a reaction at 85°C for 3 hours. The reaction solution was cooled to room temperature, and water (50 ml) was added thereto, which was extracted with ethyl acetate (100 mL×2). The organic phases were combined, dried over anhydrous sodium sulfate, filtered, and the filtrate was concentrated under reduced pressure, to obtain tert-butyl (3R)-3-[(2-chloro-4-formyl-5-methoxy-phenyl)carbamoyloxymethyl] pyrrolidine-1-carboxylate (4C) as a brown liquid, which was directly used in the next step without purification.
LCMS m/z =451.4[M+39].

### Step 3: [(3R)-pyrrolidin-3-yl]methyl N-(2-chloro-4-formyl-5-methoxy-phenyl) carbamate (4D)

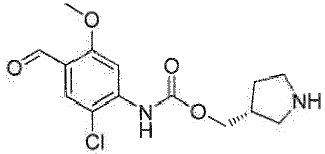

Tert-butyl (3R)-3-[(2-chloro-4-formyl-5-methoxy-phenyl)carbamoyloxymethyl] pyrrolidine-1-carboxylate (4C) obtained in Step 2 was dissolved in methylene chloride (15 ml), to which trifluoroacetic acid (2 ml) was added, followed by a reaction at room temperature for 4 hours. The reaction solution was concentrated under reduced pressure to remove methylene chloride. A saturated solution of sodium bicarbonate (50 ml) was added to the residue to adjust the pH of the solution to 7∼8, which was extracted with methylene chloride (70 mL×2). The organic phases were combined, dried over anhydrous sodium sulfate, filtered, and the filtrate was concentrated under reduced pressure. The residue was purified by silica gel column chromatography (the eluent was methylene chloride:methanol (v:v)=30:1 ∼ 10:1) to obtain [(3R)-pyrrolidin-3-yl]methyl N-(2-chloro-4-formyl-5-methoxy-phenyl) carbamate (4D) as a light-yellow solid (500 mg, total yield of the two steps: 64.35%).
LCMS m/z =313.2[M+1].

### Step 4:

### [(3R)-1-[3-[4-[(2-phenylphenyl)carbamoyloxy]-1-piperidyl]propanoyl]pyrrolidin-3-yl ]methyl N-(2-chloro-4-formyl-5-methoxy-phenyl)carbamate (4E)

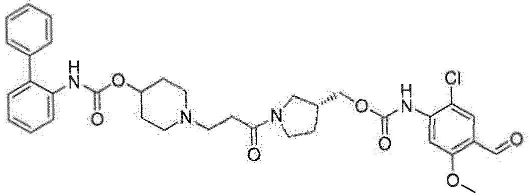

[(3R)-pyrrolidin-3-yl]methyl N-(2-chloro-4-formyl-5-methoxy-phenyl) carbamate (4D) (500 mg, 1.6 mmol) was dissolved in methylene chloride (20 ml), to which 3-[4-[(2-phenylphenyl)carbamoyloxy]-1-piperidyl]propanoic acid (1e) (708 mg, 1.92 mmol), HATU (912 mg, 2.4 mmol) and diisopropylethylamine (413 mg, 3.2 mmol) were added, followed by a reaction at room temperature for 3 hours. Water (50 ml) was added to the reaction solution, which was extracted with methylene chloride (70 mL×2). The organic phases were combined, dried over anhydrous sodium sulfate, filtered, and the filtrate was concentrated under reduced pressure, The residue was purified by silica gel column chromatography (the eluent was petroleum ether: ethyl acetate (v/v) = 6:1 to 1:1) to give [(3R)-1-[3-[4-[(2-phenylphenyl)carbamoyloxy]-1-piperidyl]propanoyl]pyrrolidin-3-yl ]methyl N-(2-chloro-4-formyl-5-methoxy-phenyl)carbamate (4E) as a yellow solid (600 mg, yield 57.14%).
LCMS m/z =663.3[M+1].

### Step 5:

### [(3R)-1-[3-[4-[(2-phenylphenyl)carbamoyloxy]-1-piperidyl]propanoyl]pyrrolidin-3-yl ]methyl N-[4-[[[(2R)-2-[tert-butyl(dimethyl)silyl]oxy-2-(8-hydroxy-2-oxo-1H-quinolin-5-yl)ethyl]aminojmethyl]-2-chloro-5-methoxy-phenyl]carbamate (4F)

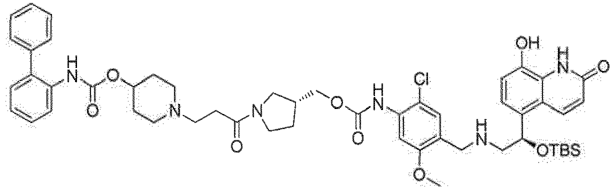

[(3R)-1-[3-[4-[(2-phenylphenyl)carbamoyloxy]-1-piperidyl]propanoyl]pyrrolidin-3-yl ]methyl N-(2-chloro-4-formyl-5-methoxy-phenyl)carbamate (4E) (600 mg, 0.91 mmol) was dissolved in methanol (15 ml), to which 5-[(1R)-2-amino-1-[t-butyl(dimethyl)silyl]oxymethyl]-8-hydroxy-1H-quinolin-2-one (1A) (360 mg, 1.08 mmol) and anhydrous zinc chloride (496 mg, 3.04 mmol) were added, followed by a reaction at 45°C for 1 h. Sodium cyanoborohydride (172 mg, 2.73 mmol) was added, followed by a reaction at 45°C for 1 h. After cooling to room temperature, water (100 ml) was added to the reaction solution, which was extracted with methylene chloride (100 mL×2). The organic phases were combined, dried over anhydrous sodium sulfate, filtered, and the filtrate was concentrated under reduced pressure, to obtain [(3R)-1-[3-[4-[(2-phenylphenyl)carbamoyloxy]-1-piperidyl]propanoyl]pyrrolidin-3-yl ]methyl N-[4-[[[(2R)-2-[tert-butyl(dimethyl)silyl]oxy-2-(8-hydroxy-2-oxo-1H-quinolin-5-yl)ethyl]amino]methyl]-2-chloro-5-methoxy-phenyl]carbamate (4F) as a white solid, which was directly used in the next step without purification.
LCMS m/z =491.4 [(M+2)/2].

### Step 6:

### [(3R)-1-[3-[4-[(2-phenylphenyl)carbamoyloxy]-1-piperidyl]propanoyl]pyrrolidin-3-yl ]methyl N-[2-chloro-4-[[[(2R)-2-hydroxy-2-(8-hydroxy-2-oxo-1H-quinolin-5-yl) ethyl]amino]methyl]-5-methoxy-phenyl]carbamate; di(2,2,2-trifluoro)acetic acid (Compound 4)

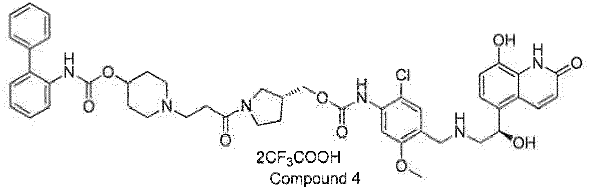

The [(3R)-1-[3-[4-[(2-phenylphenyl)carbamoyloxy]-1-piperidyl]propanoyl]pyrrolidin-3-yl ]methyl N-[4-[[[(2R)-2-[tert-butyl(dimethyl)silyl]oxy-2-(8-hydroxy-2-oxo-1H-quinolin-5-yl)ethyl]amino]methyl]-2-chloro-5-methoxy-phenyl]carbamate (4F) obtained in Step 5 was dissolved in tetrahydrofuran (15 ml), and triethylamine trihydrofluoride (1.5 ml) was added thereto, followed by a reaction at room temperature for 6 hours. Tetrahydrofuran was removed by concentration under reduced pressure, and the residue was adjusted to pH 9 with a saturated aqueous solution of sodium bicarbonate. The solution was extracted with methylene chloride (50 mL×2). The organic phases were combined, dried over anhydrous sodium sulfate, filtered, and the filtrate was concentrated under reduced pressure. The residue was purified by preparative liquid column chromatography (liquid preparation condition: C18 reverse preparative column; Mobile phase: 0.05% TFA in deionized water (A) and acetonitrile (B); Isocratic elution with B:A=25% for 20 min) to obtain [(3R)-1-[3-[4-[(2-phenylphenyl)carbamoyloxy]-1-piperidyl]propanoyl]pyrrolidin-3-yl ]methyl N-[2-chloro-4-[[[(2R)-2-hydroxy-2-(8-hydroxy-2-oxo-1H-quinolin-5-yl) ethyl]amino]methyl]-5-methoxy-phenyl]carbamate; ditrifluoroacetic acid (Compound 4) as a white solid (150 mg, yield 19.03%).
1H NMR (400 MHz, CD₃OD) δ 8.20 (dd,1H), 7.74 (d, 1H), 7.53 (d, 1H), 7.46 (s, 1H), 7.37 (m, 6H), 7.26 (m, 3H), 7.02 (d,1H), 6.77 - 6.57 (m, 1H), 5.40 (dd,1H), 4.89 (m, 1H), 4.26 (m, 2H), 4.31 - 4.16 (m, 4H), 3.89 (s, 3H), 3.70 - 3.58 (m, 2H), 3.51 - 3.34 (m, 4H), 3.21 (t, 2H), 3.09 (d, 2H), 2.98 - 2.55 (m, 3H), 2.15 (m, 2H), 2.03 - 1.60 (m, 4H).
LCMS m/z =434.3 [(M+2)/2].

### Example 5

### [(3S)-1-[3-[4-[(2-phenylphenyl)carbamoyloxy]-1-piperidyl]propanoy1]pyrrolidin-3-y1 ]methyl N-[2-chloro-4-[[[(2R)-2-hydroxy-2-(8-hydroxy-2-oxo-1H-qunolin-5-y1) ethy1]amino]methy1]-5-methoxy-pheny1]carbamate;ditrifluoroacetic acid (Compound 5)

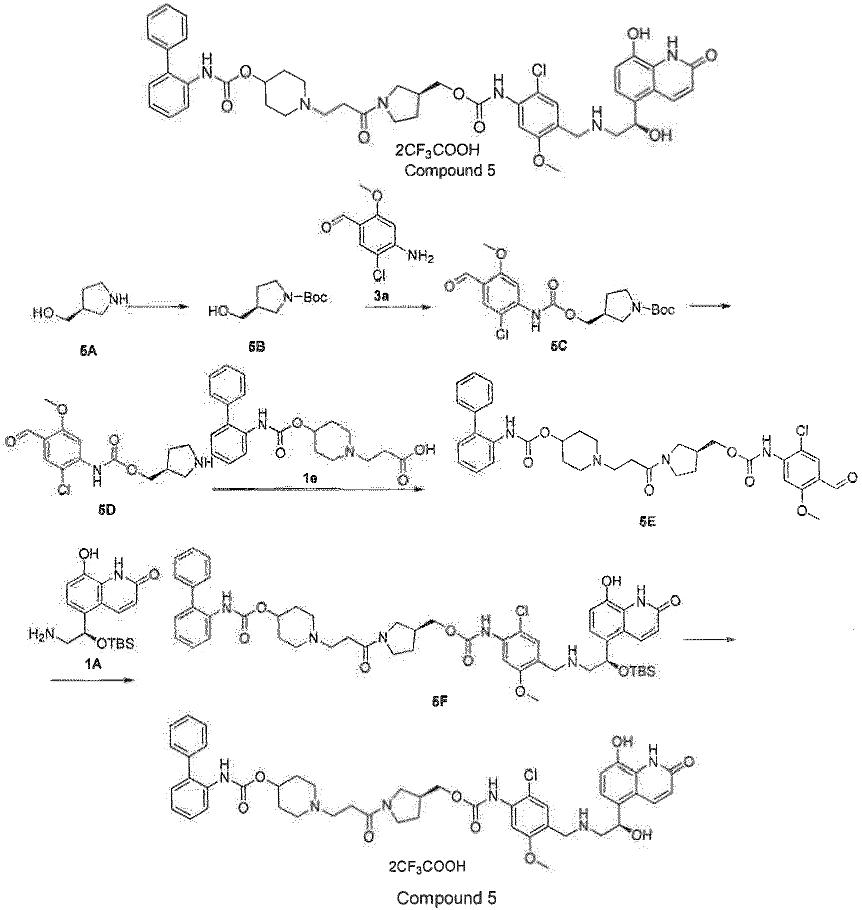

(S)-pyrrolidine-3-methanol, a stereoisomer of (R)-pyrrolidine-3-methanol (4A), was used as the starting material and followed the similar synthesis route as in Example 4, to obtain Compound 5.

### Step 1: tert-butyl (3S)-3-(hydroxymethyl)pyrrolidine-1-carboxylate (5B)

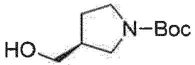

(S)-pyrrolidine-3-methanol (5A) (2 g, 19.77 mmol) was dissolved in acetonitrile (10 ml), and di(tert-butyl) dicarbonate (4.75 g, 21.75 mmol) was added thereto, followed by a reaction at room temperature overnight. The solvent was removed to dryness by rotary evaporation under reduced pressure, to obtain tert-butyl (3S)-3-(hydroxymethyl)pyrrolidine-1-carboxylate (5B) as a colorless viscous liquid (3.97 g, yield 100.00%).
LCMS m/z =224.2[M+23].

### Step 2: tert-butyl

### (3S)-3-[(2-chloro-4-formyl-5-methoxy-phenyl)carbamoyloxymethyl]pyrrolidine-1-car boxylate (5C)

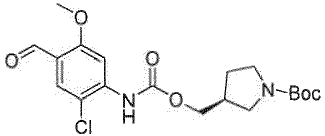

4-amino-5-chloro-2-methoxybenzaldehyde (3a) (1.38 g, 7.45 mmol) was dissolved in toluene (15 ml), triethylamine (0.75 g, 7.45 mmol) was added thereto, and then bis(trichloromethyl)carbonate (1.1 mg, 3.72 mmol) was added under N₂ protection, followed by a reaction at 120°C for 1 hour. A reaction solution was obtained after cooling to room temperature. Tert-butyl (3S)-3-(hydroxymethyl)pyrrolidine-1-carboxylate (5B) (0.6 g, 2.98 mmol) was dissolved in tetrahydrofuran (15 ml), to which triethylamine (0.75 g, 7.45 mmol) and the reaction solution were added, followed by a reaction at 85°C for 3 hours. The reaction solution was cooled to room temperature, and water (50 ml) was added thereto, which was extracted with ethyl acetate (100 mL×2).The organic phases were combined, dried over anhydrous sodium sulfate, filtered, and the filtrate was concentrated under reduced pressure, to obtain tert-butyl (3S)-3-[(2-chloro-4-formyl-5-methoxy-phenyl)carbamoyloxymethyl]pyrrolidine-1-car boxylate (5C) as a brown liquid, which was directly used in the next step.

### Step 3: [(3S)-pyrrolidin-3-yl]methyl

### N-(2-chloro-4-formyl-5-methoxy-phenyl)carbamate (5D)

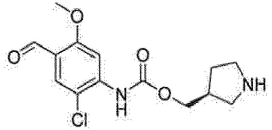

The tert-butyl (3S)-3-[(2-chloro-4-formyl-5-methoxy-phenyl)carbamoyloxymethyl]pyrrolidine-1-car boxylate (5C) obtained in the above step was dissolved in methylene chloride (15 ml), to which trifluoroacetic acid (2 ml) was added, followed by a reaction at room temperature for 4 hours. The solvent was removed by rotary evaporation under reduced pressure, and a saturated solution of sodium bicarbonate (50 ml) was added to adjust the pH of the solution to 7∼8, which was extracted with methylene chloride (70 mL×2). The organic phases were combined, dried over anhydrous sodium sulfate, filtered, and the filtrate was concentrated under reduced pressure. The residue was purified by silica gel column chromatography (the eluent was methylene chloride:methanol (v:v)=30:1 ~ 10:1) to obtain [(3S)-pyrrolidin-3-yl]methyl N-(2-chloro-4-formyl-5-methoxy-phenyl) carbamate (5D) as a light-yellow solid (500 mg, total yield of the two steps: 54.94%).
LCMS m/z =313.2[M+1].

### Step 4:

### [(3S)-1-[3-[4-[(2-phenylphenyl)carbamoyloxy]-1-piperidyl]propanoyl]pyrrolidin-3-yl ]methyl N-2-chloro-4-formyl-5-methoxy-phenyl)carbamate (5E)

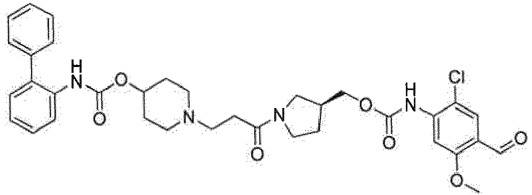

[(3S)-pyrrolidin-3-yl]methyl N-(2-chloro-4-formyl-5-methoxy-phenyl) carbamate (5D) (0.28 g, 0.9 mmol) was dissolved in methylene chloride (20 ml), to which 3-[4-[(2-phenylphenyl)carbamoyloxy]-1-piperidyl]propanoic acid (1e) (0.4 g, 1.08 mmol), HATU (0.51 g, 1.35 mmol) and diisopropylethylamine (0.23 g, 1.8 mmol) were added, followed by a reaction at room temperature for 3 hours. Water (50 ml) was added, and the solution was extracted with methylene chloride (70 mL×2). The organic phases were combined, dried over anhydrous sodium sulfate, filtered, and the filtrate was concentrated under reduced pressure. The residue was purified by silica gel column chromatography (the eluent was petroleum ether:ethyl acetate (v/v) = 6:1 to 1:1) to give [(3S)-1-[3-[4-[(2-phenylphenyl)carbamoyloxy]-1-piperidyl]propanoyl]pyrrolidin-3-yl ]methyl N-(2-chloro-4-formyl-5-methoxy-phenyl)carbamate (5E) as a yellow solid (0.4 g, yield 67.34%).
LCMS m/z =663.3[M+1].

### Step 5:

### [(3S)-1-[3-[4-[(2-phenylphenyl)carbamoyloxy]-1-piperidyl]propanoyl]pyrrolidin-3-yl] methyl N-[4-[[[(2R)-2-[tert-butyl(dimethyl)silyl]oxy-2-(8-hydroxy-2-oxo-1H-quinolin-5-yl)ethl]amino]methyl]-2-chloro-5-methoxy-phenyl]carbamate (5F)

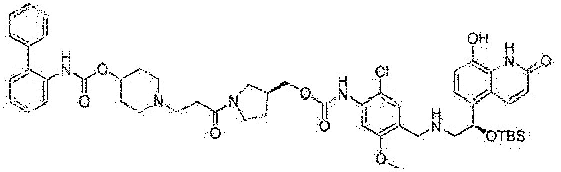

[(3S)-1-[3-[4-[(2-phenylphenyl)carbamoyloxy]-1-piperidyl]propanoyl]pyrrolidin-3-yl ]methyl N-(2-chloro-4-formyl-5-methoxy-phenyl)carbamate (5E) (0.4 g, 0.6 mmol) was dissolved in methanol (15 ml), to which 5-[(1R)-2-amino-1-[t-butyl(dimethyl)silyl]oxymethyl]-8-hydroxy-1H-quinolin-2-one (1A) (0.24 g, 0.72 mmol) and anhydrous zinc chloride (0.33 g, 2.4 mmol) were added, followed by a reaction at 45°C for 1 h. Sodium cyanoborohydride (0.13 g, 1.8 mmol) was added, followed by reaction at 45°C for 1 h. After cooling to room temperature, water (100 ml) was added, and the resultant was extracted with methylene chloride (100 mL×2). The organic phases were combined, dried over anhydrous sodium sulfate, filtered, and the filtrate was concentrated under reduced pressure, to obtain [(3S)-1-[3-[4-[(2-phenylphenyl)carbamoyloxy]-1-piperidyl]propanoyl]pyrrolidin-3-yl] methyl N-[4-[[[(2R)-2-[tert-butyl(dimethyl)silyl]oxy-2-(8-hydroxy-2-oxo-1H-quinolin-5-yl)ethyl]amino]methyl]-2-chloro-5-methoxy-phenyl]carbamate (5F) as a white solid, which was directly used in the next step.
LCMS m/z =491.4[(M+2)/2].

### Step 6:

### [(3S)-1-[3-[4-[(2-phenylphenyl)carbamoyloxy]-1-piperidyl]propanoyl]pyrrolidin-3-yl ]methyl N-[2-chloro-4-[[[(2R)-2-hydroxy-2-(8-hydroxy-2-oxo-1H-quinolin-5-yl) ethyl]amino]methyl]-5-methoxy-phenyl]carbamate; ditrifluoroacetic acid (Compound 5)

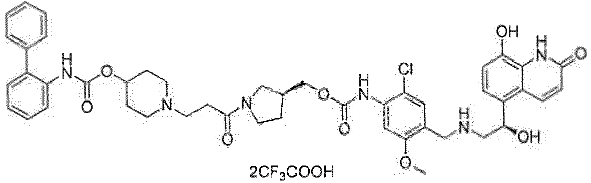

[(3S)-1-[3-[4-[(2-phenylphenyl)carbamoyloxy]-1-piperidyl]propanoyl]pyrrolidin-3-yl] methyl N-[4-[[[(2R)-2-[tert-butyl(dimethyl)silyl]oxy-2-(8-hydroxy-2-oxo-1H-quinolin-5-yl)ethyl]amino]methyl] -2-chloro-5-methoxy-phenyl] carbamate (5F) was dissolved in tetrahydrofuran (15 ml), and triethylamine trihydrofluoride (1.5 ml) was added thereto, followed by a reaction at room temperature for 6 hours. Tetrahydrofuran was removed by concentration under reduced pressure, and a saturated solution of sodium bicarbonate was added to the residue to adjust the pH to 9, which was extracted with methylene chloride (50 mL×2). The organic phases were combined, dried over anhydrous sodium sulfate, filtered, and the filtrate was concentrated under reduced pressure. The residue was used to obtain [(3S)-1-[3-[4-[(2-phenylphenyl)carbamoyloxy]-1-piperidyl]propanoyl]pyrrolidin-3-yl ]methyl N-[2-chloro-4-[[[(2R)-2-hydroxy-2-(8-hydroxy-2-oxo-1H-quinolin-5-yl) ethyl]amino]methyl]-5-methoxy-phenyl]carbamate; ditrifluoroacetic acid (Compound 5) as a white solid (0.11 g, total yield of the two steps: 21.15%).
1H NMR (400 MHz, CD₃OD) δ 8.20 (dd,1H), 7.74 (d, 1H), 7.53 (d, 1H), 7.46 (s, 1H), 7.37 (m, 6H), 7.26 (m, 3H), 7.02 (d,1H), 6.77 - 6.57 (m, 1H), 5.40 (dd,1H), 4.89 (m, 1H), 4.26 (m, 2H), 4.26 - 4.13 (m, 2H), 3.89 (s, 3H), 3.70 - 3.58 (m, 2H), 3.51 - 3.34 (m, 4H), 3.21 (t, 2H), 3.09 (d, 2H), 2.98 - 2.55 (m, 4H), 2.15 (m, 2H), 2.03 - 1.60 (m, 4H), 0.88 (m,1H).
LCMS m/z =434.3 [M/2+1].

### Example 6

### [1-[3-[4-[2-[[(2R)-2-hydroxy-2-(8-hydroxy-2-oxo-1H-quinolin-5-yl)ethyl]amino]ethy 1-methyl-carbamoyl]-1-piperidyl]-3-oxo-propyl]-4-piperidyl]N-(2-phenylphenyl)carba mate; ditrifluoroacetic acid (Compound 6)

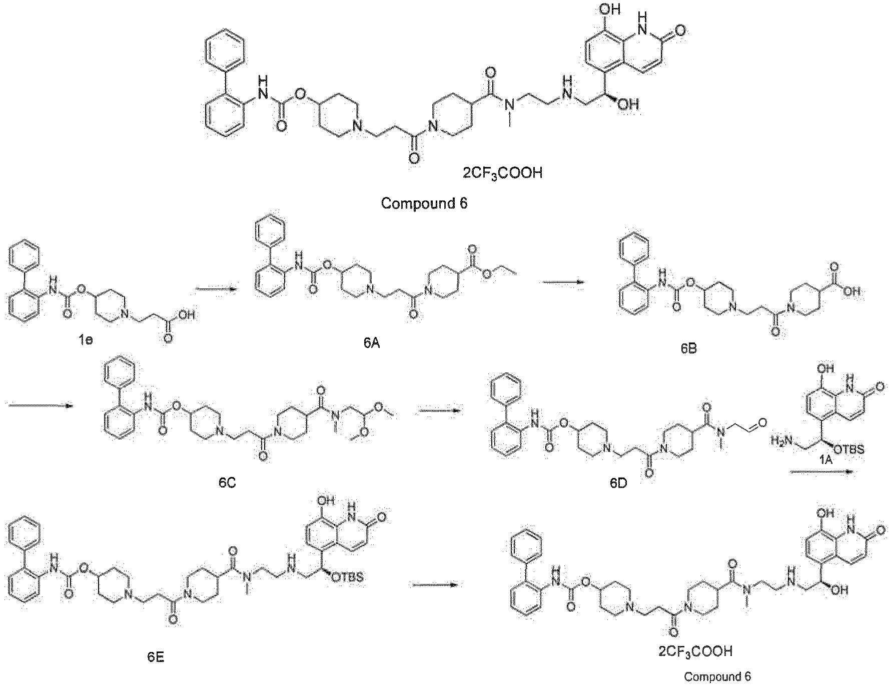

### Step 1:

### ethyl 1-[3-[4-[(2-phenylphenyl)carbamoyloxy]-1-piperidyl]propanoyl]piperidine-4-carboxylate (6A)

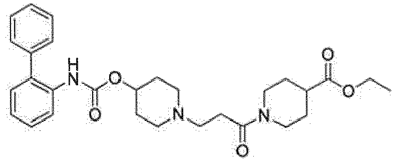

Methylene chloride (25 ml) and sulfoxide chloride (1.94 g, 16.3 mmol) were added to 3-[4-[(2-phenylphenyl)carbamoyloxy]-1-piperidyl]propanoic acid (1e) (5.00 g, 36.7 mmol), followed by stirring at room temperature for 1 hour. The solvent was removed by concentration under reduced pressure, to obtain Reaction Solution 1. Ethyl piperidine-4-carboxylate (0.854 g, 5.43 mmol) was dissolved in methylene chloride (25 ml), diisopropylethylamine (1.40 g, 10.9 mmol) was added thereto, and Reaction Solution 1 was added under stirring, followed by a reaction at room temperature for 2 hours. Water (100 ml) was added to the reaction solution, which was extracted with methylene chloride (100 mL×2), and the organic phases were combined, washed with saturated brine (100 mL×2), dried over anhydrous sodium sulfate, and concentrated under reduced pressure to remove the solvent. The residue was purified by silica gel column chromatography (the eluent was methylene chloride:methanol (v/v) = 99:1 to 97:3), to obtain ethyl 1-[3-[4-[(2-phenylphenyl)carbamoyloxy]-1-piperidyl]propanoyl]piperidine-4-carboxylate (6A) as a white solid (2.30 g, yield: 83.5%).

### Step 2:

### 1-[3-[ 4-[(2-phenylphenyl)carbamoyloxy]-1-piperidyl]propanoyl]piperidine-4-carboxy lic acid (6B)

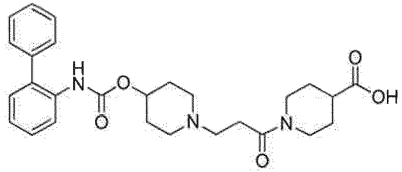

Ethyl 1-[3-[4-[(2-phenylphenyl)carbamoyloxy]-1-piperidyl]propanoyl]piperidine-4-carboxylate (6A) (2.00 g, 3.94 mmol) was dissolved in 20 ml tetrahydrofuran, to which a 5 ml aqueous solution of sodium hydroxide (3.50 g, 87.5 mmol) was added, followed by stirring at room temperature for 1 hour. Water (20 ml) was added to the reaction solution, which was extracted with methylene chloride (50 mL×3). The organic phases were combined, dried over anhydrous sodium sulfate, and concentrated under reduced pressure, to obtain 1-[3-[4-[(2-phenylphenyl)carbamoyloxy]-1-piperidyl]propanoyl]piperidine-4-carboxy lic acid (6B) as a white solid (1.8 g, yield: 95.3%).
¹H NMR (400 MHz, DMSO-d6) δ 12.15 (s, 1H), 10.13 (d, 1H), 8.79 (d, 1H), 7.49 - 7.25 (m, 8H), 4.68 (d, 1H), 4.30 - 4.15 (m, 1H), 3.78 (d, 1H), 3.60 (t, 1H), 3.46 (d, 1H), 3.36 (s, 1H), 3.29 - 3.17 (m, 2H), 3.06 (dd, 2H), 2.87 (d, 2H), 2.77 (dd, 1H), 1.96 (dd, 2H), 1.91 - 1.67 (m, 4H), 1.59 - 1.44 (m, 1H), 1.43 - 1.30 (m, 1H), 1.24 (s, 1H).

### Step 3:

### [1-[3-[4-[2,2-dimethoxyethyl(methyl)carbamoyl]-1 -piperidyl]-3-oxo-propyl]-4-piperi dyl] N-(2-phenylphenyl)carbamate (6C

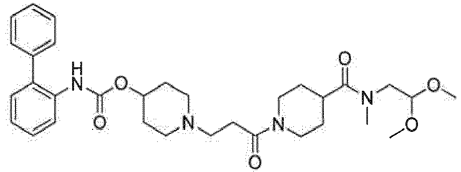

1-[3-[4-[(2-phenylphenyl)carbamoyloxy]-1-piperidyl]propanoyl]piperidine-4-carboxy lic acid (6B) (0.600 g, 1.25 mmol) was dissolved in dimethylformamide (20 ml), to which 2,2-methoxy-N-methyl-ethylamine (0.149 g, 1.25 mmol), HATU (0.713 g, 1.88 mmol) and triethylamine (0.380 g, 3.75 mmol) were added, followed by a reaction at room temperature for 1 hour. The reaction solution was extracted and partitioned by addition of methylene chloride (50 ml) and water (20 ml). The organic phases were washed with a saturated solution of sodium chloride (20 mL× 1), dried over anhydrous sodium sulfate, filtered, and the filtrate was concentrated under reduced pressure. The residue was purified by silica gel column chromatography (the eluent was methylene chloride:methanol (v/v) = 99:1 to 97:3), to obtain [1-[3-[4-[2,2-dimethoxyethyl(methyl)carbamoyl]-1-piperidyl]-3-oxo-propyl]-4-piperi dyl] N-(2-phenylphenyl)carbamate (6C) as a white solid (0.120 g, yield: 16.5%).
LCMS m/z =581.4 [M+1].

### Step 4:

### [1-[3-[4-[methyl(2-oxoethyl)carbamoyl]-1-piperidyl]-3-oxo-propyl]-4-piperidyl] N-(2-phenylphenyl)carbamate (6D)

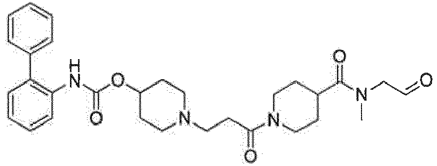

[1-[3-[4-[2,2-dimethoxyethyl(methyl)carbamoyl]-1-piperidyl]-3-oxo-propyl]-4-piperi dyl] N-(2-phenylphenyl)carbamate (6C) (1.00 g, 1.59 mmol) was dissolved in methylene chloride (10 ml), to which p-toluene sulfonic acid (0.445 g, 2.58 mmol) was added, followed by a reaction at room temperature for 2 hours. Water (30 ml) was added to the reaction solution, which was extracted and partitioned with methylene chloride (50 mL×2). The organic phases were combined, washed with a saturated aqueous solution of sodium bicarbonate (30 mL×3), dried over anhydrous sodium sulfate, filtered, and the filtrate was concentrated under reduced pressure to obtain [1-[3-[4-[methyl(2-oxoethyl)carbamoyl]-1-piperidyl]-3-oxo-propyl]-4-piperidyl] N-(2-phenylphenyl)carbamate (6D) as a yellow solid (0.200 g, yield: 72.4%).
LCMS m/z =535.3 [M+1].

### Step 5:

### [1-[3-[4-[2-[[(2R)-2-[tert-butyl(dimethyl)silyl]oxy-2-(8-hydroxy-2-oxo-1H-quinolin-5 -yl)ethyl]amino]ethyl-methyl-carbamoyl]-1-piperidyl]-3-oxo-propyl]-4-piperidyl] N-(2-phenylphenyl)carbamate (6E)

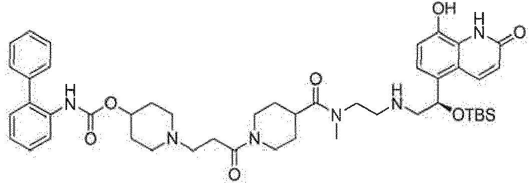

[1-[3-[4-[methyl(2-oxoethyl)carbamoyl]-1-piperidyl]-3-oxo-propyl]-4-piperidyl] N-(2-phenylphenyl)carbamate (6D) (0.200 g, 0.374 mmol) and 5-[(1R)-2-ammo-1-[t-butyl(dimethyl)silyl]oxymethyl]-8-hydroxy-1H-quinolin-2-one (1A) (0.125 g, 0.374 mmol) were dissolved in 10 ml dry methanol and stirred at room temperature for 1 hour, and then sodium cyanoborohydride (0.0705 g, 1.12 mmol) was added, followed by stirring for 3 h. The reaction solution was extracted and partitioned by addition of methylene chloride (50 ml) and water (50 ml). The organic phase was washed with a saturated aqueous solution of sodium chloride (20 mL× 1), dried over anhydrous sodium sulfate, and filtered, and the filtrate was concentrated under reduced pressure. The residue was purified by silica gel column chromatography (the eluent was methylene chloride/methanol (v/v) = 1/0 to 9/1) to obtain [1-[3-[4-[2-[[(2R)-2-[tert-butyl(dimethyl)silyl]oxy-2-(8-hydroxy-2-oxo-1H-quinolin-5 -yl)ethyl]amino]ethyl-methyl-carbamoyl]-1-piperidyl]-3-oxo-propyl]-4-piperidyl] N-(2-phenylphenyl)carbamate (6E) as a light-yellow solid (0.200 g, yield 62.7%).
LCMS m/z =427.4[(M+2)/2].

### Step 6:

### [1-[3-[4-[2-[[(2R)-2-hydroxy-2-(8-hydroxy-2-oxo-1H-quinolin-5-yl)ethyl]amino]ethy 1-methyl-carbamoyl]-1-piperidyl]-3-oxo-propyl]-4-piperidyl]N-(2-phenylphenyl)carba mate; ditrifluoroacetic acid (Compound 6)

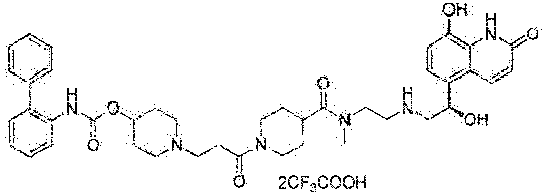

[1-[3-[4-[2-[[(2R)-2-[tert-butyl(dimethyl)silyl]oxy-2-(8-hydroxy-2-oxo-1H-quinolin-5 -yl)ethyl]amino]ethyl-methyl-carbamoyl]-1-piperidyl]-3-oxo-propyl]-4-piperidyl] N-(2-phenylphenyl)carbamate (6E) (0.250 g, 0.293 mmol) was dissolved in tetrahydrofuran (20 ml), and triethylamine trihydrofluoride (0.189 g, 1.17mmol) was added thereto, followed by a reaction at room temperature overnight. The reaction solution was adjusted to alkalinity with a saturated solution of sodium bicarbonate, and extracted with 8% methanol/methylene chloride (v/v=8:92, 100 ml). The organic phase was washed with a saturated aqueous solution of sodium chloride (50 mL×1), dried over anhydrous sodium sulfate, and filtered, and the filtrate was concentrated under reduced pressure. The residue was purified by preparative liquid column chromatography (liquid preparation condition: C18 reverse preparative column; Mobile phase: 0.05% TFA in deionized water (A) and 0.05% TFA in acetonitrile (B); Isocratic elution with A:B=10%~55% for 39 min; Flow rate: 1.0 ml/min; Column temperature: 40°C) to obtain [1-[3-[4-[2-[[(2R)-2-hydroxy-2-(8-hydroxy-2-oxo-1H-quinolin-5-yl)ethyl]amino]ethy 1-methyl-carbamoyl]-1-piperidyl]-3-oxo-propyl]-4-piperidyl]N-(2-phenylphenyl)carba mate; ditrifluoroacetic acid **(Compound** 6) as a white solid (0.200 g, yield 70.6%).
¹H NMR (400 MHz, CD₃OD) δ 8.38 (dd, 1H), 7.54 (s, 1H), 7.48 - 7.24 (m, 9H), 7.03 (dd, 1H), 6.68 (dd, 1H), 5.40 (m, 1H), 4.91 (s, 1H), 4.46 (t, 1H), 3.91 (d, 1H), 3.75 (dd, 1H), 3.65 (dd, 2H), 3.46 (d, 4H), 3.26 (d, 2H), 3.18 (dd, 5H), 2.95 (d, 3H), 2.77 (dd, 1H), 2.18 (d, 1H), 2.02 (s, 3H), 1.74 (d, 3H), 1.68 - 1.54 (m, 1H), 1.49 - 1.24 (m, 2H).
LCMS m/z =370.3[(M+2)/2].

### Example 7

### [1-[3-[4-[(2-phenylphenyl)carbamoyloxy]-1-piperidyl]propanoyl]-4-piperidyl]methyl N-[2-fluoro-4-[[[(2R)-2-hydroxy-2-(8-hydroxy-2-oxo-1 H-quinolin-5-yl)ethyl] amino] methyl]phenyl]carbamate; ditrifluoroacetic acid (Compound 7)

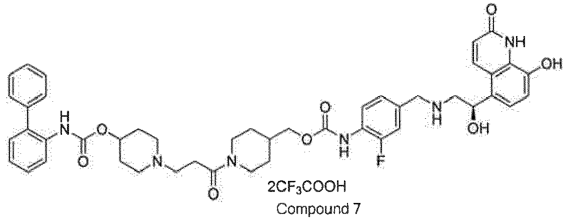

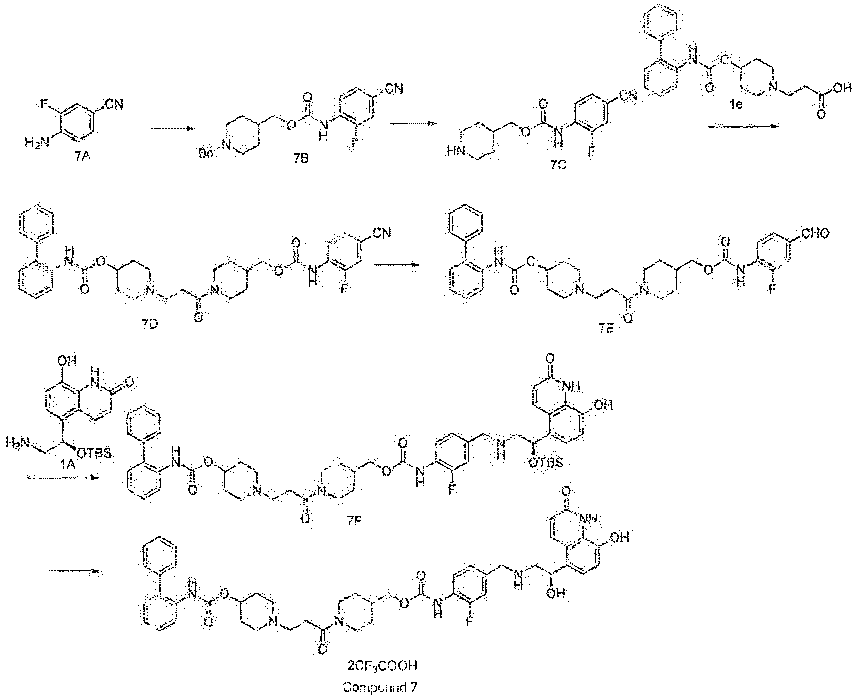

### Step 1: (1-benzylpiperidin-4-yl)methyl (4-cyano-2-fluorophenyl)carbamate (7B)

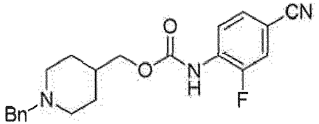

4-amino-3-fluorobenzonitrile (7A) (5.00 g, 36.7 mmol) and trichloromethyl carbonate (5.45 g, 18.4 mmol) were added to toluene (30 ml), and heated to 100°C to allow a reaction to proceed for 1 h. After cooling to room temperature, the solvent was removed by concentration under reduced pressure, and then tetrahydrofuran (30 ml), triethylamine (7.43 g, 73.5 mmol) and (N-benzylpiperidin-4-yl)methanol (8.29 g, 40.4 mmol) were added, followed by a reaction at 80°C for 1 hour. After cooling to room temperature, water (100 ml) was added to the reaction solution, which was extracted with methylene chloride (100 mL×3), and the organic phases were combined, washed with saturated brine (100 mL×2), dried over anhydrous sodium sulfate, and concentrated under reduced pressure to remove the solvent. The residue was purified by silica gel column chromatography (the eluent was methylene chloride:methanol (v/v) = 99:1 to 49:1), to obtain (1-benzylpiperidin-4-yl)methyl (4-cyano-2-fluorophenyl)carbamate (7B) as a white solid (5.0 g, yield: 37.1%).
¹H NMR (400 MHz, CDCl₃) δ 8.24 (t, 1H), 7.71 - 7.53 (m, 3H), 7.43 (dd, 4H), 7.39 - 7.32 (m, 1H), 4.26 - 4.05 (m, 4H), 3.46 (s, 2H), 2.72 (s, 2H), 2.20 (s, 2H), 1.94 (s, 3H).
LCMS m/z =368.1 [M+1].

### Step 2: piperidin-4-methyl 4-cano-2-fluorophenyl)carbamate (7C)

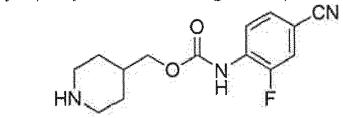

(1-benzylpiperidin-4-yl)methyl (4-cyano-2-fluorophenyl)carbamate (7B) (3.00 g, 8.17 mmol) was added to methylene chloride (20 ml) and methanol (10 ml), and a hydrogenation reaction was allowed to proceed at ambient pressure for 4 hours with palladium hydroxide/carbon (1.00 g, 33.3% w.t.). The reaction solution was filtered, and the filtrate was concentrated under reduced pressure. The residue was purified by silica gel column chromatography (the eluent was methylene chloride:methanol (v/v) = 49:1 to 9:1), to obtain piperidin-4-methyl (4-cyano-2-fluorophenyl)carbamate (7C) as a white solid (1.2 g, yield: 53.0%).

### Step 3:

### [1-[3-[4-[(2-phenylphenyl)carbamoyloxy]-1-piperidyl]propanoyl]-4-piperidyl]methyl N-(4-cyano-2-fluoro-phenyl)carbamate (7D)

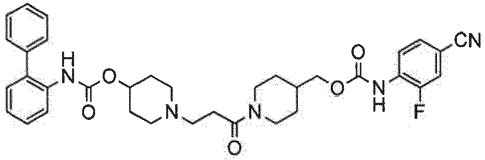

Piperidin-4-methyl (4-cyano-2-fluorophenyl)carbamate (7C) (0.800 g, 2.88 mmol) was dissolved in N,N-dimethylformamide (20 ml), to which 3-[4-[(2-phenylphenyl)carbamoyloxy]-1-piperidyl]propanoic acid (1e) (1.06 g, 2.88 mmol), HATU (1.65 g, 4.33 mmol) and triethylamine (0.876 g, 8.65 mmol) were added, followed by a reaction at room temperature for 1 hour. The reaction solution was extracted and partitioned by addition of methylene chloride (50 ml) and water (20 ml). The organic phase was washed with a saturated aqueous solution of sodium chloride (20 mL×1), dried over anhydrous sodium sulfate, and filtered, and the filtrate was concentrated under reduced pressure. The residue was purified by silica gel column chromatography (the eluent was methylene chloride:methanol (v/v) = 99:1 to 97:3), to obtain [1-[3-[4-[(2-phenylphenyl)carbamoyloxy]-1-piperidyl]propanoyl]-4-piperidyl]methyl N-(4-cyano-2-fluoro-phenyl)carbamate (7D) as a white solid (1.12 g, yield: 61.8%).
LCMS m/z =628.3 [M+1].

### Step 4:

### [1-[3-[4-[(2-phenylphenyl)carbamoyloxy]-1-piperidyl]propanoyl]-4-piperidyl]methyl N-(2-fluoro-4-formyl-phenyl)carbamate (7E)

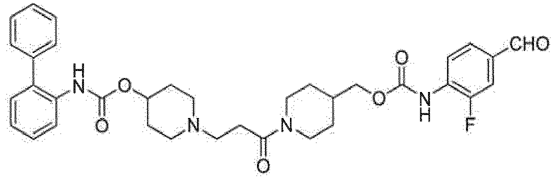

[1-[3-[4-[(2-phenylphenyl)carbamoyloxy]-1-piperidyl]propanoyl]-4-piperidyl]methyl N-(4-cyano-2-fluoro-phenyl)carbamate (7D) (1.20 g, 1.91 mmol) was dissolved in dry methanol (15 ml), water (5 ml) and aluminum-nickel alloy (0.200 g, 4.65 mmol) were added, followed by a reaction at 90°C for 2 hours. After cooling to room temperature, water (20 ml) was added to the reaction solution, which was extracted with methylene chloride (50 mL×2). The organic phases were combined, washed with a saturated aqueous solution of sodium bicarbonate (30 mL×3), dried over anhydrous sodium sulfate, filtered, and the filtrate was concentrated under reduced pressure, to obtain [1-[3-[4-[(2-phenylphenyl)carbamoyloxy]-1-piperidyl]propanoyl]-4-piperidyl]methyl N-(2-fluoro-4-formyl-phenyl)carbamate (7E) as a light-yellow solid (0.700 g, yield: 58.1%).
LCMS m/z =631.3 [M+1].

### Step 5:

### [1-[3-[4-[(2-phenylphenyl)carbamoyloxy]-1-piperidyl]propanoyl]-4-piperidyl] methyl N-[4-[[[(2R)-2-[tert-butyl(dimethyl)silyl]oxy-2-(8-hydroxy-2-oxo-1H-quinolin-5 -yl)ethyl] amino]methyl] -2-fluoro-phenyl] carbamate (7F)

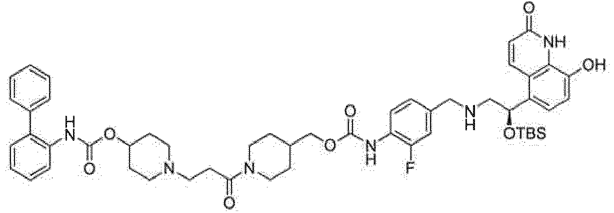

[1-[3-[4-[(2-phenylphenyl)carbamoyloxy]-1-piperidyl]propanoyl]-4-piperidyl]methyl N-(2-fluoro-4-formyl-phenyl)carbamate (7E) (0.900 g, 1.43 mmol) and 5-[(1R)-2-amino-1-[t-butyl(dimethyl)silyl]oxymethyl]-8-hydroxy-1H-quinolin-2-one (1A) (0.477 g, 1.43 mmol) were dissolved in 10 ml dry methanol and stirred at room temperature for 1 hour, and then sodium triacetoxyborohydride (0.274 g, 4.28 mmol) was added, followed by stirring for 3 h. The reaction solution was extracted and partitioned by addition of methylene chloride (50 ml) and water (50 ml). The organic phase was washed with a saturated aqueous solution of sodium chloride (20 mL×1), dried over anhydrous sodium sulfate, and filtered, and the filtrate was concentrated under reduced pressure. The residue was purified by silica gel column chromatography (the eluent was methylene chloride/methanol (v/v) = 1/0 to 9/1) to obtain [1-[3-[4-[(2-phenylphenyl)carbamoyloxy]-1-piperidyl]propanoyl]-4-piperidyl]methyl N-[4-[[[(2R)-2-[tert-butyl(dimethyl)silyl]oxy-2-(8-hydroxy-2-oxo-1H-quinolin-5-yl)ethyl]amino]methyl]-2-fluoro-phenyl]carbamate (7F) as a light-yellow solid (0.800 g, yield 59.1%).

### Step 6:

### [1-[3-[4-[(2-phenylphenyl)carbamoyloxy]-1-piperidyl]propanoyl]-4-piperidyl] methyl N-[2-fluoro-4-[[[(2R)-2-hydroxy-2-(8-hydroxy-2-oxo-1H-quinolin-5-yl)ethyl]amino] methyl]phenyl]carbamate;ditrifluoroacetic acid (Compound 7)

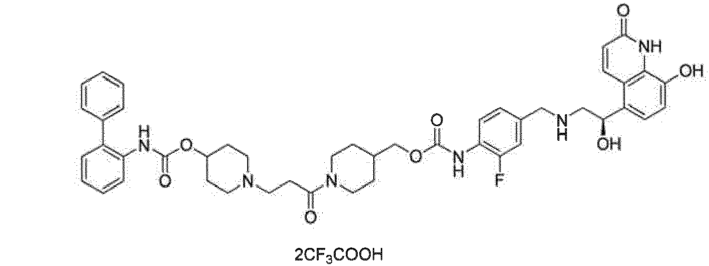

[1-[3-[4-[(2-phenylphenyl)carbamoyloxy]-1-piperidyl]propanoyl]-4-piperidyl]methyl N-[4-[[[(2R)-2-[tert-butyl(dimethyl)silyl] oxy-2-(8-hydroxy-2-oxo-1H-quinolin-5-yl)ethyl]amino]methyl]-2-fluoro-phenyl]carbamate (7F) (0.850 g, 0.895 mmol) was dissolved in tetrahydrofuran (20 ml), and triethylamine trihydrofluoride (0.0575 g, 3.58 mmol) was added thereto, followed by a reaction at room temperature overnight. The reaction solution was adjusted to alkalinity with a saturated solution of sodium bicarbonate, and extracted with 8% methanol/methylene chloride (v/v=8:92, 100 ml). The organic phase was washed with a saturated aqueous solution of sodium chloride (50 mL×1), dried over anhydrous sodium sulfate, and filtered, and the filtrate was concentrated under reduced pressure. The residue was purified by preparative liquid column chromatography (liquid preparation condition: C18 reverse preparative column; Mobile phase: 0.05% TFA in deionized water (A) and 0.05% TFA in acetonitrile (B); Isocratic elution with A:B=10%~55% for 39 min; Flow rate: 1.0 ml/min; Column temperature: 40°C) to obtain [1-[3-[4-[(2-phenylphenyl)carbamoyloxy]-1-piperidyl]propanoyl]-4-piperidyl]methyl N-[2-fluoro-4-[[[(2R)-2-hydroxy-2-(8-hydroxy-2-oxo-1H-quinolin-5-yl)ethyl]amino] methyl]phenyl]carbamate;ditrifluoroacetic acid **(Compound** 7) as a white solid (0.550 g, yield 57.8%).
¹H NMR (400 MHz, CD₃OD) δ 8.27 (d, 1H), 7.98 (t, 1H), 7.55 (d, 1H), 7.48 - 7.20 (m, 11H), 7.02 (d, 1H), 6.64 (d, 1H), 5.41 (t, 1H), 4.89 (s, 1H), 4.55 (d, 1H), 4.27 (s, 2H), 4.07 (d, 2H), 3.93 (d, 1H), 3.60 (s, 1H), 3.48 (d, 1H), 3.39 (s, 2H), 3.23 (d, 2H), 3.12 (t, 3H), 2.92 (t, 2H), 2.69 (t, 1H), 2.14 (s, 1H), 2.00 (s, 3H), 1.92 - 1.70 (m, 3H), 1.41 - 1.13 (m, 2H).
LCMS m/z =418.4[(M+2)/2].

### Example 8

### [1-[3-[4-[(2-phenylphenyl)carbamoyloxy]-1-piperidyl]propanoyl]-4-piperidyl]methyl N-[3-fluoro-4-[[[(2R)-2-hydroxy-2-(8-hydroxy-2-oxo-1H-quinolin-5-yl)ethyl] amino]methyl]phenyl]carbamate; ditrifluoroacetic acid (Compound 8

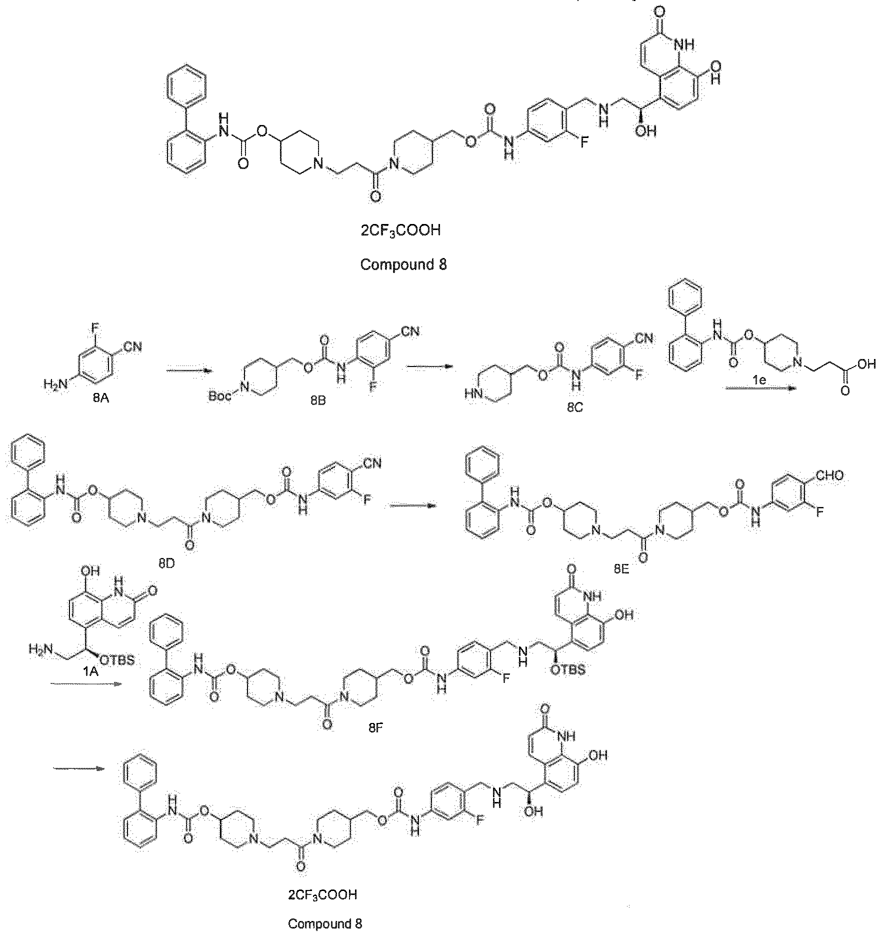

### Step 1: tert-butyl

### 4-((((4-cyano-3-fluorophenyl)carbamoyl)oxy)methyl)piperidine-1-carboxylate (8B)

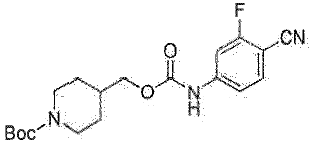

4-amino-2-fluorobenzonitrile (5.00 g, 36.7 mmol) and trichloromethyl carbonate (5.45 g, 18.4 mmol) were added to toluene (30 ml), and heated to 100°C to allow a reaction to proceed for 1 h. The reaction solution was cooled to room temperature and concentrated under reduced pressure to remove the solvent, and then tetrahydrofuran (30 ml), triethylamine (7.43 g, 73.5 mmol) and t-butyl 4-(hydroxymethyl)piperidine-1-carboxylate (8.70 g, 40.4 mmol) were added, followed by a reaction at 80°C for 1 hour. After cooling to room temperature, water (100 ml) was added to the reaction solution, which was extracted with methylene chloride (100 mL×3), and the organic phases were combined, washed with saturated brine (100 mL×2), dried over anhydrous sodium sulfate, and concentrated under reduced pressure to remove the solvent. The residue was purified by silica gel column chromatography (the eluent was methylene chloride:methanol (v/v) = 99:1 to 49:1),to obtain tert-butyl 4-((((4-cyano-3-fluorophenyl)carbamoyl)oxy)methyl)piperidine-1-carboxylate (8B) as a white solid (5.0 g, yield: 36.1%).
LCMS m/z =400.3 [M+23].

### Step 2: piperidin-4-ylmethyl (4-cyano-3-fluorophenyl)carbamate (8C)

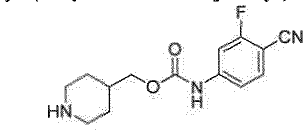

Tert-butyl 4-((((4-cyano-3-fluorophenyl)carbamoyl)oxy)methyl)piperidine-1-carboxylate (8B) (3.85 g, 2.80 mmol) was dissolved in methylene chloride (20 ml), to which trifluoroacetic acid (10 ml) was added, followed by stirring at room temperature for 2 hours. The solvent was removed by concentration under reduced pressure, and water (20 ml) and a saturated solution of sodium bicarbonate (10 ml) were added. The resultant was extracted with 10% (v/v) methanol/methylene chloride (100 mL×3), and the organic phases were combined, dried over anhydrous sodium sulfate, and concentrated under reduced pressure, to obtain piperidin-4-ylmethyl (4-cyano-3-fluorophenyl)carbamate (8C) as a white solid (2.8 g, yield 99.0%).
¹H NMR (400 MHz, DMSO-d6) δ 10.40 (s, 1H), 7.88 - 7.73 (m, 1H), 7.62 (dd, 1H), 7.38 (dd, 1H), 4.01 (d, 2H), 3.21 - 3.03 (m, 2H), 2.76 - 2.56 (m, 2H), 1.86 (m, 1H), 1.73 (d, 2H), 1.31 -1.21 (m, 2H).
LCMS m/z =278.1[M+1].

### Step 3:

### [1-[3-[4-[(2-phenylphenyl)carbamoyloxy]-1-piperidyl]propanoyl]-4-piperidyl]methyl N-(4-cyano-3-fluoro-phenyl)carbamate (8D)

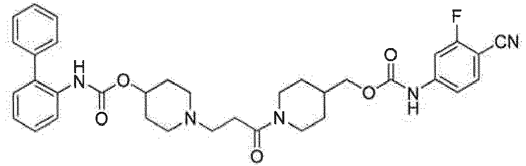

Piperidin-4-ylmethyl (4-cyano-3-fluorophenyl)carbamate (1B) (1.11 g, 4.00 mmol) was dissolved in dimethylformamide (20 ml), to which 3-[4-[(2-phenylphenyl)carbamoyloxy]-1-piperidyl]propanoic acid (1e) (1.47 g, 4.00 mmol), HATU (2.28 g, 6.00 mmol) and triethylamine (1.22 g, 12.0 mmol) were added, followed by a reaction at room temperature for 1 hour. The reaction solution was extracted and partitioned by addition of methylene chloride (50 ml) and water (20 ml). The organic phase was washed with a saturated aqueous solution of sodium chloride (20 mL× 1), dried over anhydrous sodium sulfate, and filtered, and the filtrate was concentrated under reduced pressure. The residue was purified by silica gel column chromatography (the eluent was methylene chloride:methanol (v/v) = 99:1 to 97:3), to obtain [1-[3-[4-[(2-phenylphenyl)carbamoyloxy]-1-piperidyl]propanoyl]-4-piperidyl]methyl N-(4-cyano-3-fluoro-phenyl)carbamate (8D) as a white solid (1.52 g, yield: 60.5%).
¹H NMR (400 MHz, DMSO-d6) δ 10.43 (s, 1H), 8.60 (s, 1H), 7.82 (t, 1H), 7.62 (dd, 1H), 7.46 - 7.26 (m, 10H), 4.41 (d, 2H), 4.07 - 3.98 (m, 2H), 3.91 (d, 1H), 3.30 (d, 1H), 3.00 (t, 1H), 2.69 - 2.52 (m, 3H), 2.49 - 2.38 (m, 3H), 2.23 - 2.05 (m, 2H), 1.91 (t, 1H), 1.71 (s, 4H), 1.40 (d, 2H), 1.24 (s, 1H), 1.12-1.00 (m, 1H).

### Step 4:

### [1-[3-[4-[(2-phenylphenyl)carbamoyloxy]-1-piperidyl]propanoyl]-4-piperidyl]methyl N-(3-fluoro-4-formyl-phenyl)carbamate (8E)

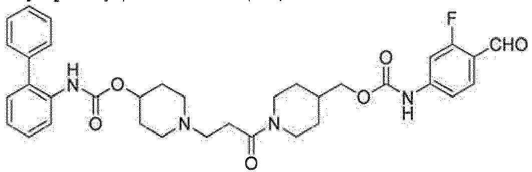

[1-[3-[ 4-[(2-phenylphenyl)carbamoyloxy]-1-piperidyl]propanoyl]-4-piperidyl]methyl N-(4-cyano-3-fluoro-phenyl)carbamate (8D) (1.00 g, 1.59 mmol) was dissolved in dry formic acid (7.5 ml), water (2.5 ml) and aluminum-nickel alloy (0.800 g, 18.6 mmol) were added, followed by a reaction at 90°C for 4 hours. After cooling to room temperature, water (20 ml) was added to the reaction solution, which was extracted with methylene chloride (50 mL×2). The organic phases were combined, washed with a saturated aqueous solution of sodium bicarbonate (30 mL×3), dried over anhydrous sodium sulfate, filtered, and the filtrate was concentrated under reduced pressure, to obtain [1-[3-[4-[(2-phenylphenyl)carbamoyloxy]-1-piperidyl]propanoyl]-4-piperidyl]methyl N-(3-fluoro-4-formyl-phenyl)carbamate (8E) as a light-yellow solid (1.00 g, yield: 99.5%).
¹H NMR (400 MHz, DMSO-d6) δ 10.39 (s, 1H), 10.07 (s, 1H), 8.59 (s, 1H), 7.77 (t, 1H), 7.53 (d, 1H), 7.43 - 7.26 (m, 10H), 4.40 (d, 2H), 4.01 (d, 3H), 3.90 (d, 1H), 2.99 (t, 1H), 2.60 (s, 2H), 2.46 (d, 3H), 2.13 (t, 2H), 1.92 (s, 1H), 1.71 (s, 4H), 1.46 - 1.35 (m, 2H), 1.26 -1.14 (m, 2H), 1.07 (d, 1H).
LCMS m/z =631.3 [M+1].

### Step 5:

### [1-[3-[4-[(2-phenylphenyl)carbamoyloxy]-1-piperidyl]propanoyl]-4-piperidyl]methyl N-[4-[[[(2R)-2-[tert-butyl(dimethyl)silyl]oxy-2-(8-hydroxy-2-oxo-1H-quinolin-5-yl)ethyl]amino]methyl]-3-fluoro-phenyl]carbamate (8F)

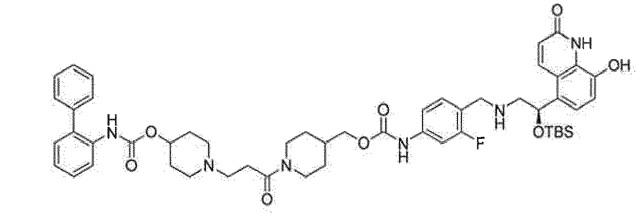

[1-[3-[4-[(2-phenylphenyl)carbamoyloxy]-1-piperidyl]propanoyl]-4-piperidyl]methyl N-(3-fluoro-4-formyl-phenyl)carbamate (8E) (1.10 g, 1.74 mmol) and 5-[(1R)-2-amino-1-[t-butyl(dimethyl)silyl]oxymethyl]-8-hydroxy-1H-quinolin-2-one (1A) (0.583 g, 1.74 mmol) were dissolved in dry methanol (10 ml) and stirred at room temperature for 1 hour, and then sodium triacetoxyborohydride (0.335 g, 5.23 mmol) was added, followed by stirring for 3 h. The reaction solution was extracted and partitioned by addition of methylene chloride (50 ml) and water (50 ml). The organic phase was washed with a saturated aqueous solution of sodium chloride (20 mL×1), dried over anhydrous sodium sulfate, and filtered, and the filtrate was concentrated under reduced pressure. The residue was purified by silica gel column chromatography (the eluent was methylene chloride/methanol (v/v) = 1/0 to 9/1) to obtain [1-[3-[4-[(2-phenylphenyl)carbamoyloxy]-1-piperidyl]propanoyl]-4-piperidyl]methyl N-[4-[[[(2R)-2-[tert-butyl(dimethyl)silyl]oxy-2-(8-hydroxy-2-oxo-1H-quinolin-5-yl)ethyl]amino]methyl]-3-fluoro-phenyl]carbamate (8F) as a light-yellow solid (1.10 g, yield 66.5%).

### Step 6:

### [1-[3-[4-[(2-phenylphenyl)carbamoyloxy]-1-piperidyl]propanoyl]-4-piperidyl]methyl N-[3-fluoro-4-[[[(2R)-2-hydroxy-2-(8-hydroxy-2-oxo-1H-quinolin-5-yl)ethyl]amino] methyl]phenyl]carbamate; ditrifluoroacetic acid (Compound 8)

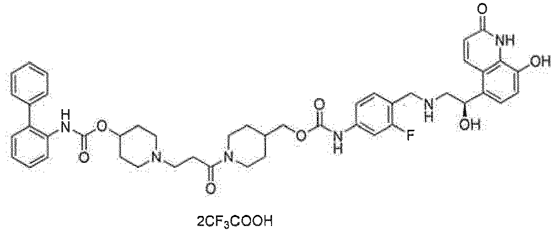

[1-[3-[4-[(2-phenylphenyl)carbamoyloxy]-1-piperidyl]propanoyl]-4-piperidyl]methyl N-[4-[[[(2R)-2-[tert-butyl(dimethyl)silyl]oxy-2-(8-hydroxy-2-oxo-1H-quinolin-5-yl)ethyl]amino]methyl]-3-fluoro-phenyl]carbamate (8F) (1.45 g, 1.53 mmol) was dissolved in tetrahydrofuran (20 ml), and triethylamine trihydrofluoride (0.0980 g, 6.11 mmol) was added thereto, followed by a reaction at room temperature overnight. The reaction solution was adjusted to alkalinity with a saturated solution of sodium bicarbonate, and extracted with 8% methanol/methylene chloride (v/v=8:92, 100 ml). The organic phase was washed with a saturated aqueous solution of sodium chloride (50 mL×1), dried over anhydrous sodium sulfate, and filtered, and the filtrate was concentrated under reduced pressure. The residue was purified by preparative liquid column chromatography (liquid preparation condition: C18 reverse preparative column; Mobile phase: 0.05% TFA in deionized water (A) and 0.05% TFA in acetonitrile (B); Isocratic elution with A:B=10%~55% for 39 min; Flow rate: 1.0 ml/min; Column temperature: 40°C) to obtain [1-[3-[4-[(2-phenylphenyl)carbamoyloxy]-1-piperidyl]propanoyl]-4-piperidyl]methyl N-[3-fluoro-4-[[[(2R)-2-hydroxy-2-(8-hydroxy-2-oxo-1H-quinolin-5-yl)ethyl]amino] methyl]phenyl]carbamate; ditrifluoroacetic acid **(Compound** 8) as a white solid (0.710 g, yield 43.7%).
¹H NMR (400 MHz, CD₃OD) δ 8.29 (d, 1H), 7.59 - 7.49 (m, 2H), 7.41 (m, 7H), 7.32 - 7.20 (m, 4H), 7.03 (d, 1H), 6.64 (d, 1H), 5.43 (t, 1H), 4.89 (s, 1H), 4.54 (d, 1H), 4.41 - 4.27 (m, 2H), 4.04 (d, 2H), 3.93 (d, 1H), 3.60 (s, 1H), 3.48 (d, 1H), 3.39 (s, 2H), 3.24 (t, 2H), 3.13 (dd, 3H), 2.92 (s, 2H), 2.69 (t, 1H), 2.23 - 1.92 (m, 4H), 1.91 - 1.70 (m, 3H), 1.41 - 1.12 (m, 2H).
LCMS m/z =418.3[(M+2)/2].

### Example 9

### [1-[3-[4-[2-[4-[[[(2R)-2-hydroxy-2-(8-hydroxy-2-oxo-1H-quinolin-5-yl)ethyl]amino] methyl]anilino]-2-oxo-ethyl]-1-piperidyl]-3-oxo-propyl]-4-piperidyl] N-(2-phenylphenyl)carbamate; di(trifluoroacetic acid) (Compound 9)

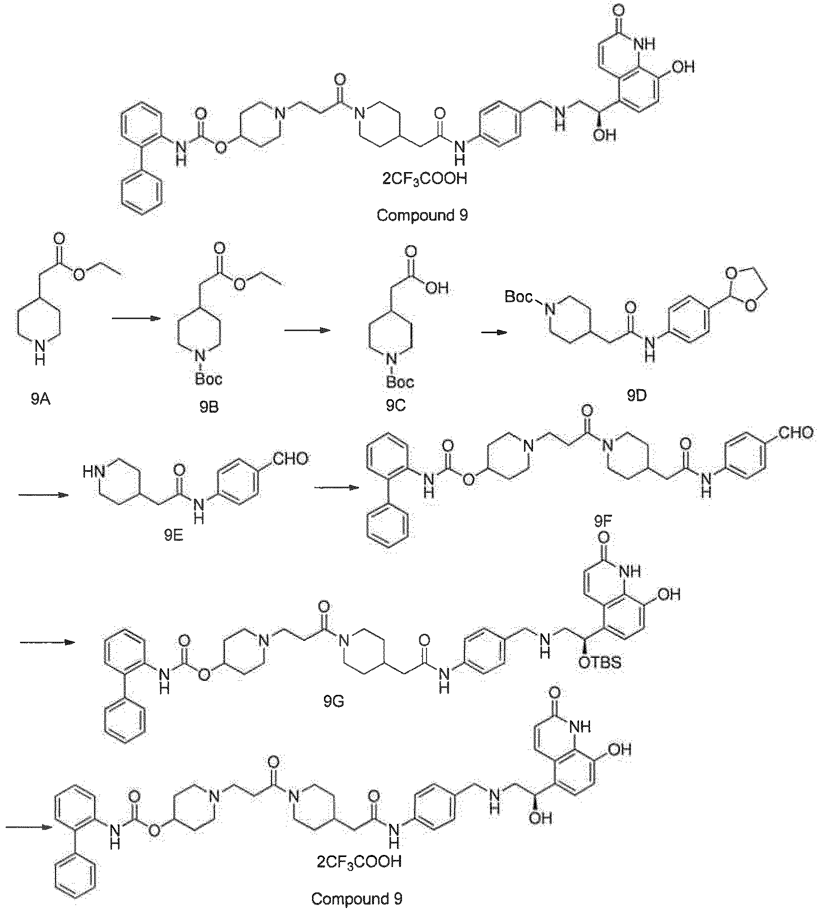

### Step 1: tert-butyl 4-(2-ethoxy-2-oxo-ethyl)piperidine-1-carboxylate (1B)

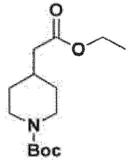

Ethyl 2-(4-piperidinyl)acetate (9A) (10.0 g, 58.4 mmol) was dissolved in dry tetrahydrofuran (80 ml), and di(tert-butyl) dicarbonate (19.1 g, 87.6 mmol) was added thereto, followed by a reaction at 55°C for 4 hours. The reaction solution was cooled to room temperature, and concentrated under reduced pressure. The residue was purified by silica gel column chromatography (petroleum ether:ethyl acetate (v/v) = 0:1 to 3:1) to give tert-butyl 4-(2-ethoxy-2-oxo-ethyl)piperidine-1-carboxylate (9B) as a colorless liquid (2.5 g, yield: 16%).
¹H NMR (400 MHz, CDCl₃) δ 4.13 (m, 2H), 4.08 (m, 2H), 2.72 (m, 2H), 2.23 (m, 2H), 1.93 (m, 1H), 1.69 (dd, 2H), 1.44 (m, 9H), 1.26 (m, 3H), 1.15 (m, 2H). LCMS m/z =272.2 [M+1].

### Step 2: 2-(1-tert-butoxycarbonyl-4-piperidyl)acetic acid (9C)

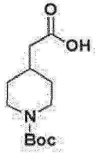

Tert-butyl 4-(2-ethoxy-2-oxo-ethyl)piperidine-1-carboxylate (9B) (2.5 g, 9.2 mmol) was dissolved in ethanol (15 ml), to which an aqueous solution (5 ml) of sodium hydroxide (1.8 g, 46 mmol) was added, and the temperature was raised to 90°C to allow a reaction to proceed for 1 hour. The reaction solution was cooled to room temperature, to which an aqueous solution of 2M hydrochloric acid was added dropwise slowly on an ice bath to adjust the pH to 2, and was extracted with ethyl acetate (20 ml×2). The organic phases were combined, washed with a saturated sodium chloride solution (20 ml× 1), dried over anhydrous sodium sulfate, and concentrated. The residue was purified by silica gel column chromatography (petroleum ether:ethyl acetate (v/v) = 0:1 to 1:1) to give 2-(1-tert-butoxycarbonyl-4-piperidyl)acetic acid (9C) as a yellow solid (1.9 g, yield: 85%).
¹H NMR (400 MHz, CDCl₃) δ 4.09 (d, 2H), 2.73 (t, 2H), 2.29 (d, 2H), 1.93 (d, 1H), 1.73 (d, 2H), 1.45 (s, 9H), 1.21 (m, 2H).
LCMS m/z =266.3 [M+23].

### Step 3: tert-butyl

### 4-[2-[4-(1,3-dioxolan-2-yl)anilino]-2-oxo-ethyl]piperidine-1-carboxylate (9D)

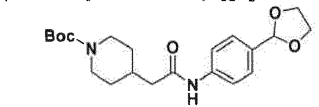

2-(1-tert-butoxycarbonyl-4-piperidyl)acetic acid (9C) (1.9 g, 7.7 mmol) was dissolved in dichloromethane (50 ml), to which 4-(1,3-dioxolan-2-yl)aniline (1.4 g, 8.65 mmol) and HATU (4.5 g, 11.7 mmol) were added, and N,N-diisopropylethylamine (2.0 g, 15.6 mmol) was added dropwise, followed by a reaction at room temperature for 3 hours. The reaction solution was extracted and partitioned by addition of water (50 ml). The organic phase was washed with a saturated aqueous solution of sodium chloride (50 mL× 1), dried over anhydrous sodium sulfate, and filtered, and the filtrate was concentrated under reduced pressure. The residue was purified by silica gel column chromatography (petroleum ether:ethyl acetate (v/v) = 0:1 to 3:1) to give tert-butyl 4-[2-[4-(1,3-dioxolan-2-yl)anilino]-2-oxo-ethyl]piperidine-1-carboxylate (9D) as a yellow solid (3.0 g, yield: 98%).
¹H NMR (400 MHz, DMSO-d6) δ 9.94 (s, 1H), 7.59 (m, 2H), 7.33 (m, 2H), 5.65 (s, 1H), 4.03 (m, 2H), 3.92 (m, 4H), 2.80 (s, 2H), 2.26 (t, 2H), 1.93 (m, 1H), 1.64 (d, 2H), 1.36 (m, 9H), 1.07 (m, 2H).
LCMS m/z =413.3 [M+23].

### Step 4: N-(4-formylphenyl)-2-(4-piperidyl)acetamide (9E)

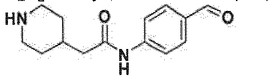

Tert-butyl 4-[2-[4-(1,3-dioxolan-2-yl)anilino]-2-oxo-ethyl]piperidine-1-carboxylate (9D) (3.0 g, 7.7 mmol) was dissolved in methylene chloride (15 ml), to which trifluoroacetic acid (4.4 g, 38.4 mmol) was added, followed by a reaction at room temperature for 3 hours. The pH of the reaction solution was adjusted to 8~9 with aqueous ammonia, and the solution was extracted and partitioned by addition of methylene chloride (20 ml) and water (20 ml). The aqueous phase was extracted with a mixed solvent of 10% (v/v) methanol/methylene chloride (20mL×5). The organic phase mixed with methanol/methylene chloride was dried over anhydrous sodium sulfate, and filtered, and the filtrate was concentrated under reduced pressure, to obtain the yellow oily N-(4-formylphenyl)-2-(4-piperidyl)acetamide (9E) (1.7 g, yield: 90%).
LCMS m/z =247.3 [M+23].

### Step 5:

### [1-[3-[4-[2-(4-formylanilino)-2-oxo-ethyl]-1-piperidyl]-3-oxo-propyl]-4-piperidyl]N-( 2-phenylphenyl)carbamate (9F)

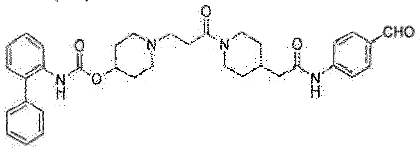

N-(4-formylphenyl)-2-(4-piperidyl)acetamide (9E) (1.7 g, 6.8 mmol) and 3-[4-[(2-phenylphenyl)carbamoyloxy]-1-piperidyl]propanoic acid (1e) (2.5 g, 6.5 mmol) were dissolved in methylene chloride (10 ml) and cooled to 0°C, then HATU (3.9 g, 10 mmol) was added, and diisopropylethylamine (2.6 g, 20 mmol) was added dropwise, followed by a reaction at room temperature for 3 hours. The reaction solution was extracted and partitioned by addition of methylene chloride (50 ml) and water (50 ml). The organic phase was washed with a saturated aqueous solution of sodium chloride (50 mL× 1), dried over anhydrous sodium sulfate, and filtered, and the filtrate was concentrated under reduced pressure. The residue was purified by silica gel column chromatography (methylene chloride:methanol (v/v) = 1:0 to 9:1), to obtain [1-[3-[4-[2-(4-formylanilino)-2-oxo-ethyl]-1-piperidyl]-3-oxo-propyl]-4-piperidyl]N-( 2-phenylphenyl)carbamate (9F) as a yellow solid (1.7 g, yield: 42%).
LCMS m/z =597.3 [M+1].

### Step 6:

### [1-[3-[4-[2-[4-[[[(2R)-2-[tert-butyl(dimethyl)silyl]oxy-2-(8-hydroxy-2-oxo-1H-quinol in-5-yl)ethyl]ammo]methyl]anilmo]-2-oxo-ethyl]-1-piperidyl]-3-oxo-propyl]-4-piperi dyl] N-(2-phenylphenyl)carbamate (9G)

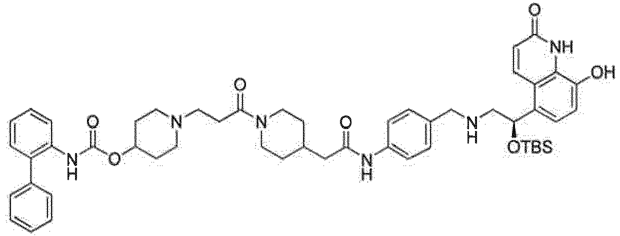

[1-[3-[4-[2-(4-formylanilino)-2-oxo-ethyl]-1-piperidyl]-3-oxo-propyl]-4-piperidyl]N-( 2-phenylphenyl)carbamate (9F) (0.7 g, 1.2 mmol) and 5-[(1R)-2-amino-1-[t-butyl(dimethyl)silyl]oxymethyl]-8-hydroxy-1H-quinolin-2-one (1A) (0.39 g, 1.2 mmol) were dissolved in methanol/methylene chloride (v/v=1/1) (10 ml) and stirred at room temperature for 1 hour, and then sodium triacetoxyborohydride (0.37 g, 1.8 mmol) and acetic acid (0.07 g, 1.2 mmol) were added, followed by stirring for 12 h. The reaction solution was extracted by addition of methylene chloride (30 ml) and a saturated solution of sodium bicarbonate (30 ml). The aqueous phase was extracted with methylene chloride (20 mL×1). The organic phases were combined. The organic phase was dried over anhydrous sodium sulfate and filtered, and the filtrate was concentrated under reduced pressure, to obtain [1-[3-[4-[2-[4-[[[(2R)-2-[tert-butyl(dimethyl)silyl]oxy-2-(8-hydroxy-2-oxo-1H-quinol in-5-yl)ethyl]amino]methyl]anilino]-2-oxo-ethyl]-1-piperidyl]-3-oxo-propyl]-4-piperi dyl] N-(2-phenylphenyl)carbamate (9G) as a yellow solid (0.7 g, yield: 65%).
LCMS m/z =458.4[(M+2)/2].

### Step 7:

### [1-[3-[4-[2-[4-[[[(2R)-2-hydroxy-2-(8-hydroxy-2-oxo-1H-quinolin-5-yl)ethyl]amino] methyl]anilino]-2-oxo-ethyl]-1-piperidyl]-3-oxo-propyl]-4-piperidyl] N-(2-phenylphenyl)carbamate; di(trifluoroacetic acid) (Compound 9)

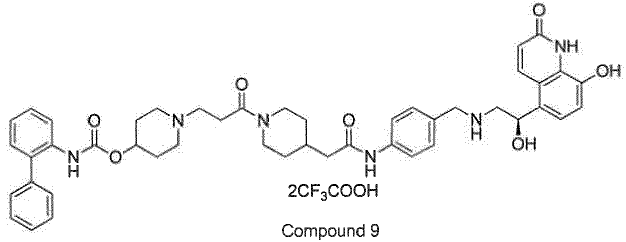

[1-[3-[4-[2-[4-[[[(2R)-2-[tert-butyl(dimethyl)silyl]oxy-2-(8-hydroxy-2-oxo-1H-quinol in-5-yl)ethyl]amino]methyl]anilino]-2-oxo-ethyl]-1-piperidyl]-3-oxo-propyl]-4-piperi dyl] N-(2-phenylphenyl)carbamate (9G) (0.7 g, 0.77 mmol) was dissolved in tetrahydrofuran (10 ml), and triethylamine trihydrofluoride (1.2 g, 7.7 mmol) was added thereto, followed by a reaction at room temperature overnight. The reaction solution was adjusted to alkalinity with a saturated solution of sodium bicarbonate, and extracted with 8% methanol/methylene chloride (v/v) (100 ml×1). The organic phase was washed with a saturated aqueous solution of sodium chloride (50 mL×1), dried over anhydrous sodium sulfate, and filtered, and the filtrate was concentrated under reduced pressure. The residue was purified by preparative liquid column chromatography (liquid preparation condition: C18 reverse preparative column; Mobile phase: 0.05% TFA in deionized water (A) and 0.05% TFA in acetonitrile (B); gradient elution with A:B=10%~55% for 39 min; Flow rate: 1.0 ml/min; Column temperature: 40°C) to obtain [1-[3-[4-[2-[4-[[[(2R)-2-hydroxy-2-(8-hydroxy-2-oxo-1H-quinolin-5-yl)ethyl]amino] methyl]anilino]-2-oxo-ethyl]-1-piperidyl]-3-oxo-propyl]-4-piperidyl] N-(2-phenylphenyl)carbamate; di(trifluoroacetic acid) **(Compound** 9) as a light-yellow solid (0.26 g, yield 42%).
¹H NMR (400 MHz, CD₃OD) δ 8.20 (d, 1H), 7.67 (d, 2H), 7.54 (s, 1H), 7.37 (m, 11H), 7.02 (d, 1H), 6.62 (d, 1H), 5.36 (dd, 1H), 4.53 (d, 1H), 4.26 (s, 2H), 3.91 (d, 1H), 3.53 (d, 2H), 3.39 (s, 2H), 3.16 (m, 5H), 2.91 (s, 2H), 2.71 (t, 1H), 2.36 (d, 2H), 2.14 (s, 2H), 1.91 (m, 6H), 1.26 (m, 2H).
LCMS m/z =401.2[(M+2)/2].

### Biological tests

### Test Example 1: Inhibitory activity against human muscarine receptor M3

CHO cells (PerkinElmer, ES-212-AF) that stably express human muscarine receptor 3 (hM3) and apo-Aequorin were cultured at 37°C, 5% CO₂ in Ham's F12 media (Invitrogen 12500-062) containing 10% fetal bovine serum (FBS) (Gibico 10099-141), 400 µg/mL G418 (sigma G5013) and 250 µg/mL Zeocin (Invivogen ant-zn-5p), until a confluency of 90% to 100% was reached. Cells were separated by rinsing with PBS/5 mM EDTA, collected by centrifugation, re-suspended in phenol red-free Ham's F12 media (Invitrogen 12500-062) containing 0.1% BSA (BOVOGEN BSAS 100), and counted. The cell concentration was adjusted to 1×10⁶ cells/ml. 15 ml of the cell suspension was added to a 50-ml centrifuge tube, to which Coelenterazine-h (Promega S2011) was added to a final concentration of 5 µM. The tube was wrapped with a tin foil to shade light, and incubated on a rotary shaker at 20°C for 4 hours. The cells were diluted with a phenol red-free/0.1% BSA Ham's F12 medium to a final concentration of 5.0×10⁵ cells/ml, placed on a rotary shaker in low-speed rotation, and incubated at room temperature for at least 1 hour. The compounds of the Examples were formulated with DMSO into 10 mM stock solutions, diluted in series with a phenol red-free/0.1% BSA Ham's F12 medium (log(M): -7, -8, -9, -10, -11), and added to a 96-well plate at 50 µl/well. 50 µ1 cell suspension was further added to each well (25,000 cells/well) and incubated for 15 min at room temperature. The 96-well plate was placed under a microplate reader (Perkin Elmer, Envision), and a 50 µ1 solution of acetylcholine chloride (Sigma A6625) was added to each well at a concentration of 112.92 nM (hM3) with a microplate reader sample injector. The luminescence was recorded over 20 seconds, and IC₅₀ values were calculated and analyzed by Origin 7.5. The inhibitory activity of the compounds of the present invention against human muscarine receptor was determined through the above experiments, and the IC₅₀ values obtained are shown in Table 1 below.

**Table 1. Inhibitory activity of test compounds against human muscarine receptor M3.**

| Examples | IC₅₀ for hM3 receptor (nM) |
|---|---|
| Compound 1 | 14.44 |
| Compound 2 | 1.69 |
| Compound 3 | 3.76 |
| Compound 4 | 2.26 |
| Compound 5 | 0.89 |
| Compound 6 | 6.49 |
| Compound 7 | 1.31 |
| Compound 8 | 3.33 |
| Compound 9 | 3.73 |

Conclusion: the compounds of the present invention showed significant inhibitory activity against the human muscarine receptor M3.

### Test Example 2: Agonistic activity on human β-adrenergic receptor

The agonistic activity of the compounds of the Examples on the human adrenergic receptor was measured by the LANCE Ultra cAMP Assay.

CHO cells (PerkinElmer, ES-034-AF) that stably express human adrenergic receptor (hβ₂) were cultured at 37°C, 5% CO₂ in MEM-alpha media (Invitrogen 12561-056) containing 10% fetal bovine serum (FBS) (Gibico 10099-141) and 250 µg/ml Zeocin (InvivoGen ant-zn-5p), until a confluency of 90% to 100% was reached. Then the agonism on cAMP was tested by using a LANCE Ultra cAMP Assay kit (PerkinElmer TRF0263). Cells were separated with PBS/5 mM EDTA, collected by centrifugation, re-suspended in Stimulation Buffer (1 x HBSS, 5 mM HEPES, 0.5 mM IBMX, 0.1% BSA, pH=7.4), and adjusted to a cell concentration of 6×10⁵ cells/ml. The compounds of the Examples were formulated with DMSO into 10 mM stock solutions, diluted in series with Stimulation Buffer, and added to a 384-well plate at 5 µl/well. 5 µl cell suspension was further added to each well (3,000 cells/well), incubated for 30 min at room temperature, then 5 µl of a 4 x Eu-cAMP trace working solution was added to each well, and then 5 µ1 of a 4 x Ulight-anti-cAMP working solution was added to each well, followed by incubation at room temperature for 1 hour. The 384-well plate was detected for TR-FRET under a microplate reader (Perkin Elmer, Envision), and EC₅₀ values were calculated and analyzed by Origin 7.5. The agonistic activity of the compounds of the present invention on the human adrenergic receptor was determined through the above experiments, and the EC₅₀ values obtained are shown in Table 2 below.

**Table 2. Agonistic activity of test compounds on human β₂-adrenergic receptor**

| Examples | EC₅₀ for hβ₂ receptor (nM) |
|---|---|
| Compound 2 | 1.85 |
| Compound 3 | 10.21 |
| Compound 6 | 17.41 |
| Compound 7 | 6.01 |
| Compound 8 | 3.34 |
| Compound 9 | 3.96 |

Conclusion: the compounds of the present invention showed significant agonistic activity on the β₂-adrenergic receptor.

### Test Example 3: Inhibition against methacholine-induced bronchoconstriction in guinea pigs

8-week-old guinea pigs (all male) were purchased from Vital River Laboratory Animal Technology Co., Ltd., and adapted for 3 days before the experiments were started. The test compounds were formulated into 0.6 mM stock solutions with 83% dry ethanol + 17% Tween80, which were diluted 500 times before administration. Before administration, the animals were anesthetized with 5% isoflurane by using a small-animal anesthetic apparatus (Matrx: VME2), wherein the anesthetic duration was 1.5 to 2 min. The anesthetized guinea pigs were fixed on a tracheal cannula platform, and intratracheally administrated with the compounds at 250 µl/animal by using a rat liquid-aerosol administration kit (penn-century; MSA-250-R). 4 hours and 24 hours after administration, the enhanced pauses (PenH) of the guinea pigs were measured in a whole-body plethysmograph (DSI; GS220A12-R7B). 3 mg/ml methacholine (Mch) was administrated by nebulization for 36 sec, and data was recorded over 7 min. The average of PenH was calculated (see J Pharmacol Exp Ther 345:260-270). The experimental results are shown in Table 3.

PenH calculation formula: PenH = PEP/PIP*Pause; Pause=(Te-Tr)/Tr
Te: Duration of expiratory phase (s)
Tr: Duration of relaxation phase (s)
PEP: Expiratory peak flow rate (ml/s)
PIP: Inspiratory peak flow rate (ml/s)

**Table 3. Inhibitory effect of compounds on methacholine-induced bronchoconstriction in guinea pigs**

| Compounds | Administration dose (per animal) | PenH Inhibition (%) | |
|---|---|---|---|
| | | 4 h | 24 h |
| Compound 2 | 0.3 nmol | 94.8% | 53.8% |
| Compound 5 | 0.3 nmol | 96.4% | 78.6% |
| Compound 7 | 0.3 nmol | 72.1% | 55.8% |
| Compound 8 | 0.3 nmol | 78.2% | 45.3% |

Conclusion: The compounds of the present invention showed significant inhibition against methacholine-induced bronchoconstriction in guinea pigs, and the inhibition remained significant 24 hours after administration.

### Test Example 4: Pharmacokinetic evaluation

18 male SD rats, eight weeks old, were purchased from Vital River Laboratory Animal Technology Co., Ltd., and adapted for 3 days before the experiments were started. The SD rats were randomized into 3 groups, fasted one day before administration but allowed access to water. The 3 groups of animals were administrated with 1 mg/kg compound either by tail vein injection (iv) or by intratracheal (it) administration. The compounds were formulated into 20 mg/ml stock solutions with 83% ethanol and 17% Tween80. For intravenous injection, 0.1 ml of the stock solution was diluted to the final volume with 9.9 ml physiological saline. For intratracheal administration, 0.25 ml of the stock solution was diluted with 4.75 ml physiological saline to the final volume. For the group for intravenous injection, right before administration (0 h) and 5 min, 15 min, 30 min, 1.0, 2.0, 4.0, 8.0, 24.0 h after administration, 0.1 ml blood was taken from the orbit, heparin was added for anticoagulation, and plasma was separated after centrifugation at 4°C, 3000 rpm for 10 min and stored at -80°C before testing. For the group for intratracheal administration, right before administration and 5 min, 15 min, 30 min, 1.0, 2.0, 4.0, 8.0, 24.0 h after administration, blood was taken and treated the same way as for the group for intravenous injection. 30 µ1 plasma of rat at each of the time points was mixed with 200 µl acetonitrile solution containing an internal standard, vortexed for 1 min, and centrifuged at 11300 rpm for 10 min, and 175 µ1 of the supernatant was added to a 96-well plated for LC-MS/MS analysis. The major pharmacokinetic parameters were analyzed using the software WinNonlin 6.3 at the non-compartment mode.

**Table 4. Results of pharmacokinetic evaluation**

| Compound | Administration route | Pharmacokinetic parameters | | | |
|---|---|---|---|---|---|
| | | **Cmax** (ng/mL) | **AUC_{0→∞}** (ng*h /mL) | **t_{1/2}** (h) | **tₘₐₓ** (h) |
| Compound 2 | iv | - | 9.27 | 0.46 | - |
| | it | 7.08 | 11.6 | 0.75 | 0.58 |

Conclusion: After iv and it administrations, Compound 2 showed a low *in vivo* exposure level, and is expected to have good systemic safety.

## Claims

1. A compound represented by general formula (I), or a stereoisomer, a hydrate, a metabolite, a solvate, a pharmaceutically acceptable salt, a cocrystal, or a prodrug thereof, 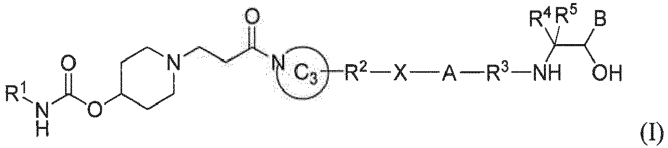 wherein
R¹ is selected from 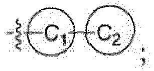 rings C₁ and C₂ are each independently selected from a C₆₋₁₀carbocycle or a 5- to 10-membered heterocycle, wherein the carbocycle or heterocycle is optionally further substituted with 0 to 5 substituents selected from the group consisting of F, Cl, Br, I, CF₃, NH₂, OH, carboxyl, cyano, a C₁₋₄alkyl, a C₁₋₄alkoxy, a C₁₋₄alkylthio, -NHC₁₋₄alkyl, -N(C₁₋₄alkyl)₂, -S(=O)-C₁₋₄alkyl, -S(=O)₂-C₁₋₄alkyl, and -C(=O)O-C₁₋₄alkyl, and wherein the heterocycle contains 1 to 3 heteroatoms selected from N, O or S;
ring C₃ is 4- to 7-membered azacycle, wherein the azacycle is optionally further substituted with 0 to 4 substituents selected from the group consisting of F, Cl, Br, I, OH, cyano, CF₃, a C₁₋₄alkyl, and a C₁₋₄alkoxy, and wherein the azacycle contains 1 to 3 heteroatoms selected from N, O or S;
R² is selected from a direct bond or a C₁₋₄alkylene, wherein the alkylene is optionally further substituted with 0 to 5 substituents selected from the group consisting of F, Cl, Br, I, OH, cyano, a C₁₋₄alkyl, and a C₁₋₄alkoxy;
X is selected from a direct bond, -O-, -C(=O)O-, -OC(=O)-, -S-, -S(=O)-, -S(=O)₂-, -C(=O)NR^{x}-, -NR^{x}C(=O)-, -OC(=O)NR^{x}-, -NR^{x}C(=O)O-, -NR^{x}C(=O)NR^{x}-, -NR^{x}S(=O)₂-, -S(=O)₂NR^{x}-, -NR^{x}S(=O)₂NR^{x}-, or -NR^{x}-;
R^{x}s are each independently selected from H or a C₁₋₄ alkyl;
A is selected from a direct bond, a C₆₋₁₀carbocycle or a 5- to 10-membered heterocycle, wherein the carbocycle or heterocycle is optionally further substituted with 0 to 5 R^{A}s, and wherein the heterocycle contains 1 to 4 heteroatoms selected from N, O or S;
R^{A} is selected from F, Cl, Br, I, OH, NH₂, carboxyl, cyano, nitro, (=O), a C₁₋₄alkyl, a C₂₋₄alkenyl, a C₂₋₄alkynyl, a C₁₋₄alkoxy, a -OC₃₋₆cycloalkyl, a C₁₋₄alkylthio, -S(=O)-C₁₋₄alkyl, -S(=O)₂-C₁₋₄alkyl, -C(=O)-C₁₋₄alkyl, -C(=O)O-C₁₋₄alkyl, -OC(=O)-C₁₋₄alkyl, a 5- or 6-membered heteroaryl, or -C(=O)NH₂, wherein the alkyl, alkoxy, cycloalkyl, alkenyl, alkynyl, heteroaryl, NH₂ and -C(=O)NH₂ are optionally further substituted with 0 to 4 substituents selected from the group consisting of F, Cl, Br, I, CF₃, a C₁₋₄alkyl, a C₁₋₄alkoxy, and -C(=O)-C₁₋₄alkyl;
R³ is C₁₋₆alkylene, wherein the alkylene is optionally further substituted with 0 to 5 substituents selected from R^{3a};
R^{3a} is selected from F, Cl, Br, I, cyano, OH, a C₁₋₄alkyl, a C₁₋₄alkoxy, phenyl, or phenyl-C₁₋₄alkylene;
alternatively, two R^{3a}s may form a 3- to 6-membered carbocycle together with the atoms to which they are attached, wherein the carbocycle is optionally further substituted with 0 to 5 substituents selected from the group consisting of F, Cl, Br, I, cyano, OH, a C₁₋₄alkyl, and a C₁₋₄alkoxy;
R⁴ and R⁵ are each independently selected from H or a C₁₋₄alkyl; and 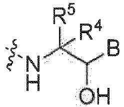 represents a β-adrenergic receptor binding group.

2. The compound according to claim 1, or a stereoisomer, a hydrate, a metabolite, a solvate, a pharmaceutically acceptable salt, a cocrystal, or a prodrug thereof, wherein
B is selected from 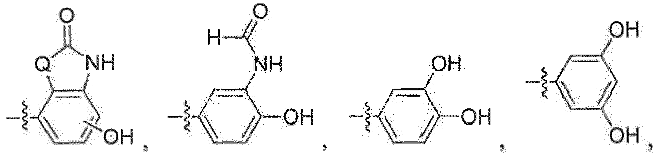 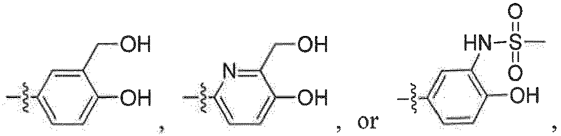 wherein Q is selected from -CH=CH-, -CH₂CH₂-, -O-, -S-, -CH₂O-, -OCH₂-, -C(CH₃)₂O- or -OC(CH₃)₂-.

3. The compound according to claim 2, or a stereoisomer, a hydrate, a metabolite, a solvate, a pharmaceutically acceptable salt, a cocrystal, or a prodrug thereof, wherein rings C₁ and C₂ are each independently selected from a C₆₋₁₀carbocycle or a 5- to 10-membered heterocycle, wherein the carbocycle or heterocycle is optionally further substituted with 0 to 5 substituents selected from the group consisting of F, Cl, Br, I, CF₃, NH₂, OH, cyano, a C₁₋₄alkyl, a C₁₋₄alkoxy, a C₁₋₄alkylthio, -NHC₁₋₄alkyl, and -N(C₁₋₄alkyl)₂, and wherein the heterocycle contains 1 to 3 heteroatoms selected from N, O or S;
R^{A} is each independently selected from F, Cl, Br, I, OH, NH₂, carboxyl, cyano, (=O), a C₁₋₄alkyl, a C₂₋₄ alkynyl, a C₁₋₄alkoxy, a -OC₃₋₆cycloalkyl or a C₁₋₄alkylthio, wherein the alkyl, alkynyl, alkoxy, cycloalkyl and the NH₂ is optionally further substituted with 0 to 4 substituents selected from the group consisting of F, Cl, Br, I, CF₃, a C₁₋₄ₐlkyl, a C₁₋₄alkoxy, and -C(=O)-C₁₋₄alkyl.

4. The compound according to claim 3, or a stereoisomer, a hydrate, a metabolite, a solvate, a pharmaceutically acceptable salt, a cocrystal, or a prodrug thereof, wherein: rings C₁ and C₂ are each independently selected from a benzene ring, thiophene ring, thiazole ring, isothiazole ring, furan ring, oxazole ring, isoxazole ring, pyrrole ring, imidazole ring, pyridine ring, benzothiophene ring, benzothiazole ring, benzofuran ring or benzoxazole ring, wherein the benzene ring, thiophene ring, thiazole ring, isothiazole ring, furan ring, oxazole ring, isoxazole ring, pyrrole ring, imidazole ring, pyridine ring, benzothiophene ring, benzothiazole ring, benzofuran ring or benzoxazole ring is optionally further substituted with 0 to 5 substituents selected from the group consisting of F, Cl, Br, I, CF₃, NH₂, OH, cyano, a C₁₋₄alkyl, a C₁₋₄alkoxy, a C₁₋₄alkylthio, -NHC₁₋₄alkyl and -N(C₁₋₄alkyl)₂;
ring C₃ is 4- to 7-membered azacycle, wherein the azacycle is optionally further substituted with 0 to 4 substituents selected from the group consisting of F, Cl, Br, I, CF₃, methyl and ethyl, and wherein the azacycle contains 1 to 3 heteroatoms selected from N, O or S;
R² is selected from a direct bond, methylene, ethylene or propylene, wherein the methylene, ethylene or propylene is optionally further substituted with 0 to 5 substituents selected from the group consisting of F, Cl, Br, I, cyano, OH, methyl, ethyl, methoxy and ethoxy;
A is selected from a direct bond, phenylene, or pyridylene, wherein the phenylene or pyridylene is optionally further substituted with 0 to 4 substituents selected from the group consisting of F, Cl, Br, cyano, methyl, ethyl, propyl, isopropyl, CHF₂, CF₃, methoxy, ethoxy, -OCHF₂, -OCF₃, ethynyl, and propynyl;
X is selected from a direct bond, -O-, -C(=O)NR^{x}-, -NR^{x}C(=O)-, -OC(=O)NR^{x}-, or -NR^{x}C(=O)O-;
R^{x} is selected from H, methyl, ethyl or propyl;
R³ is selected from methylene, ethylene, propylene, butylene, pentylene, or 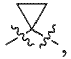 wherein the methylene, ethylene, propylene, butylene, pentylene, or 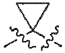 is optionally further substituted with 0 to 5 substituents selected from the group consisting of F, Cl, Br, I, cyano, OH, methyl, ethyl, methoxy and ethoxy;
R⁴ and R⁵ are each independently selected from H, methyl or ethyl;
B is selected from

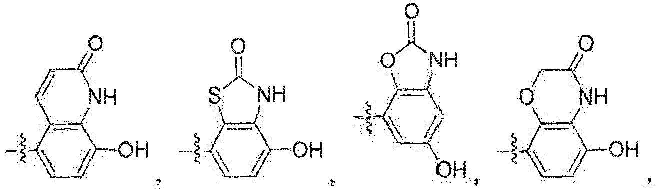

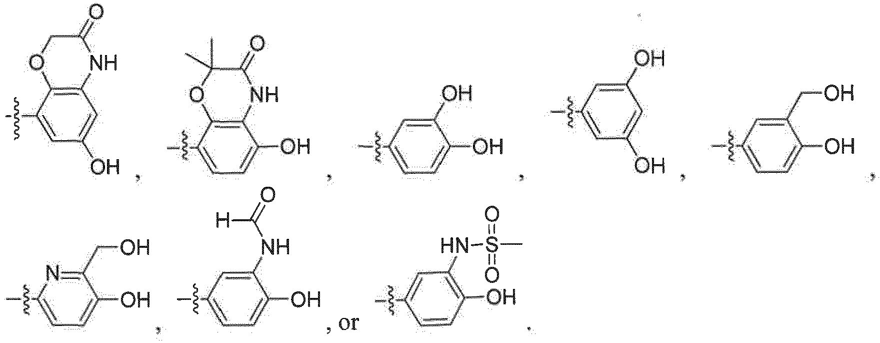

5. The compound according to claim 4, or a stereoisomer, a hydrate, a metabolite, a solvate, a pharmaceutically acceptable salt, a cocrystal, or a prodrug thereof, wherein:
R¹ is selected from 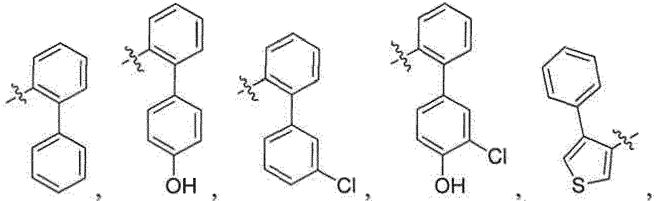 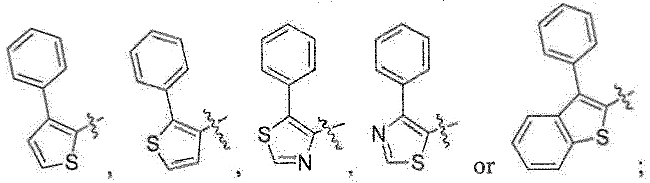 R² is selected from a direct bond, methylene, ethylene or propylene;
R³ is selected from methylene, ethylene, propylene, -CH₂CH(CH₃)-, -CH(CH₃)CH₂-, -CH₂C(CH₃)₂-, -C(CH₃)₂CH₂-, butylene, -CH(CH₃)CH₂CH₂-, -CH₂CH(CH₃)CH₂-, -CH₂CH(CH₃)CH₂-, 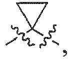 or pentylene;
A is selected from a direct bond, phenylene, or pyridylene, wherein the phenylene or pyridylene is optionally further substituted with 0 to 4 substituents selected from the group consisting of F, Cl, Br, CHF₂, CF₃, cyano, methyl, ethyl, methoxy, ethoxy, -OCHF₂, -OCF₃, ethynyl and propynyl.

6. The compound according to claim 4, or a stereoisomer, a hydrate, a metabolite, a solvate, a pharmaceutically acceptable salt, a cocrystal, or a prodrug thereof, wherein the compound is represented by general formula (II): 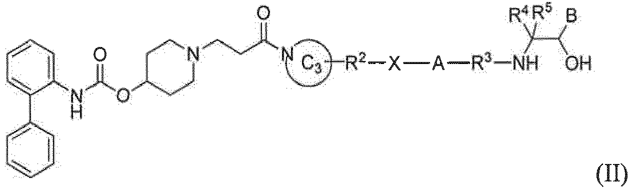 ring C₃ is selected from 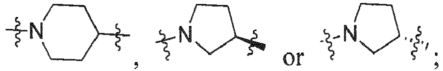 A is selected from a direct bond or phenylene, wherein the phenylene is optionally further substituted with 0 to 4 substituents selected from the group consisting of F, Cl, Br, CHF₂, CF₃, cyano, methyl, ethyl, methoxy, ethoxy, -OCHF₂, -OCF₃, ethynyl, and propynyl.

7. The compound according to claim 1, or a stereoisomer, a hydrate, a metabolite, a solvate, a pharmaceutically acceptable salt, a cocrystal, or a prodrug thereof, wherein the compound is selected from:

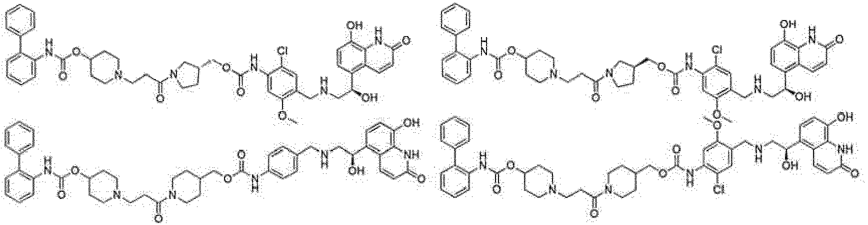

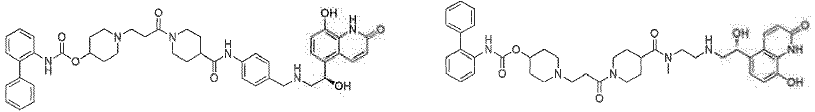

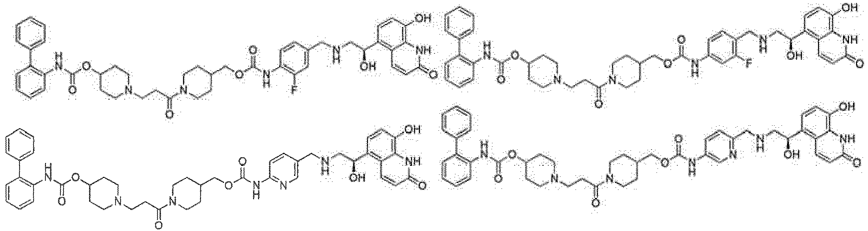

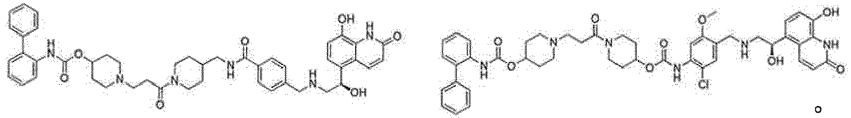

8. A pharmaceutical composition comprising:
a therapeutically effective amount of a compound according to any one of claims 1 to 7, or a stereoisomer, a hydrate, a metabolite, a solvate, a pharmaceutically acceptable salt, a cocrystal, or a prodrug thereof; and
a pharmaceutically acceptable carrier, diluent, adjuvant, medium, or excipient;
wherein the composition may further comprise one or more secondary therapeutic agents.

9. The pharmaceutical composition according to claim 8, wherein the secondary therapeutic agents are selected from one or more of PDE4 inhibitors, M receptor antagonists, corticosteroids and β-adrenergic receptor agonists.

10. Use of a compound according to any one of claims 1 to 7, or a stereoisomer, a hydrate, a metabolite, a solvate, a pharmaceutically acceptable salt, a cocrystal, or a prodrug thereof, or a pharmaceutical composition according to claim 8 or 9, in the manufacture of a medicament for treatment of an airway obstructive disease.

11. The use according to claim 10, wherein the airway obstructive disease is selected from asthma, chronic obstructive pulmonary disease (COPD) or bronchitis.

12. A method for treating an airway obstructive disease, comprising administering a compound according to any one of claims 1 to 7, or a stereoisomer, a hydrate, a metabolite, a solvate, a pharmaceutically acceptable salt, a cocrystal, or a prodrug thereof, or a pharmaceutical composition according to claim 8 or 9.

13. The method according to claim 12, wherein the airway obstructive disease is selected from asthma, chronic obstructive pulmonary disease (COPD) or bronchitis.

14. An intermediate for preparing a compound of general formula (I) or a stereoisomer thereof, said intermediate being selected from compounds of general formula (I-M1) or (I-M2) or a stereoisomer thereof: 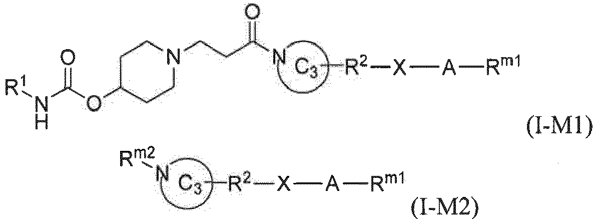
wherein R^{m1} is selected from -COOH, a -COOC₁₋₄alkyl, cyano, -CHO, 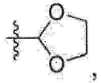 C(OC₁₋₄alkyl)₂, 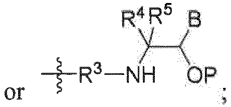
R^{m2} is selected from H or a protective group for amino;
P is a protective group for hydroxyl;
R¹, R², R³, R⁴, R⁵, A, B, ring C₃ and X are as defined in claim 1.

15. The intermediate according to claim 14, being one of:
